# EUROPEAN PATENT SPECIFICATION

(11) **EP 3 082 834 B1**
(45) Date of publication and mention of the grant of the patent: **11.03.2020**
(21) Application number: 14869630.5
(22) Date of filing: 11.12.2014
(51) Int. Cl.: A61K 48/00, A61K 35/35, A61K 35/545, C07K 14/705, A61K 38/17, A61K 9/00, A61K 35/28, A61K 35/30, A61K 35/51, A61K 35/763, C12N 7/00, A61P 35/00

(54) **STEM CELL DELIVERED ONCOLYTIC HERPES SIMPLEX VIRUS AND METHODS FOR TREATING BRAIN TUMORS**
VON STAMMZELLEN ABGEGEBENER ONKOLYTISCHER HERPES-SIMPLEX-VIRUS UND VERFAHREN ZUR BEHANDLUNG VON HIRNTUMOREN
VIRUS HERPES SIMPLEX ONCOLYTIQUE DÉLIVRÉ PAR UNE CELLULE SOUCHE ET PROCÉDÉS DE TRAITEMENT DE TUMEURS DU CERVEAU

(30) Priority: 11.12.2013 US 201361914481 P
(43) Date of publication of application: 26.10.2016
(73) Proprietor: The General Hospital Corporation DBA Massachusetts, Boston, MA 02114 (US)
(72) Inventor: SHAH, Khalid, Andover, Massachusetts 01810 (US)
(74) Representative: Barker Brettell LLP
(86) International application number: PCT/US2014/069734
(87) International publication number: WO 2015/089280

(56) References cited:
- EP-A2- 1 909 849
- WO-A1-2014/018113
- WO-A2-2012/106281
- US-A1- 2005 214 266
- US-A1- 2010 272 686
- US-A1- 2011 177 032
- US-A1- 2013 189 189
- US-B2- 7 790 451
- US-B2- 8 222 036
- Tyrel Smith ET AL: "Human Bone Marrow-Derived Mesenchymal Stem Cells for Intranasal Delivery of Oncolytic Herpes Simplex Virus Type I to Human Gliomas", Molecular Therapy, 1 June 2013 (2013-06-01), pages s246-s247, XP055369722, Retrieved from the Internet: URL:http://www.cell.com/molecular-therapy- family/molecular-therapy/pdf/S1525-0016(16 )34981-4.pdf [retrieved on 2017-05-04]
- VOSS ET AL: "Delivery of Oncolytic Herpes Simplex Virus to Infiltrative Brain Tumor Sites Via Neuronal Stem Cells", MOLECULAR THERAPY, ACADEMIC PRESS, US, vol. 13, 1 January 2006 (2006-01-01), page S411, XP005676178, ISSN: 1525-0016, DOI: 10.1016/J.YMTHE.2005.10.013
- HIROAKI WAKIMOTO ET AL: "Human glioblastoma-derived cancer stem cells: establishment of invasive glioma models and treatment with oncolytic herpes simplex virus vectors", CANCER RESEARCH, AMERICAN ASSOCIATION FOR CANCER RESEARCH, US, vol. 69, no. 8, 15 April 2009 (2009-04-15) , pages 3472-3481, XP008141764, ISSN: 0008-5472, DOI: 10.1158/0008-5472.CAN-08-3886 [retrieved on 2009-04-07]
- KAORU TAMURA ET AL: "Multimechanistic Tumor Targeted Oncolytic Virus Overcomes Resistance in Brain Tumors", MOLECULAR THERAPY, vol. 21, no. 1, 1 January 2013 (2013-01-01), pages 68-77, XP055369717, US ISSN: 1525-0016, DOI: 10.1038/mt.2012.175
- JIANG Y ET AL: "A new approach with less damage: intranasal delivery of tetracycline-inducible replication-defective herpes simplex virus type-1 vector to brain", NEUROSCIENCE, NEW YORK, NY, US, vol. 201, 22 October 2011 (2011-10-22), pages 96-104, XP028437742, ISSN: 0306-4522, DOI: 10.1016/J.NEUROSCIENCE.2011.10.043 [retrieved on 2011-11-10]
- Prakash Rath ET AL: "Stem Cells as Vectors to Deliver HSV/tk Gene Therapy for Malignant Gliomas", Current Stem Cell Research & Therapy, vol. 4, no. 1, 1 January 2009 (2009-01-01) , pages 44-49, XP055481262, AE ISSN: 1574-888X, DOI: 10.2174/157488809787169138
- Prakash Rath ET AL: "Stem Cells as Vectors to Deliver HSV/tk Gene Therapy for Malignant Gliomas", Current Stem Cell Research & Therapy, vol. 4, no. 1, 1 January 2009 (2009-01-01) , pages 44-49, XP055481273, AE ISSN: 1574-888X, DOI: 10.2174/157488809787169138
- James J. Cody ET AL: "Preclinical Evaluation of Oncolytic [Delta] [gamma] 1 34.5 Herpes Simplex Virus Expressing Interleukin-12 for Therapy of Breast Cancer Brain Metastases", International Journal of Breast Cancer, vol. 2012, 1 January 2012 (2012-01-01), pages 1-12, XP055481292, ISSN: 2090-3170, DOI: 10.1155/2012/628697
- James J. Cody ET AL: "Preclinical Evaluation of Oncolytic [Delta] [gamma] 1 34.5 Herpes Simplex Virus Expressing Interleukin-12 for Therapy of Breast Cancer Brain Metastases", International Journal of Breast Cancer, vol. 2012, 1 January 2012 (2012-01-01), pages 1-12, XP055481295, ISSN: 2090-3170, DOI: 10.1155/2012/628697

## Description

### RELATED APPLICATIONS

This Application claims the benefit under 35 U.S.C. § 119(e) of U.S. Provisional Application serial number 61/914,481, filed December 11, 2013.

### GOVERNMENTAL SUPPORT

This invention was made with Government support under RO1 CA138922 and RO1 NS071197 awarded by the National Institutes of Health. The Government has certain rights in the invention.

### FIELD OF THE INVENTION

The present invention relates to the field of cancer therapeutics.

### BACKGROUND OF THE INVENTION

The current treatment regimen for malignant glioblastoma multiforme (GBM) is tumor-resection followed by chemo- and radiation therapies. Despite the proven safety of oncolytic herpes simplex virus (oHSV) in clinical trials for GBMs, its efficacy is sub-optimal mainly due to insufficient viral spread post-tumor resection. Glioblastoma multiforme (GBM) is the most common brain tumor in adults and despite great advances in its molecular understanding it remains one of the most difficult to treat malignancies [1]. Although GBM tumor resection constitutes an important therapeutic intervention, standard treatment with radiation and temozolomide chemotherapy post-tumor resection only provides modest clinical benefits [2, 3]. Therefore, the development of novel local therapeutics that can be administered directly into the GBM tumor resection cavity post tumor debulking are urgently needed. Previous studies attempting to use local therapy with clinically approved Gliadel wafers, polyanhydride wafers containing the chemotherapeutic agent, BCNU, in the cavity of resected GBM, have been shown to have limited therapeutic benefit [4]. In the ongoing search for therapeutics that are capable of eliminating such tumor residues post tumor resection, oncolytic viruses have shown great potential in preclinical studies [5-8]. These viruses are typically genetically engineered so as to only replicate in and kill neoplastic cells, an approach that fits well in the brain where actively proliferating tumor cells are amidst non-or slowly-proliferating normal cells. Among therapeutic viruses, oncolytic Herpes Simplex Virus (oHSV) is one of the most promising candidates for GBM therapy as it is an inherently neurotropic virus and is less dependent on certain host cell receptors, mutations or intracellular pathways for its oncolytic effect [5, 9, 10]. Also, oHSV has a well-studied genome and a large transgene capacity for insertion of additional therapeutic genes to further enhance its oncolytic potency [11, 12]. Although phase I and Ib oHSV clinical trials conducted to date for GBM have shown signs of anti-tumor activity, clinical response rates have been sub-optimal [7, 13-16]. The standard procedure in clinical trials involves direct injections of purified virus into brain parenchyma adjacent to the tumor resection cavity after tumor debulking [13, 14]. The sub-optimal response rates in these trials may be partly because of the secondary bleeding caused by the surgical intervention and influx of cerebrospinal fluid into the resection cavity rinse out and disperse injected virus, resulting in delivery issues. In addition, oncolytic viruses can be subjected to neutralizing antibody mediated degradation within the tumor resection cavity, or get absorbed by non-neoplastic cells of the brain, thus blunting virus replication. To improve delivery of viral therapeutics and circumvent antiviral immunity, a number of studies have explored the possibility of using infected cells as delivery vehicles for oncolytic viruses [17-23]. Mesenchymal stem cells (MSC) have shown great promise in this respect and several studies have employed MSC for delivery of oncolytic adenoviruses to GBM [17, 19, 23, 24]. Although promising, these studies have been limited by their inability to explore the therapeutic efficacy of MSC loaded oncolytic viruses that could be translated into clinics in GBM patients.

### SUMMARY OF THE INVENTION

Aspects of the invention relate to a composition for use in treating a secondary multifocal metastatic cancer in the brain, wherein the composition comprises an isolated non-cancer stem cell or population thereof comprising infectious recombinant oncolytic herpes simplex virus (oHSV), wherein the isolated stem cell or population is selected from the group consisting of a mesenchymal stem cell (MSC), a neuronal stem cell, and an induced pluripotent stem cell, and wherein the composition is administered via intracarotid injection. In one embodiment, the isolated stem cell or population thereof of is human. In one embodiment, the MSC is derived from bone marrow, umbilical cord blood, or adipose tissue. In one embodiment, the oncolytic HSV is engineered to be inducible by addition of an exogenous factor. In one embodiment, the oncolytic HSV is engineered to have a tetracycline inducible promoter driving ICP27 expression. In one embodiment, the oncolytic HSV is engineered to comprise a nucleic acid sequence encoding tumor necrosis factor-related apoptosis-inducing ligand (TRAIL) or a biologically active fragment thereof, in expressible form. In one embodiment, the TRAIL is a secreted form of TRAIL (S-TRAIL). In one embodiment, the oHSV is selected from the group consisting of 207, G47Δ HSV-R3616, 1716, R3616, and R4009. In one embodiment, the TRAIL is a TRAIL fusion protein. In one embodiment, wherein the TRAIL is regulated by the HSV immediate early 4/5 promoter. In one embodiment, the virus contains an additional exogenous nucleic acid in expressible form. In one embodiment, the virus contains no additional exogenous nucleic acids. In one embodiment, the isolated stem cell or population thereof is encapsulated in a synthetic extracellular matrix (sECM).

Another aspect of the invention relates to a pharmaceutical composition comprising the isolated stem cell or population thereof described herein, and a pharmaceutically acceptable carrier.

### Definitions

An "oncolytic virus" is any virus which typically is able to kill a tumor cell (non-resistant) by infecting the tumor cell.

An oncolytic virus is "replication-selective" if it is more capable of replicating or is capable of replicating to a greater extent (e.g. burst size) in a tumor cell of a subject than in a non-tumor cell of the subject.

An "effective amount" as the term is used herein, is used to refer to an amount that is sufficient to produce at least a reproducibly detectable amount of the desired results. An effective amount will vary with the specific conditions and circumstances. Such an amount can be determined by the skilled practitioner for a given situation.

As used herein, the terms "treat," treating," "treatment," and the like refer to reducing or ameliorating a disorder and/or symptoms associated therewith. Beneficial or desired clinical results include, but are not limited to, elimination of symptoms, alleviation of symptoms, diminishment of extent of condition, stabilized (i.e., not worsening) state of condition, delay or slowing of progression of the condition. It will be appreciated that, although not precluded, treating a disorder or condition does not require that the disorder, condition or symptoms associated therewith be completely eliminated. More than one dose may be required for treatment of a disease or condition.

The term "therapeutically effective amount" refers to an amount that is sufficient to produce a therapeutically significant reduction in one or more symptoms associated with a disease being treated, when administered to a typical subject with that condition. Such symptom may include tumor progression, tumor size, tumor number, as well as degree of tumorigenicity (e.g., invasiveneness of tumor), patient relapse time, patient remission, survival rate and survival time. A therapeutically significant reduction in a symptom is, e.g. about 10%, about 20%, about 30%, about 40%, about 50%, about 60%, about 70%, about 80%, about 90%, about 100%, about 125%, about 150% or more as compared to a control or non-treated subject.

Such amounts for therapy will depend, of course, on the particular condition being treated, the severity of the condition and individual patient parameters including age, physical condition, size, weight and concurrent treatment. These factors are well known to those of ordinary skill in the art and can be addressed with no more than routine experimentation. It is preferred generally that a maximum dose be used, that is, the highest safe dose according to sound medical judgment. It will be understood by those of ordinary skill in the art, however, that a lower dose or tolerable dose can be administered for medical reasons, psychological reasons or for virtually any other reasons.

The term "inhibiting tumor cell growth or proliferation" means decreasing a tumor cell's growth or proliferation by at least 10%, 20%, 30%, 40%, 50%, 60%, 70%, 80%, 90%, 95%, or 100%, and includes inducing cell death in a cell or cells within a cell mass.

The term "tumor progression" refers to all stages of a tumor, including tumorigenesis, tumor growth and proliferation, invasion, and metastasis.

The term "inhibiting tumor progression" means inhibiting the development, growth, proliferation, or spreading of a tumor, including without limitation the following effects: inhibition of growth of cells in a tumor, (2) inhibition, to some extent, of tumor growth, including slowing down or complete growth arrest; (3) reduction in the number of tumor cells; (4) reduction in tumor size; (5) inhibition (i.e., reduction, slowing down or complete stopping) of tumor cell infiltration into adjacent peripheral organs and/or tissues; (6) inhibition (i.e. reduction, slowing down or complete stopping) of metastasis; (7) increase in the length of survival of a patient or patient population following treatment for a tumor; and/or (8) decreased mortality of a patient or patient population at a given timepoint following treatment for a tumor.

A tumor "responds" to a particular agent if tumor progression is inhibited as defined above.

The term "pharmaceutical composition" refers to compositions or formulations that usually comprise an excipient, such as a pharmaceutically acceptable carrier that is conventional in the art and that is suitable for administration to a subject, such as a mammals, and preferably humans. Such compositions can be specifically formulated for administration via one or more of a number of routes described herein. The pharmaceutical composition may further provide a suitable environment for preservation of the viability of any cells contained therein to be administered in the composition.

The "pharmaceutically acceptable carrier" means any pharmaceutically acceptable means to mix and/or deliver the targeted delivery composition to a subject. The term "pharmaceutically acceptable carrier" as used herein means a pharmaceutically acceptable material, composition or vehicle, such as a liquid or solid filler, diluent, excipient, solvent or encapsulating material, involved in carrying or transporting the subject agents from one organ, or portion of the body, to another organ, or portion of the body. Each carrier must be "acceptable" in the sense of being compatible with the other ingredients of the formulation and is compatible with administration to a subject, for example a human.

The terms "patient", "subject" and "individual" are used interchangeably herein, and refer to an animal, particularly a human, to whom treatment including prophylaxic treatment is provided. This includes human and non-human animals. The term "non-human animals" and "non-human mammals" are used interchangeably herein includes all vertebrates, e.g., mammals, such as non-human primates, (particularly higher primates), sheep, dog, rodent (e.g. mouse or rat), guinea pig, goat, pig, cat, rabbits, cows, and non-mammals such as chickens, amphibians, reptiles etc. In one embodiment, the subject is human. In another embodiment, the subject is an experimental animal or animal substitute as a disease model. Patient or subject includes any subset of the foregoing, e.g., all of the above, but excluding one or more groups or species such as humans, primates or rodents. A subject can be male or female. A subject can be a fully developed subject (e.g., an adult) or a subject undergoing the developmental process (e.g., a child, infant or fetus).

Preferably, the subject is a mammal. The mammal can be a human, non-human primate, mouse, rat, dog, cat, horse, or cow, but are not limited to these examples. Mammals other than humans can be advantageously used as subjects that represent animal models of disorders associated with unwanted neuronal activity. In addition, the methods and compositions described herein can be used to treat domesticated animals and/or pets.

The term "mammal" refers to any animal classified as a mammal, including humans, non-human primates, domestic and farm animals, and zoo, sports, or pet animals, such as dogs, cats, cattle, horses, sheep, pigs, goats, rabbits, etc.

The terms "cell proliferative disorder" and "proliferative disorder" refer to disorders that are associated with some degree of abnormal cell proliferation. In one embodiment, the cell proliferative disorder is a tumor. In one embodiment, the cell proliferative disorder is cancer.

The term "tumor," as used herein, refers to all neoplastic cell growth and proliferation, whether malignant or benign, and all pre-cancerous and cancerous cells and tissues. The terms "cancer," "cancerous," "cell proliferative disorder," "proliferative disorder" and "tumor" are not mutually exclusive as referred to herein. In one embodiment, tumors are benign. Examples of benign tumors include, without limitation, schwannomas, lipoma, chondroma, adenomas (e.g, hepatic adenoma), and benign brain tumors (e.g., glioma, astrocytoma, meningioma).

The terms "cancer" and "cancerous" refer to or describe the physiological condition in mammals that is typically characterized by unregulated cell growth/proliferation. Examples of cancer include, but are not limited to, carcinoma, lymphoma (e.g., Hodgkin's and non-Hodgkin's lymphoma), blastoma, sarcoma, and leukemia. More particular examples of such cancers include melanoma, squamous cell cancer, small-cell lung cancer, non-small cell lung cancer, adenocarcinoma of the lung, squamous carcinoma of the lung, cancer of the peritoneum, hepatocellular cancer, gastrointestinal cancer, pancreatic cancer, cervical cancer, ovarian cancer, liver cancer, bladder cancer, hepatoma, breast cancer, colon cancer, colorectal cancer, endometrial or uterine carcinoma, salivary gland carcinoma, kidney cancer, liver cancer, prostate cancer, vulval cancer, thyroid cancer, hepatic carcinoma, leukemia and other lymphoproliferative disorders, and various types of head and neck cancer.

As used herein, the term "primary cell", refers to a cell that is obtained directly from an organism. The cells can undergo several rounds of proliferation, or rounds of passages, to expand the population prior to use in the methods described herein to produce the stem cells. In one embodiment, the primary cells undergo few to no rounds of proliferation prior to use.

The term "multipotent" is used to refer to cells that can differentiate into a number of different cell types, especially those of a closely related family of cells. Such cells are also referred to in the art as "stem cells" as the term is used herein.

The term "purified" is used to refer to a molecule that is substantially free of other cellular material, culture medium, chemical precursors or other chemicals. The term "purified" when used in reference to a polypeptide or virus refers to the fact that it is removed from the majority of other cellular components from which it was generated or in which it is typically present in nature. The polypeptides and viruses described herein may be in a state where they are purified or semi-purified. For example, purified is about 80% free, about 85% free, about 90% free, or about 95% free from other materials.

By "isolated" is meant a material that is free to varying degrees from components which normally accompany it as found in its native state. "Isolate" denotes a degree of separation from the original source or surroundings. Typically, an isolated cell has been removed from the organism of origin and placed in a culture dish for propogation, or into another animal. Isolated is not meant as being removed from all other cells since more than a single cell is typically removed from the organism of origin. The term "isolated" when used in reference to a nucleic acid sequence refers to the fact that the nucleic acid sequence is removed from the context of other nucleic acid sequences in which it is present in nature (e.g.,. in the context of a chromosome). The nucleic acids of the invention are typically present in isolated form.

By "population" is meant at least 2 cells. In a preferred embodiment, population is at least 5, 10, 50, 100, 500, 1000, or more cells. The invention relates to stem cells described herein. As such, use of the term "cell" or "isolated cell" when describing the invention is also intended to describe a population of such cells. Populations of cells and isolated cells described herein are also encompassed by the present invention. A population may be comprised of genetically identical cells. The population may contain only a single cell type, or may contain multiple cell types. For example, the population may contain a single cell type described herein (e.g., mesenchymal stem or neural stem cells) or may contain a plurality of different cell types, (e.g., at various stages of differentiation). The population may contain unrelated cell types as well.

The term "in expressible form" when used in the context of a DNA molecule means operably linked (e.g., located within functional distance) to sequences necessary for transcription of the DNA into RNA by the RNA polymerase transcription machinery found in eukaryotic cells (e.g., promoter sequences, and other 5' regulatory sequences). One example is a DNA molecule in the context of an expression vector. Expression can refer to transcription of DNA into RNA, and when protein coding sequences are involved, expression may also encompass translation of the mRNA into protein. Viral expression vectors may comprise the viral genome in the context of a virion that is used to infect a cell.

The term "operably linked" is used herein to refer to a functional relationship of one nucleic acid sequence to another nucleic acid sequence. Nucleic acid sequences are "operably linked" when placed into a functional relationship with one another. For example, a promoter or enhancer is operably linked to a coding sequence if it affects the transcription of the sequence; or a ribosome binding site is operably linked to a coding sequence if it is positioned so as to facilitate translation. The DNA sequences being linked may be contiguous, or separated by intervening sequences, and when necessary in the same reading phase and/or appropriate orientation. Linking is accomplished, for example, by ligation at convenient restriction sites. If such sites do not exist, the synthetic oligonucleotide adaptors or linkers are used in accordance with conventional practice.

The term "heterologous" is used herein to describe the relationship of one nucleic acid or amino acid sequence to one or more different nucleic acid or amino acid sequences, respectively. The term heterologous, in reference to two or more such sequences, indicates that the different sequences are found in nature within separate, different and distinct larger nucleic acids or polypeptides. The joining of heterologous sequences creates a non-naturally occurring juxtaposition of sequences. Such joining is the product of engineering performed in the laboratory. The products of such joining are referred to as "recombinant". When such amino acid sequences are joined (e.g., by expression of the joined respective encoding nucleic acid sequences), the resulting protein is referred to herein as a fusion protein.

As the term is used herein, "transfection" refers to the introduction of nucleic acid into a cell (e.g., for the purpose of propagation and/or expression of the nucleic acid by the cell). Examples of methods of transfection are electroporation, calcium phosphate, lipofection, and viral infection utilizing a viral vector. Often nucleic acid is introduced into a cell in expressible form. That means that the nucleic acid is in the appropriate context of regulatory sequences such that the cellular machinery will recognize it and process it (e.g., transcribe RNA from DNA, translate protein from RNA). In one embodiment, a nucleic acid is in expressible form when it is inserted into an expression vector in the proper orientation to confer expression.

The term "stem cell" refers to a subset of progenitors that have the capacity or potential, under particular circumstances, to differentiate to a more specialized or differentiated phenotype, and which retains the capacity, under certain circumstances, to proliferate without substantially differentiating. In one embodiment, the term stem cell refers generally to a naturally occurring mother cell whose descendants (progeny) specialize, often in different directions, by differentiation, e.g., by acquiring completely individual characters, as occurs in progressive diversification of embryonic cells and tissues. Cellular differentiation is a complex process typically occurring through many cell divisions. A differentiated cell may derive from a multipotent cell which itself is derived from a multipotent cell, and so on. While each of these multipotent cells may be considered stem cells, the range of cell types each can give rise to may vary considerably. Some differentiated cells also have the capacity to give rise to cells of greater developmental potential. Such capacity may be natural or may be induced artificially upon treatment with various factors. In many biological instances, stem cells are also "multipotent" because they can produce progeny of more than one distinct cell type, but this is not required for "stem-ness." Self-renewal is the other classical part of the stem cell definition, and it is essential as used in this document. In theory, self-renewal can occur by either of two major mechanisms. Stem cells may divide asymmetrically, with one daughter retaining the stem state and the other daughter expressing some distinct other specific function and phenotype. Alternatively, some of the stem cells in a population can divide symmetrically into two stems, thus maintaining some stem cells in the population as a whole, while other cells in the population give rise to differentiated progeny only. Formally, it is possible that cells that begin as stem cells might proceed toward a differentiated phenotype, but then "reverse" and re-express the stem cell phenotype, a term often referred to as "dedifferentiation" or "reprogramming" or "retrodifferentiation".

As used herein, the term "adult cell" refers to a cell found throughout the body after embryonic development.

### BRIEF DESCRIPTION OF THE DRAWINGS

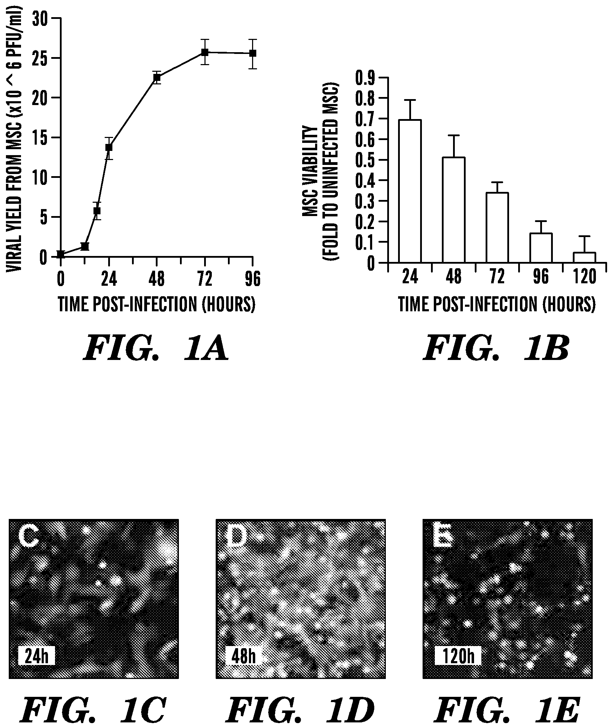

Figure 1A - Figure 1Q are graphs and images of experimental results that indicate mesenchymal stem cells loaded with oHSV allow replication and release of viral particles, resulting in strong anti-tumor efficacy *in vitro* and *in vivo.* MSC were infected with oHSV-mCh and followed over time for virus yield and MSC viability. (A) Plot showing oHSV yield in MSC *in vitro* over 4 days. *Bars,* +SD. (B) Plot showing survival of MSC-oHSV-mCh *in vitro. Bars,* +SD. (C-E) Photomicrographs of MSC-oHSV-mCh undergoing different stages of transgene expression (mCh) and cytopathic effect at 24 (C), 48 (D) and 120 (E) hours post infection. (F) MSC expressing firefly luciferase (Fluc) were loaded with oHSV-mCh and MSC survival was followed in mice brains for a period of 5 days. Fluc signal activity as a measure of MSC survival in mice is shown. *Bars,* +SE. (G-I) Photomicrographs of brain sections from mice after implantation of MSC-oHSV-mCh. Mice were sacrificed at 24 (G), 48 (H) and 120 (I) hours post-implantation respectively. Arrowheads show cells in the first stages of virus replication (G), cytopathic effect (H) and cell debris after virus mediated cell lysis (I). (J-P) Different human GBM lines engineered to express GFl fusion marker, Gli36vIII-GFl and U87-GFl, were co-cultured with 3% of MSC-oHSV-mCh, and oHSV spread and corresponding changes in their survival were followed. Photomicrographs of Gli36vIII-GFl (J-L) and U87-GFl (M-O) at 24 (J,M), 48 (K,N) and 96 (L,O) hours respectively are shown. Green cells represent GBM-GFl cells, red cells MSC-oHSV-mCh and yellow cells represent GBM-GFl cells which have been infected with oHSV-mCh released from MSC carrier cells (P) Plot showing survival of Gli36vIII-GFl and U87-GFl after co-culture with MSC-oHSV-mCh. *Bars,* +SD. (Q) Plot showing tumor volume changes in mice bearing intracerebral Gli36vIII-GFl tumors which were treated with MSC or MSC-oHSV-mCh. *Bars,* +SE. Original magnifications: x4 (J-O) and x10 (C-E, G-I). In all panels, *, p<0.05 versus controls.

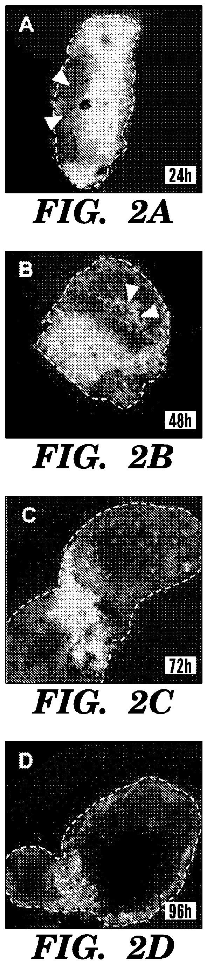

Figure 2A- Figure 2M are fluorescent images and graphs of experimental results that indicate detailed effects and progression of viral oncolysis caused by stem cell delivered oHSV-mCh. (A-D) Gli36vIII-GFl tumor bearing mice were treated with MSC-oHSV-mCh. Production of oHSV-mCh (mCh) in MSC and infection of Gli36vIII-GFl cells (GFP+mCh) were monitored over time. Low magnification sections showing viral infection (visualized as red) of tumor cells (visualized as green) over 4 days post MSC-oHSV-mCh implantation. Arrowheads show MSC-oHSV-mCh implantation site. Viral production in hMSC and infection of Gli36vIII-GFl resulted in almost entire destruction of original tumor mass (white dotted line). (E-F) Large amounts of yellow cells were seen (EGFP+ mCherry+, arrowheads) at the tumor areas surrounding the MSC implantation site 24 hours post MSC implantation were observed. (G-H) Virus penetrating into the tumor at 48 hours with widespread cytopathic effect (black arrowheads), and ongoing rounds of tumor infection (white arrowheads). (I-J) Forefront of mCherry+ area extended to near tumor periphery at 72 hours leaving mCh+ cell debris behind. (K) High magnification photomicrograph showing infected GBM cells at varying stages of infection (L) H&E and X-gal staining of a brain section adjacent to the one shown in (E) to confirm that fluorescence imaging results correlate exactly with histological analysis. (M) Plot showing quantification of uninfected Gli36-vIII tumor cells (GFP+), oHSV-mCh infected MSC (mCherry+), and oHSV-mCh infected Gli36-vIII (GFP and mCherry+) at different times post treatment. Cells were counted and plotted relative to total cell number. *Bars,* +SD. Original magnifications: x4 (A-D), x10 (E, G, I, L), x20 (F, H, J), x60 (M)

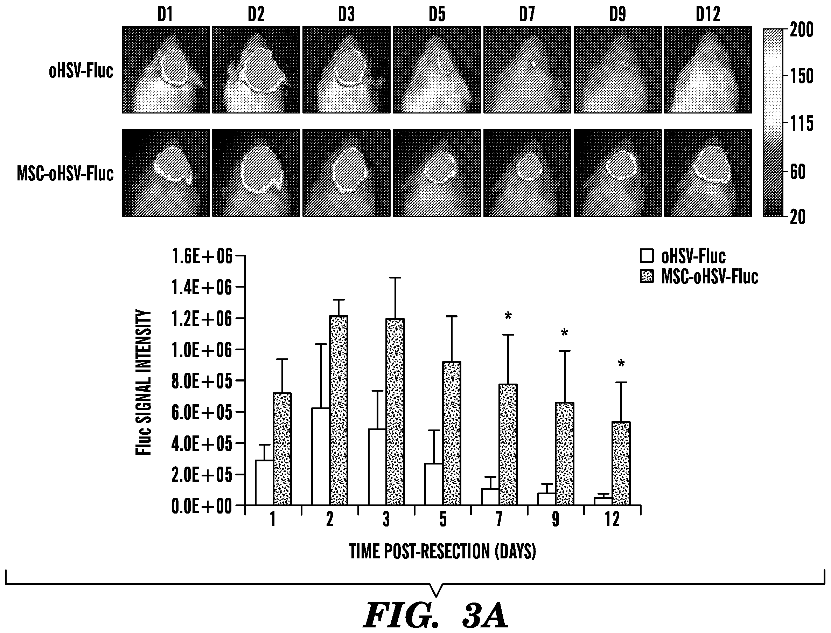

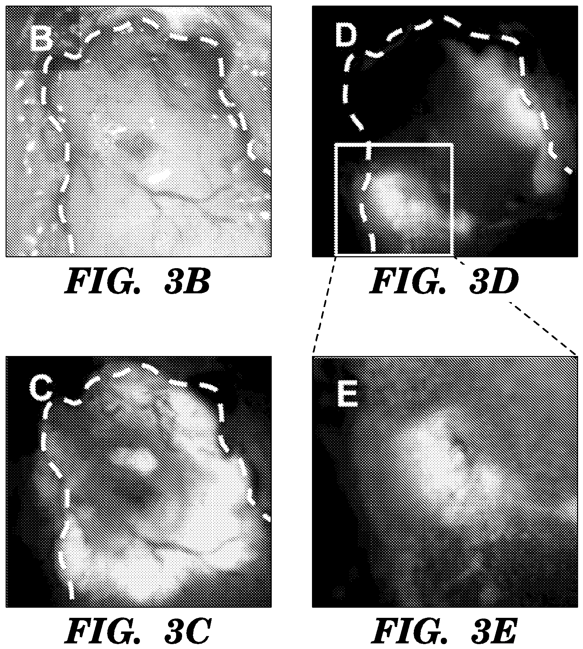

Figure 3A- Figure 3G are fluorescent images and graphs of experimental results that indicate sECM-encapsulated MSC loaded with oHSV significantly prolong the persistence of oHSV and increase survival time of mice in a clinically relevant GBM resection model. Mice bearing established intracranial Gli36vIII-GFP tumors were resected and treated with intracavitary injections of purified oHSV-Fluc or sECM-encapsulated MSC-oHSV-Fluc. (A) Plot showing changes in Fluc activity as a measure of virally infected cells monitored over time. A representative bioluminescence image from a mouse of each group and time is shown. (B) Light microscopic image of the brain after craniotomy. White dotted line showing the drilled rim around a cranial window. (C, D) Fluorescent microscopic images showing GFP+ intracerebral tumor visualized through the cranial window pre- (C) and post-resection (D). (E) Fluorescent image showing sECM encapsulated MSC-oHSV-mCh (red) implanted at close proximity to the residual GFP+ tumor cells. Original magnifications: x4 (B-D), x10 (E). (F) Plot showing Fluc signal as a measure of Gli36vIII-GFl tumor burden followed over time after intracavitary injections of purified oHSV-mCh or sECM-encapsulated MSC-oHSV-mCh. (G) Established intracranial Gli36vIII-GFl tumors were resected and treated with intracavitary injections of PBS, purified oHSV-mCh, or sECM-encapsulated MSC-oHSV-mCh. Kaplan-Meier survival curves of treated mice. p<0.05, MSC-oHSV-mCh versus oHSV-mCh and versus PBS. In all panels, *Bars,* +SE and *, p<0.05 versus controls.

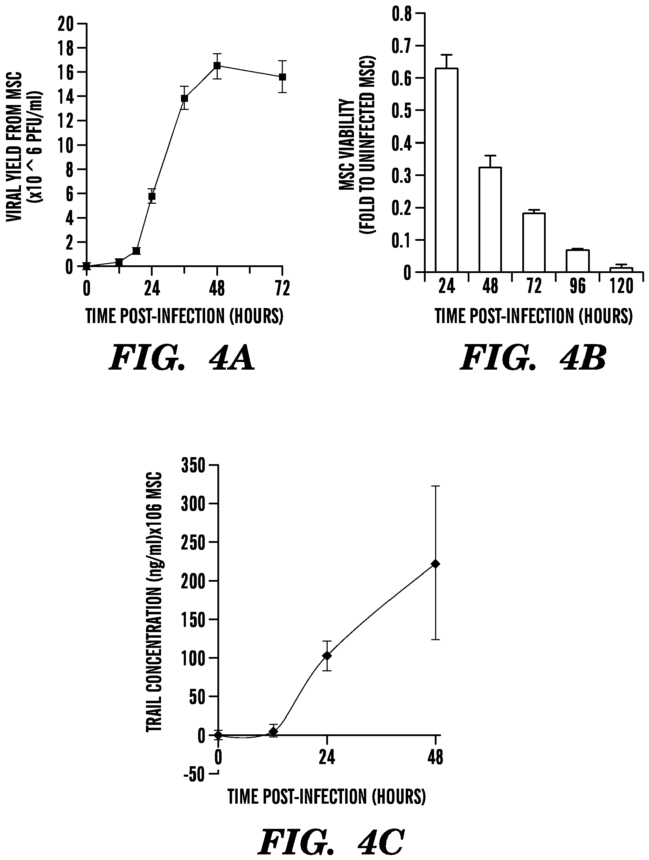

Figure 4A- Figure 4F are graphs and an image of a western blot of experimental results that indicate MSC loaded with oHSV-TRAIL target both oHSV- and TRAIL-resistant GBMs *in vitro.* MSC were infected with oHSV-TRAIL and followed over time for virus yield and MSC viability. (A) Plot showing viral yield of MSC-oHSV-TRAIL *in vitro* over time. (B) Plot showing survival of MSC-oHSV-TRAIL *in vitro* over time. (C) ELISA showing secretion of S-TRAIL from MSC-oHSV-TRAIL over time. (D-E) Co-culture assay of MSC, MSC-oHSV-mCh or MSC-oHSV-TRAIL with different fully or semi TRAIL resistant GBM lines (LN229, LN319, U138, U251) engineered to express GFP-Fluc. Plots showing tumor cell viability at day 3 (D) and caspase-3/7 activation in GBM lines at day 2 (E) show increased tumor cell killing and caspase activation by MSC-oHSV-TRAIL. (F) Western blotting analysis of TRAIL resistant LN229 cell lysate collected after 20 and 40 hours of incubation with MSC, MSC-oHSV-mCh or MSC-oHSV-TRAIL. In all panels, *Bars,* +SD and *, p<0.05 versus controls.

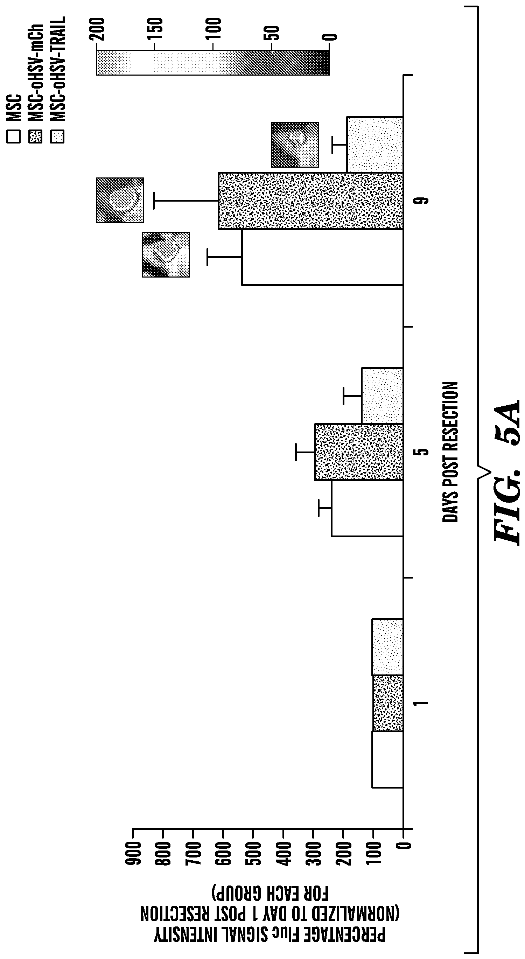

Figure 5A- Figure 5D are graphs and images of experimental results that indicate MSC loaded with oHSV-S-TRAIL target both oHSV- and TRAIL-resistant GBM *in vivo.* (A-D) Mice bearing established LN229-GFl intracranial tumors were resected and treated with sECM-encapsulated MSC-oHSV-TRAIL and controls. (A) Plot showing bioluminescence signal intensity over time with increased tumor suppression in the MSC-oHSV-TRAIL group against MSC and MSC-oHSV-mCh groups. A representative bioluminescence image from a mouse of each group at day 9 is shown. *Bars,* +SE. (B-C) Representative serial MR images showing tumor regression after MSC-oHSV-TRAIL treatment (1^{st} and 3^{rd} row) and tumor relapse after MSC-oHSV-mCh treatment (2^{nd} and 4^{th} row) in T2-weighted sequences (B) and T1-weighted sequences with contrast agent (C). White arrowheads in (B) point out edema caused by tumor growth, which persists in the treatment group. Despite no obvious tumor regrowth on day 15 (C), area with high T2 signal persisted after MSC-oHSV-TRAIL treatment (white arrowheads in B). (D) Kaplan-Meier survival curves of mice treated with sECM-encapsulated MSC-oHSV-mCh or MSC-oHSV-TRAIL. p<0.05, MSC-oHSV-TRAIL versus MSC-oHSV-mCh.

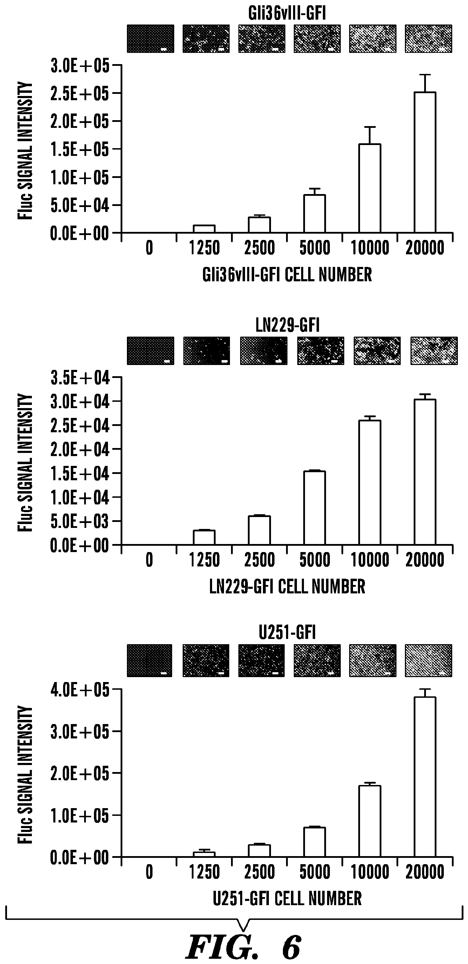

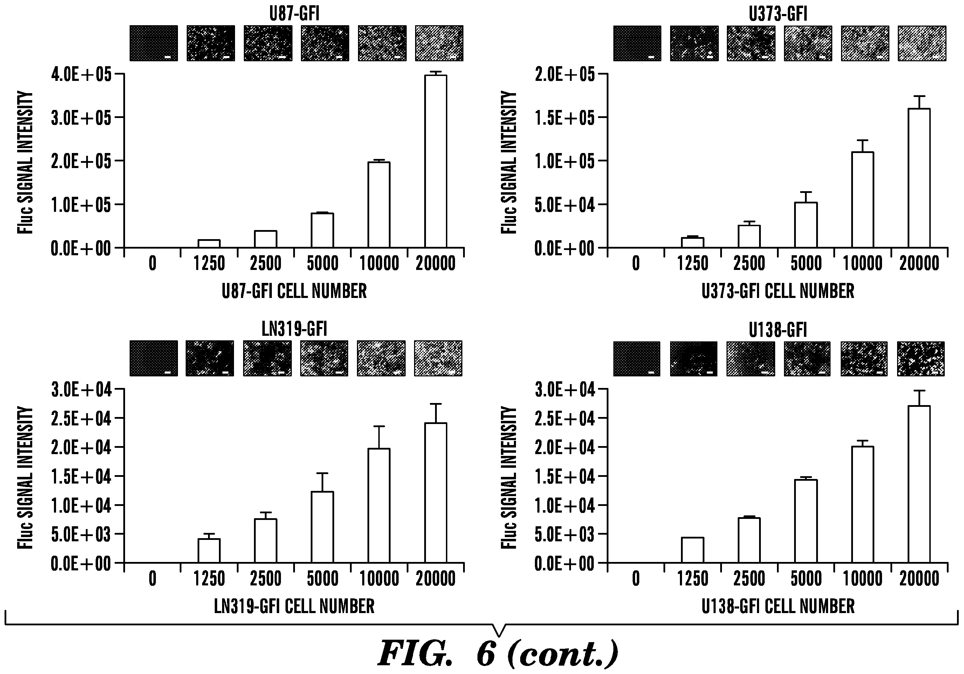

Figure 6 is a collection of bar graphs and corresponding images of experimental results that indicate expression of bimodal imaging transgenes using engineered lentiviral vectors. Serial dilutions of GBM cell lines expressing a bimodal fluorescent and bioluminescent fused protein GFP-firefly luciferase (Fluc) (GFl) were plated and 24 hours later Fluc signal intensity was determined. Plot showing a direct correlation between GBM-GFl cell number, Fluc signal intensity and GFP+ cells. *Bars,* +SD. Original magnifications: x10.

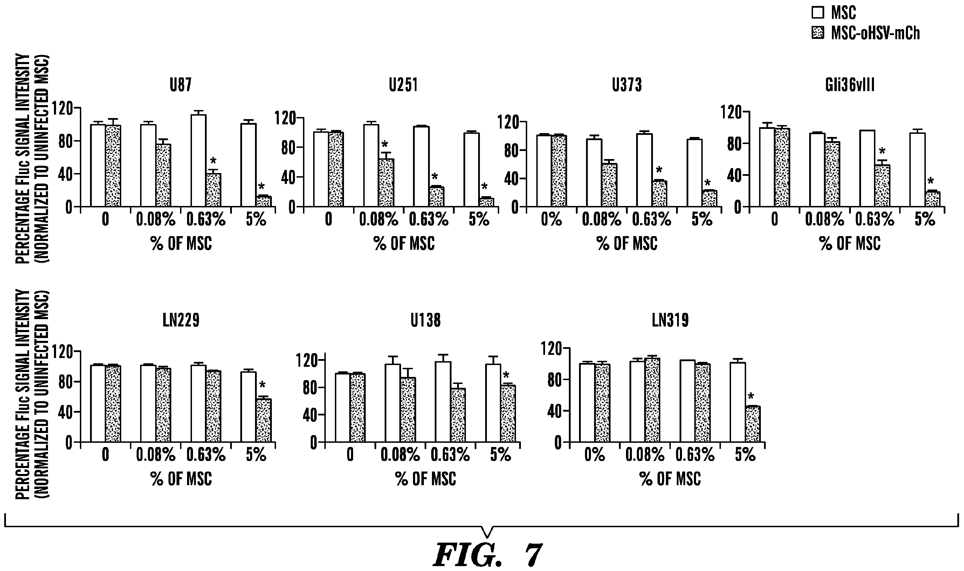

Figure 7 is a collection of bar graphs of experimental data that indicate efficacy of MSC to produce oHSV-mCh and reduce cell viability in different GBM lines. Different human GBM lines engineered to express GFl fusion marker were co-cultured with MSC or MSC-oHSV-mCh. Plots showing tumor cell viability in the oHSV sensitive (U87, U251, U373 and Gli36vIII) and oHSV resistant (LN229, U138 and LN319) GBM lines expressing GFl at day 3. In all panels, *Bars,* +SD. and *, p<0.05 versus controls.

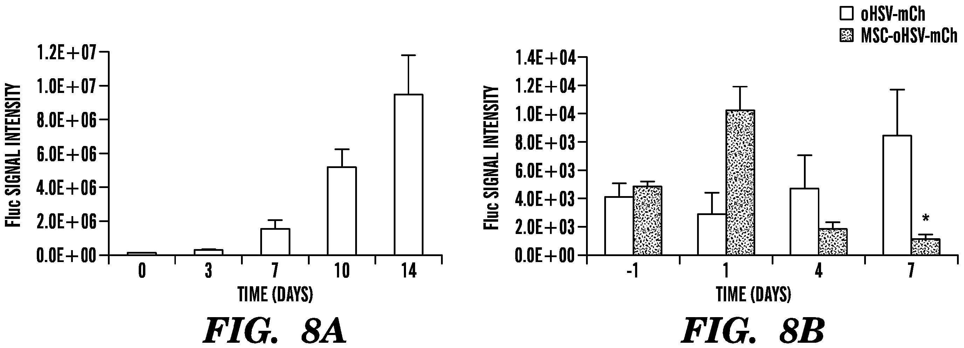

Figure 8 is a pair of bar graphs of experimental results that indicate *in vivo* growth dynamics of the highly proliferative GBM line Gli36vIII-GFI and therapeutic effect of direct injection of oHSV-mCh or MSC-oHSV-mCh on established Gli36vIII-GFI tumors. (A) Plot showing Fluc signal intensity indicating in *vivo* growth dynamics of the highly aggressive GBM line, Gli36vIII-GFI, over 14 days post implantation. (B) Mice with established Gli36vIII-GFI tumors were treated with either purified oHSV-mCh or MSC-oHSV-mCh. Plot showing bioluminescence Fluc signal intensity relative to untreated control over time. *p<0.05 versus purified oHSV. In all panels, *Bars,* +SE.

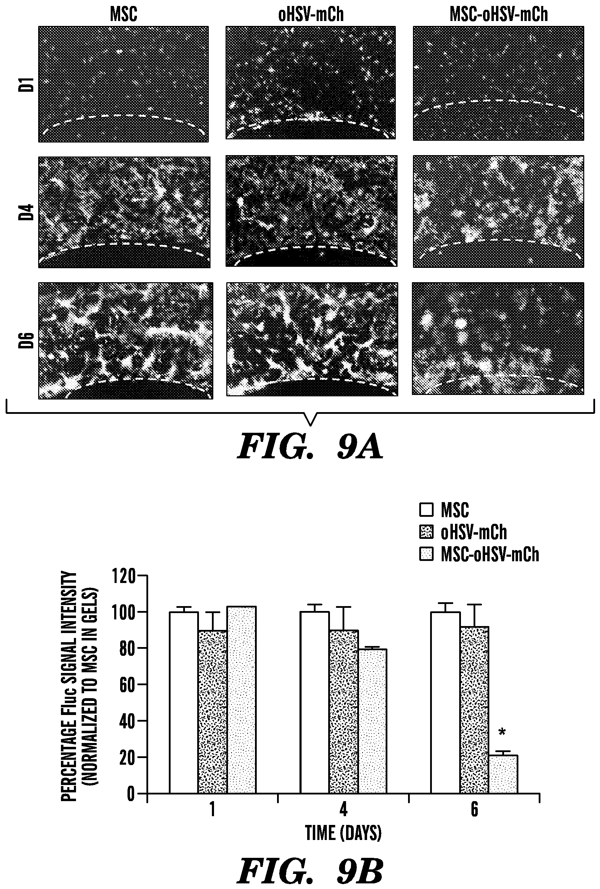

Figure 9A-Figure 9B is a collection of images and bar graphs of experimental results that indicate in *vitro* co-culture of sECM encapsulated MSC-oHSV-mCh with GBM cells (A-B) sECM encapsulated MSC, oHSV-mCh or MSC-oHSV-mCh were co-cultured with U87-GFl tumor cells. Photomicrographs of U87-GFl at day 1, 4 and 6 (A). Original magnifications: x4. Plot showing changes in Fluc activity as a measure of survival of U87-GFl tumor cells. (B). *Bars,* +SE. and *, p<0.05 versus controls.

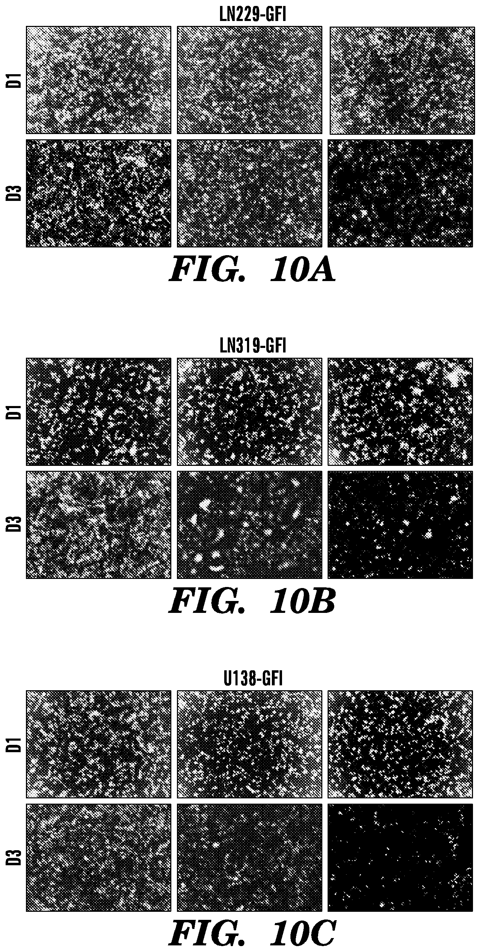

Figure 10A- Figure 10C is a collection of images of experimental results that indicate efficacy of MSC-oHSV-S-TRAIL to target both oHSV- and TRAIL-resistant GBM lines *in vitro.* Co-culture assay of MSC (left), MSC-oHSV-mCh (middle) or MSC-oHSV-TRAIL (right) with TRAIL resistant GBM lines LN229-GFl (A), LN319-GFl (B) and U138-GFl (C). Photomicrographs showing tumor cell viability (GFP+ cells) at day 1 and 3. Original magnifications: x4 (A-C).

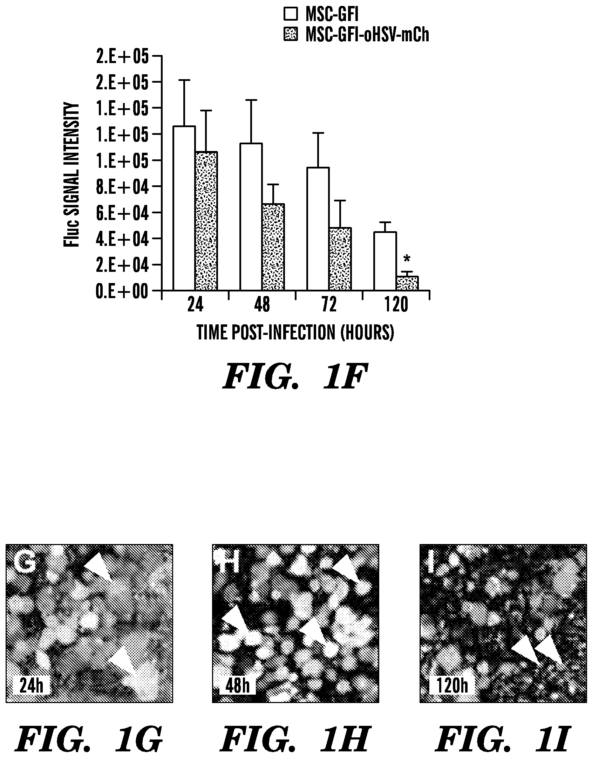

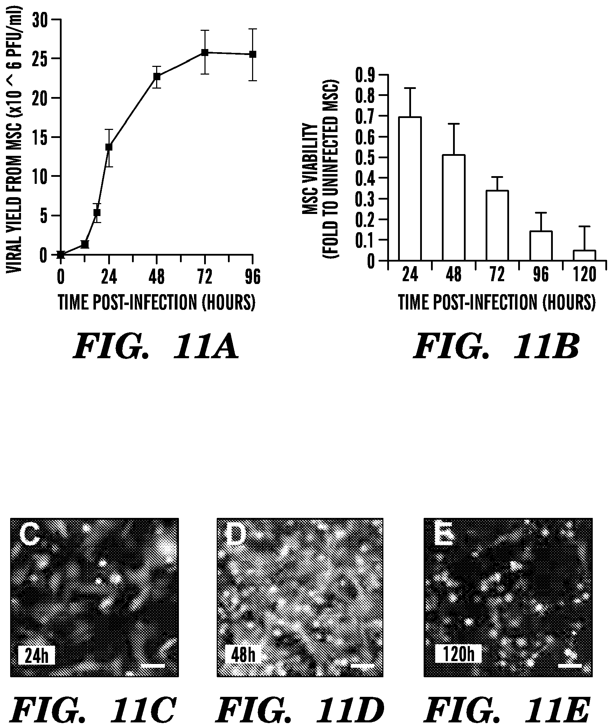

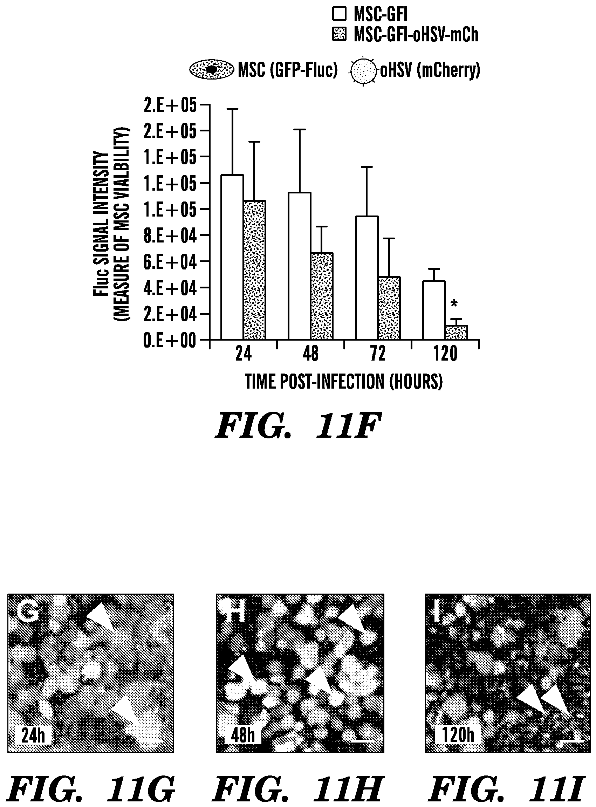

Figure 11A- Figure 11I are graphs and images of experimental results that indicate the fate of human mesenchymal Stem Cells loaded with oHSV *in vitro* and *in vivo.* MSC were infected with oHSV-mCh and followed over time for virus yield and MSC viability. (A) Plot showing oHSV yield in MSC *in vitro* over 4 days. *Bars*,+SD. (B) Plot showing survival of MSC-oHSV-mCh *in vitro. Bars*,+SD. (C-E) Photomicrographs of MSC-oHSV-mCh undergoing different stages of transgene expression (mCh) and cytopathic effect at 24 (C), 48 (D) and 120 (E) hours post infection. (F) MSC expressing firefly luciferase (Fluc) were loaded with oHSV-mCh and MSC survival was followed in mice brains for a period of 5 days. Fluc signal activity as a measure of MSC survival in mice is shown. *Bars,* +SD. (G-I) Photomicrographs of brain sections from mice after implantation of MSC-oHSV-mCh. Mice were sacrificed at 24 (G), 48 (H) and 120 (I) hours post-implantation respectively. Arrowheads show cells in the first stages of virus replication (G), cytopathic effect (H) and cell debris after virus mediated cell lysis (I). *Bars,* +SD. Sizing of scale bars: Figure 1C-E and 1G-I (100µm). In all panels, *, p<0.05 versus controls (t-test two-sided).

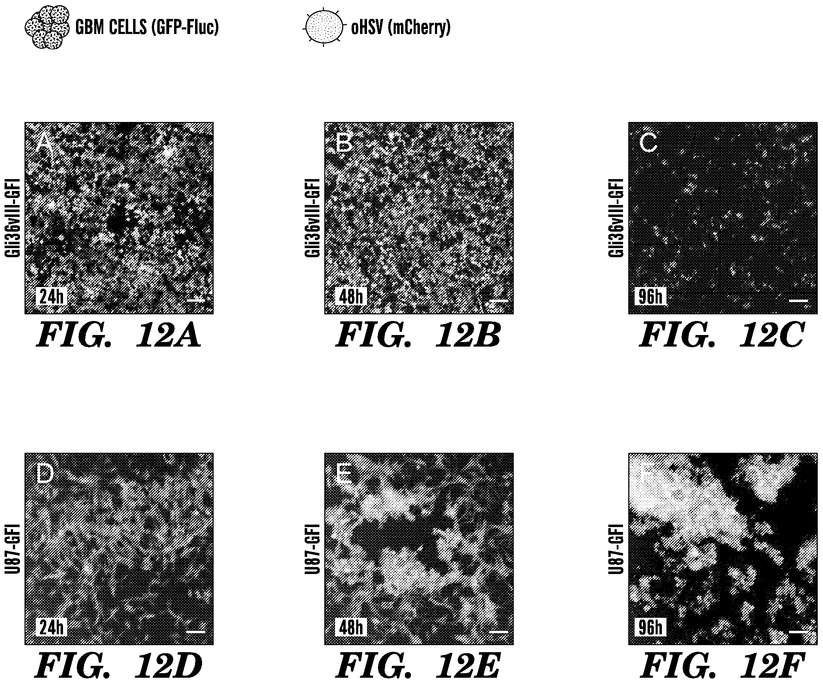

Figure 12A-Figure 12H are graphs and images of experimental results that indicate therapeutic efficacy of Human mesenchymal Stem Cells loaded with oHSV *in vitro* and *in vivo.* (A-G) Different human GBM lines engineered to express GFl fusion marker, Gli36vIII-GFl and U87-GFl, were co-cultured with 3% of MSC-oHSV-mCh, and oHSV spread and corresponding changes in their survival were followed. Photomicrographs of Gli36vIII-GFl (A-C) and U87-GFl (D-F) at 24 (A,D), 48 (B,E) and 96 (C,F) hours respectively are shown. Cells visualized as green represented GBM-GFl cells, cells visualized as red represented MSC-oHSV-mCh and cells visualized as yellow represented GBM-GFl cells which have been infected with oHSV-mCh released from MSC carrier cells. (G) Plot showing survival of Gli36vIII-GFl and U87-GFl after co-culture with MSC-oHSV-mCh. *Bars*,+SD. (H) Plot showing bioluminescence Fluc signal intensity as a measure of tumor volume changes in mice bearing intracerebral Gli36vIII-GFl tumors treated with MSC, oHSV-mCh or MSC-oHSV-mCh. *Bars,* +SD. Sizing of scale bars: Figure 12A-F (200µm). In all panels, *, p<0.05 versus controls, †, p<0.05 versus purified oHSV-mCh (t-test two-sided).

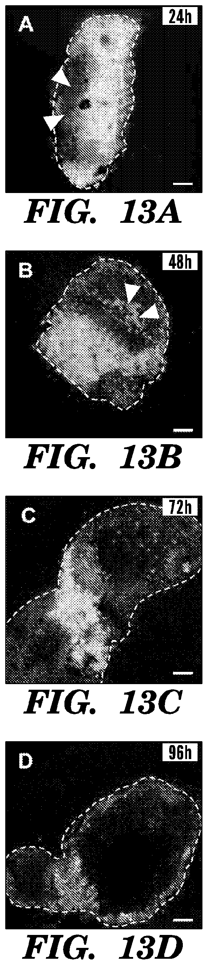

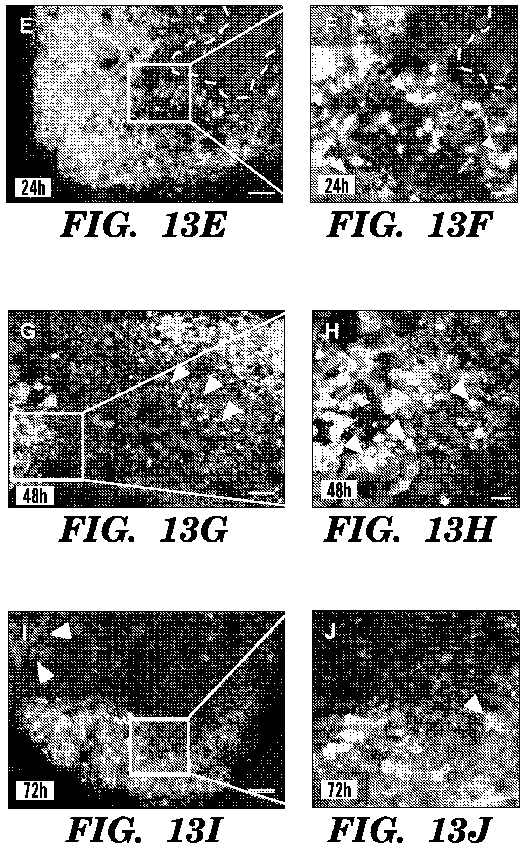

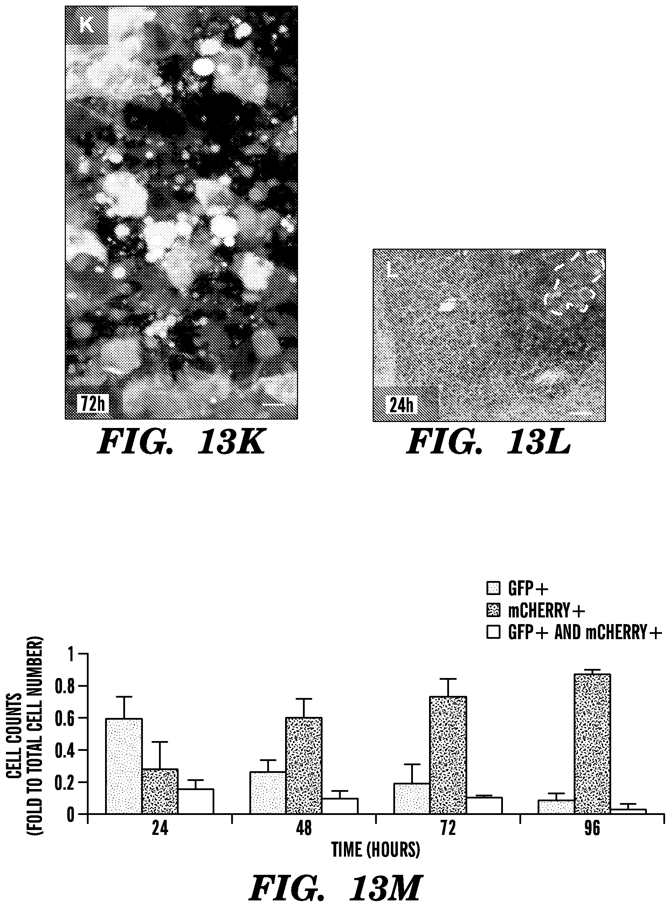

Figure 13A- Figure 13M are images and graphs of experimental results of fluorescent imaging to follow progression of viral oncolysis by MSC loaded oHSV-mCh. (A-D) Gli36vIII-GFl tumor bearing mice were treated with MSC-oHSV-mCh. Production of oHSV-mCh (mCh) in MSC and infection of Gli36vIII-GFl cells (GFP+mCh) were monitored over time. Low magnification sections showing viral infection (visualized as red) of tumor cells (visualized as green) over 4 days post MSC-oHSV-mCh implantation. Arrowheads show MSC-oHSV-mCh implantation site. Viral production in hMSC and infection of Gli36vIII-GFl resulted in almost entire destruction of original tumor mass (white dotted line). (E-F) Large amounts of yellow cells were seen (EGFP+ mCherry+, arrowheads) at the tumor areas surrounding the MSC implantation site 24 hours post MSC implantation were observed. (G-H) Virus penetrating into the tumor at 48 hours with widespread cytopathic effect (black arrowheads), and ongoing rounds of tumor infection (white arrowheads). (I-J) Forefront of mCherry+ area extended to near tumor periphery at 72 hours leaving mCh+ cell debris behind. (K) High magnification photomicrograph showing infected GBM cells at varying stages of infection (L) H&E and X-gal staining of a brain section adjacent to the one shown in (E) to confirm that fluorescence imaging results were associated exactly with histological analysis. (M) Plot showing quantification of uninfected Gli36-vIII tumor cells (GFP+), oHSV-mCh infected MSC (mCherry+), and oHSV-mCh infected Gli36-vIII (GFP and mCherry+) at different times post treatment. Cells were counted and plotted relative to total cell number. *Bars*,+SD. Sizing of scale bars: Figure 13A-D (200µm), Figure 13E, G, I, L (100µm), Figure 13F, H, J (50µm), Figure 13M (20µm).

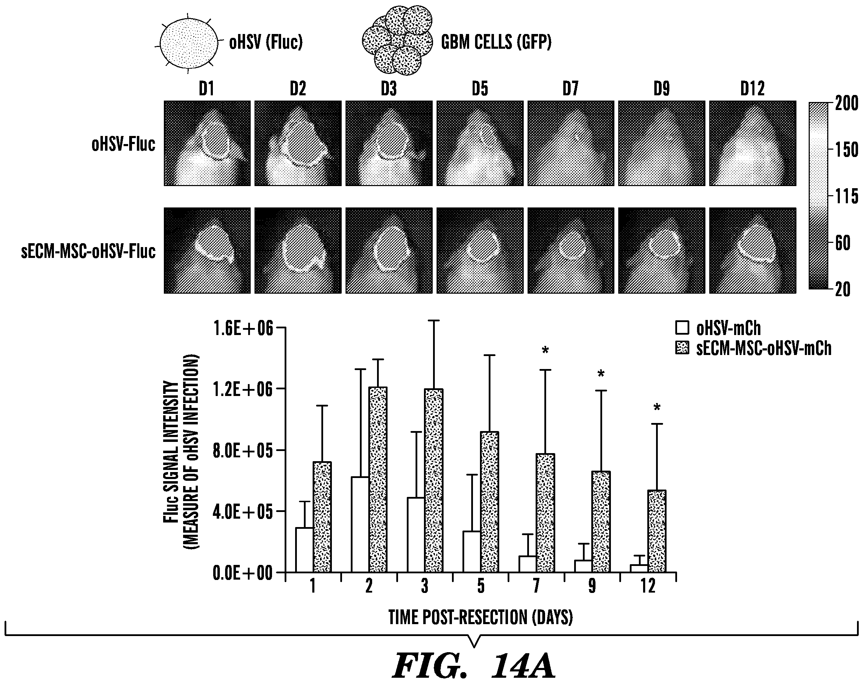

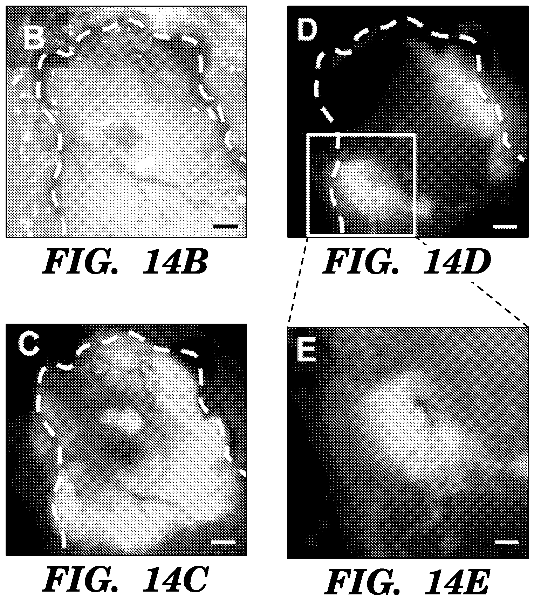

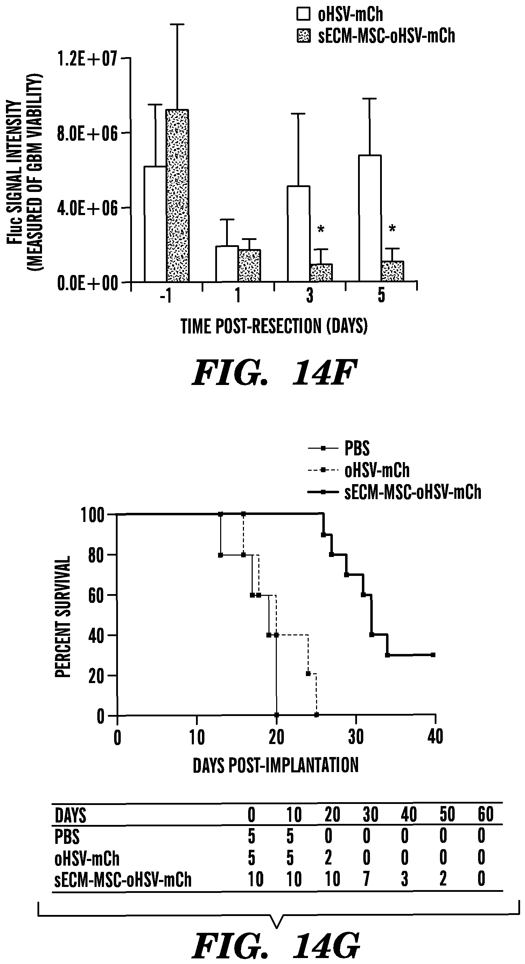

Figure 14A- Figure 14G are images, graphs, and a table of experimental results that indicate the fate and therapeutic efficacy of sECM-encapsulated MSC loaded with oHSV in a clinically relevant GBM resection model. Mice bearing established intracranial Gli36vIII-GFP tumors were resected and treated with intracavitary injections of purified oHSV-Fluc or sECM-encapsulated MSC-oHSV-Fluc. (A) Plot showing changes in Fluc activity as a measure of virally infected cells monitored over time. A representative bioluminescence image from a mouse of each group and time is shown (13,2 ± 1,82 times higher Fluc expression in the MSC-oHSV-Fluc group in comparison to purified oHSV-Fluc group) (B) Light microscopic image of the brain after craniotomy. White dotted line showing the drilled rim around a cranial window. (C,D) Fluorescent microscopic images showing GFP+ intracerebral tumor visualized through the cranial window pre- (C) and post-resection (D). (E) Fluorescent image showing sECM encapsulated MSC-oHSV-mCh (visualized as red) implanted at close proximity to the residual GFP+ tumor cells. Original magnifications: x4 (B-D), x10 (E). (F) Plot showing Fluc signal as a measure of Gli36vIII-GFl tumor burden followed over time after intracavitary injections of purified oHSV-mCh or sECM-encapsulated MSC-oHSV-mCh. (G) Established intracranial Gli36vIII-GFl tumors were resected and treated with intracavitary injections of PBS, purified oHSV-mCh, or sECM-encapsulated MSC-oHSV-mCh. Kaplan-Meier survival curves of treated mice. p<0.001 (Wilcoxon test), sECM-MSC-oHSV-mCh versus oHSV-mCh and versus PBS. The number of mice at risk is shown below the graph. In all panels, *Bars,* +SD and *, p<0.05 versus controls (t-test two-sided). Sizing of scale bars: Figure 14B-E (400µm),

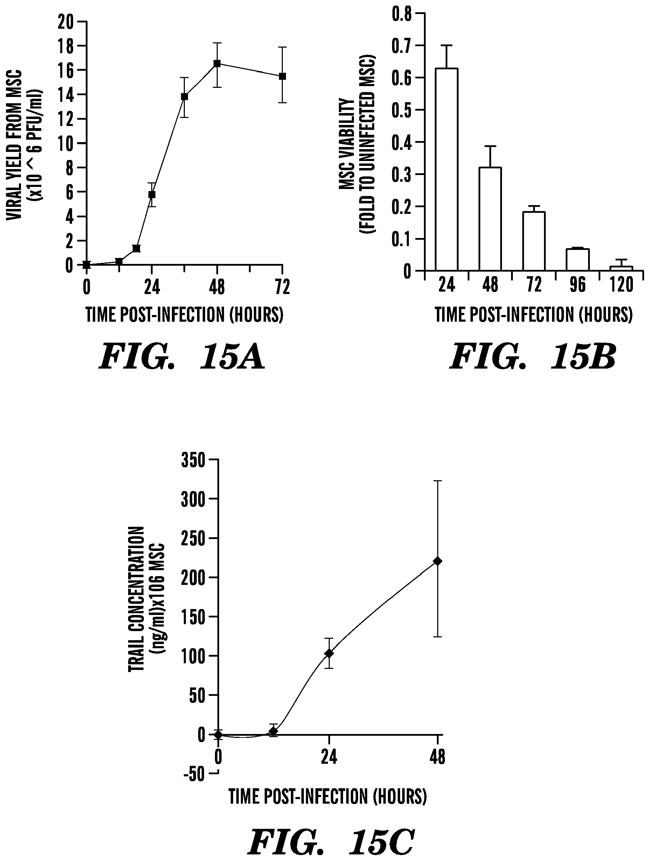

Figure 15A-Figure 15F are graphs and an image of a western blot showing experimental results that indicate the therapeutic efficacy of MSC loaded with oHSV-TRAIL in oHSV- and TRAIL-resistant GBMs *in vitro.* MSC were infected with oHSV-TRAIL and followed over time for virus yield and MSC viability. (A) Plot showing viral yield of MSC-oHSV-TRAIL *in vitro* over time. (B) Plot showing survival of MSC-oHSV-TRAIL *in vitro* over time. (C) ELISA showing secretion of S-TRAIL from MSC-oHSV-TRAIL over time. (D-E) Co-culture assay of MSC, MSC-oHSV-mCh or MSC-oHSV-TRAIL with different fully or semi TRAIL resistant GBM lines (LN229, LN319, U138, U251) engineered to express GFP-Fluc. Plots representing tumor cell viability at day 3 (D) and caspase-3/7 activation in GBM lines at day 2 (E) show increased tumor cell killing and caspase activation by MSC-oHSV-TRAIL. (F) Western blotting analysis of TRAIL resistant LN229 cell lysate collected after 20 and 40 hours of incubation with MSC, MSC-oHSV-mCh or MSC-oHSV-TRAIL. In all panels, *Bars*,+SD and *, p<0.05 versus controls (t-test two-sided).

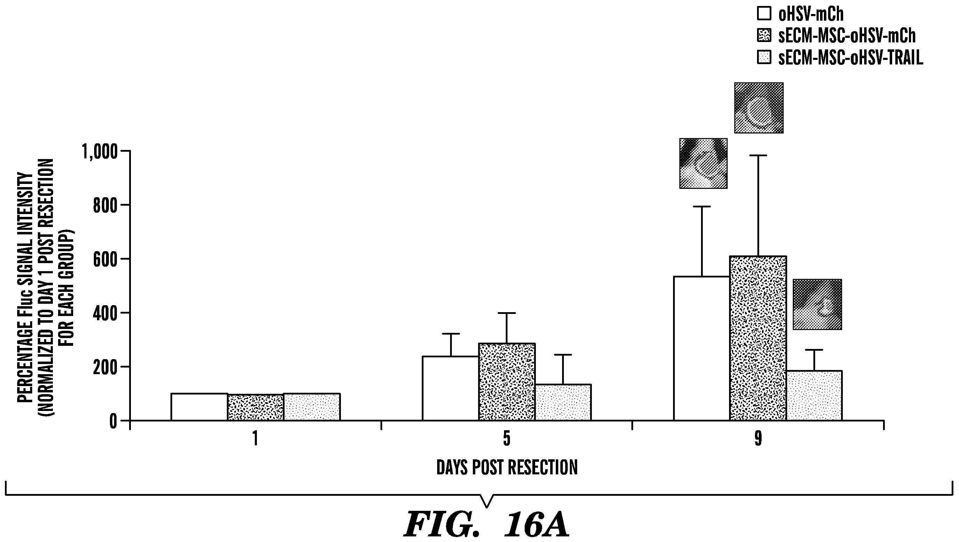

Figure 16A-Figure 16D are graphs, images and a table of experimental results that indicate therapeutic efficacy of sECM-MSC loaded with oHSV-S-TRAIL in oHSV- and TRAIL-resistant GBM *in vivo.* (A-D) Mice bearing established LN229-GFl intracranial tumors were resected and treated with sECM-encapsulated MSC, MSC-oHSV-mCh or MSC-oHSV-TRAIL. (A) Plot showing bioluminescence signal intensity over time with increased tumor suppression in the sECM-MSC-oHSV-TRAIL group against sECM-MSC and sECM-MSC-oHSV-mCh groups. A representative bioluminescence image from a mouse of each group at day 9 is shown. *Bars*,+SD. (B-C) Representative serial MR images showing tumor regression after sECM-MSC-oHSV-TRAIL treatment (1^{st} and 3^{rd} row) and tumor relapse after sECM-MSC-oHSV-mCh treatment (2^{nd} and 4^{th} row) in T2-weighted sequences (B) and T1-weighted sequences with contrast agent (C). White arrowheads in (B) point out edema caused by tumor growth, which persists in the treatment group. Despite no obvious tumor regrowth on day 15 (C), area with high T2 signal persisted after sECM-MSC-oHSV-TRAIL treatment (white arrowheads in B). (D) Kaplan-Meier survival curves of mice treated with sECM-encapsulated MSC-oHSV-mCh or MSC-oHSV-TRAIL (n=6 mice/group). p<0.001 (Chi-square contingency test), sECM-MSC-oHSV-TRAIL versus sECM-MSC-oHSV-mCh. The number of mice at risk is shown below the graph.

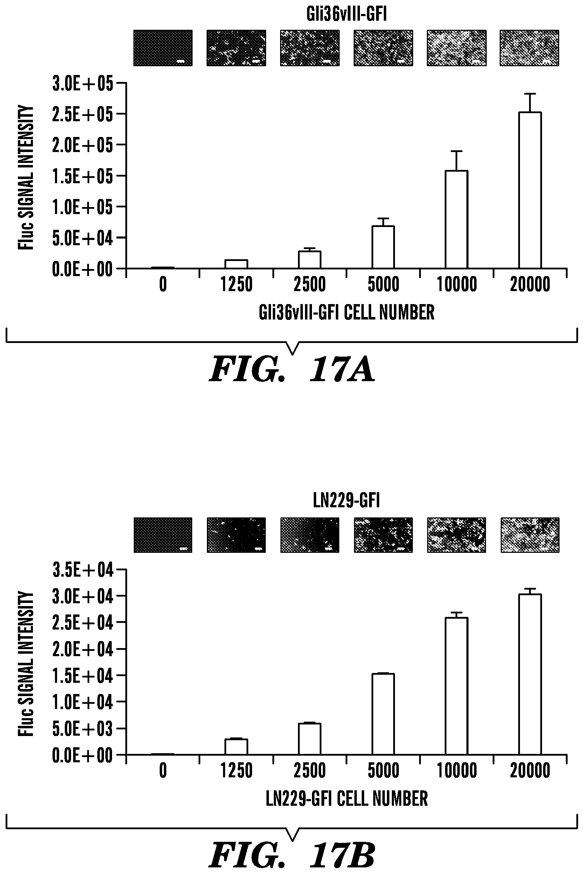

Figure 17A-Figure 17G are graphs and images of experimental results that indicate expression of bimodal imaging transgenes using engineered lentiviral vectors. Serial dilutions of GBM cell lines expressing a bimodal fluorescent and bioluminescent fused protein GFP-firefly luciferase (Fluc) (GFl) were plated and 24 hours later Fluc signal intensity was determined. Images show (A) Gli36vIII-GFI, (B) LN229-GFI, (C) U251-GFI, (D) U87-GFI, (E) LN319-GFI, (F) U-373-GFI, and (G) U138-GFI cells. Sizing of scale bars: Figure 17A-G (200µm), Bar graphs below the images show the mean +SD (standard deviation bar) of Fluc signal intensity. The data represents the mean of three replicates in a single experiment.

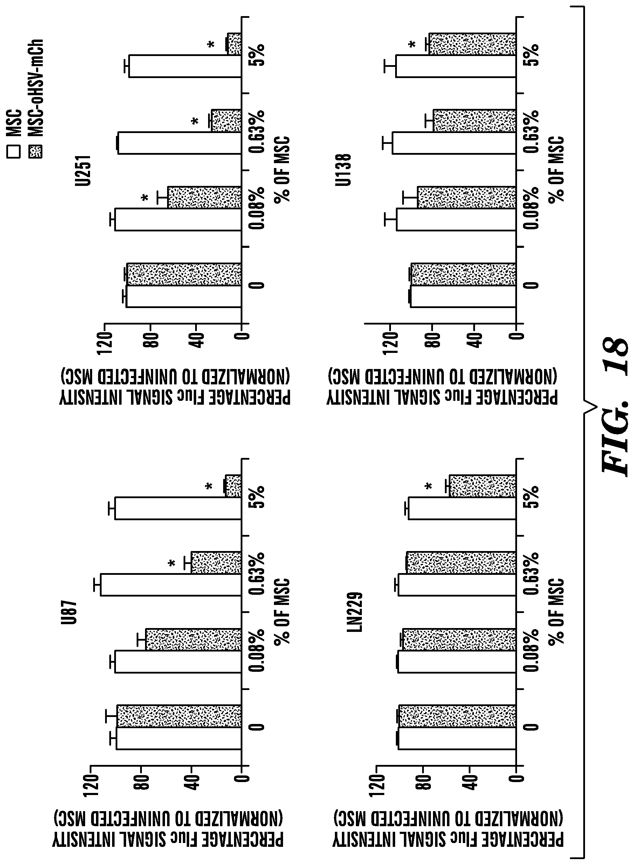

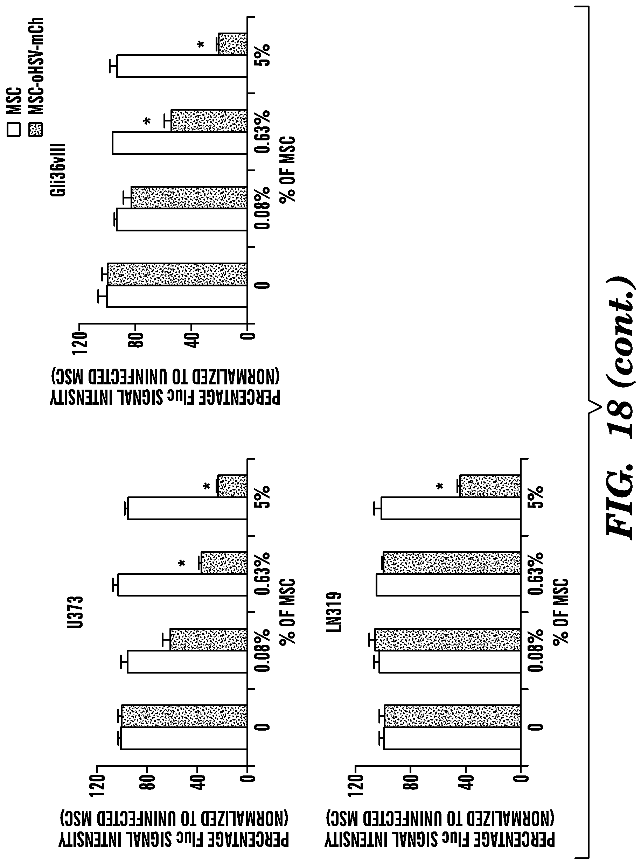

Figure 18 is a group of bar graphs of experimental results that indicate efficacy of MSC to produce oHSV-mCh and reduce cell viability in different GBM lines. Different human GBM lines engineered to express GFl fusion marker were co-cultured with MSC or MSC-oHSV-mCh. Plots showing tumor cell viability in the oHSV sensitive (U87, U251, U373 and Gli36vIII) and oHSV resistant (LN229, U138 and LN319) GBM lines expressing GFl at day 3. In all panels, *Bars*,+SD. (standard deviation bar) and *, p<0.05 versus controls (t-test two-sided). The data represents the mean of three replicates in a single experiment.

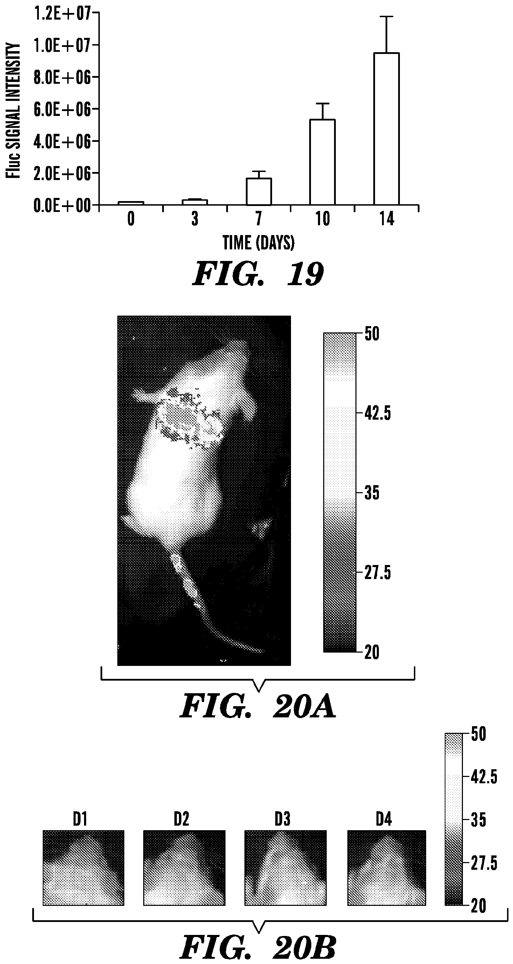

Figure 19 is a bar graph of experimental results that indicate *in vivo* growth dynamics of the highly proliferative GBM line Gli36vIII-GFI. Plot showing Fluc signal intensity indicating *in vivo* growth dynamics of the highly aggressive GBM line, Gli36vIII-GFI, over 14 days post implantation. *Bars*,+SD (t-test two-sided).

Figure 20A- Figure 20B is a set of images of experimental results from intravenous injection of MSC-FmC in tumor bearing mice. Intracranial Gli36-GFP tumor bearing mice were treated intravenously with MSC-FmC and the fate of MSC was determined over time. (A) Bioluminescence image from the brain of a representative mouse at different time points is shown. (B) Bioluminescence image from the representative whole mouse at day 1 is shown.

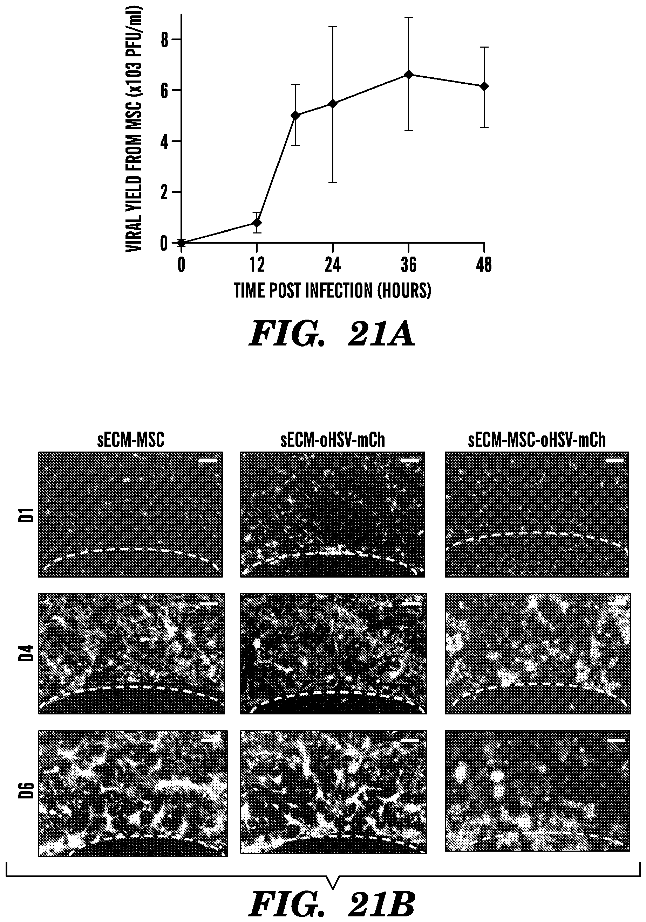

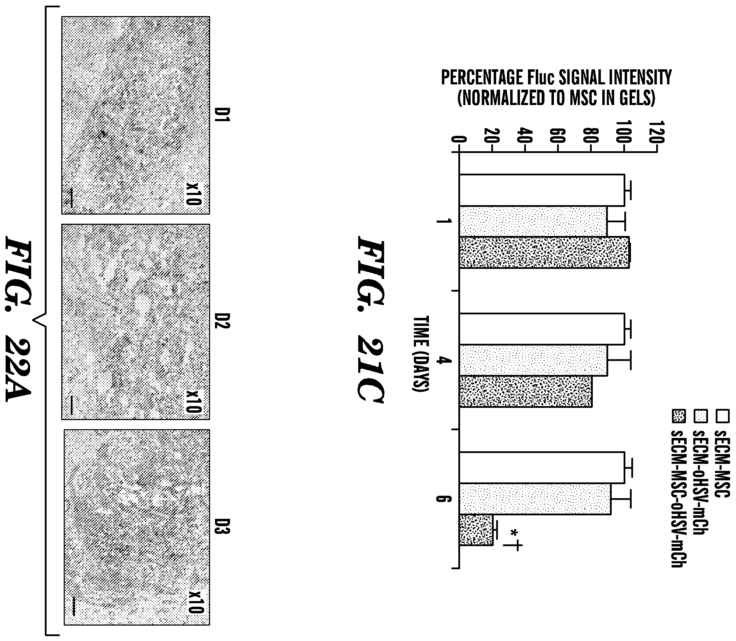

Figure 21A-Figure 21C is a set of graphs and images of experimental results that indicate *in vitro* viral production and antitumor effect of sECM encapsulated MSC-oHSV-mCh. MSC were infected with oHSV-mCh, encapsulated in sECM and followed over time for virus yield. (A) Plot showing viral yield of sECM-MSC-oHSV-mCh *in vitro* over time. (B-C) sECM encapsulated MSC, oHSV-mCh or MSC-oHSV-mCh were co-cultured with U87-GFl tumor cells. Photomicrographs of U87-GFl at day 1, 4 and 6 (B). Original magnifications: x4. Plot showing changes in Fluc activity as a measure of survival of U87-GFl tumor cells. (B). *Bars,* +SD, *, p<0.01 sECM-MSC-oHSV-mCh versus sECM-MSC, †, p<0.01 sECM-MSC-oHSV-mCh versus sECM-oHSV-mCh (t-test two-sided). The experiment represents the mean of three replicates in a single experiment.

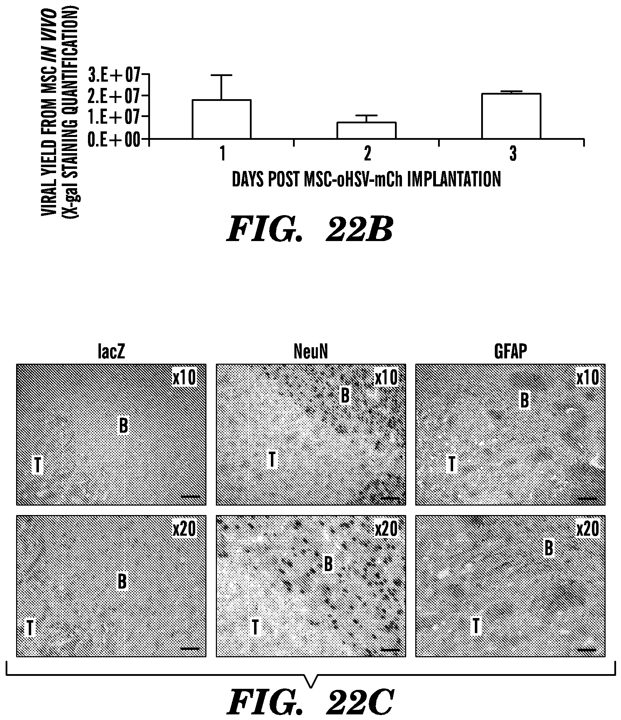

Figure 22A-Figure 22C is a collection of images and graphs of experimental results that indicate viral production and safety of MSC-oHSV-mCh *in vivo.* (A-C) Gli36vIII-GFl tumor bearing mice were treated with MSC-oHSV-mCh and viral production from MSC-oHSV-mCh was monitored by LacZ staining over time. (**A**) Photomicrographs showing X-gal staining in the tumor mass at different time points are shown. (**B**) Quantification of X-gal staining at day 1, 2 and 3 as a measure of viral yield *in vivo. Bars,* +SD. The experiment represents the mean of two replicates in a single experiment. (C) Brain sections from mice sacrificed at day 12 were stained for LacZ (measure of oHSV infection), NeuN (neuronal marker) or GFAP (astrocyte marker). Sizing of scale bars: Figure 22A (100µm), Figure 22C upper panels (100µm) and lower panels (50µm). T: tumor; B: brain.

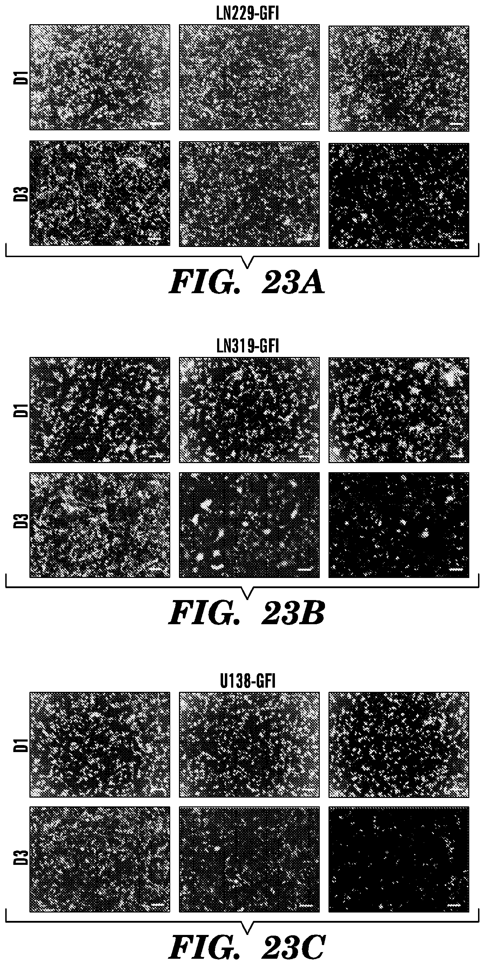

Figure 23A-Figure 23C is a set of images of experimental results that indicate efficacy of MSC-oHSV-S-TRAIL to target both oHSV- and TRAIL-resistant GBM lines *in vitro.* Co-culture assay of MSC (left), MSC-oHSV-mCh [6] or MSC-oHSV-TRAIL (right) with TRAIL resistant GBM lines LN229-GFl (A), LN319-GFl (B) and U138-GFl (C). Photomicrographs showing tumor cell viability (GFP+ cells) at day 1 and 3. The experiment was done in triplicates in a single experiment. Sizing of scale bars: Figure 23A-C (200µm).

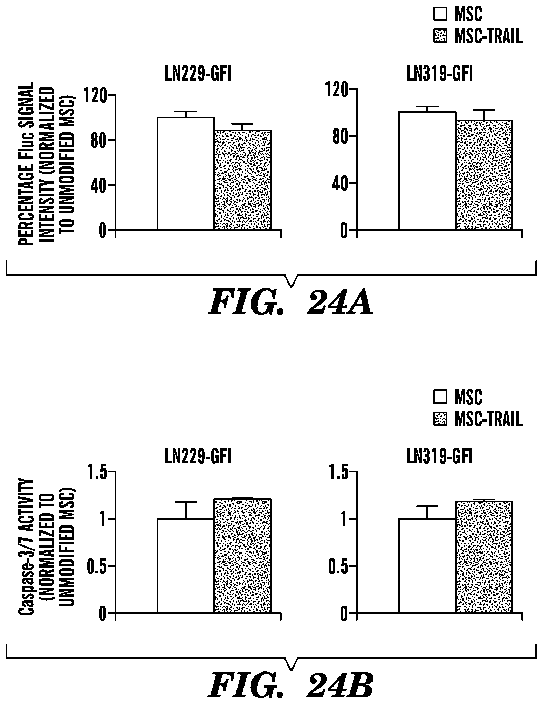

Figure 24A-Figure 24B is a set of bar graphs of experimental results that indicate the effect of MSC-TRAIL in TRAIL-resistant GBM lines *in vitro.* Co-culture assay of MSC or MSC-TRAIL with TRAIL-resistant GBM lines, LN229 and LN319, engineered to express GFP-Fluc. Plots representing tumor cell viability at day 3 (A) and caspase-3/7 activation in GBM lines at day 2 (B) do not show any tumor cell killing or caspase activation by MSC-TRAIL in TRAIL-resistant GBM lines. In all panels, *Bars*,+SD (t-test two-sided). The experiment represents the mean of three replicates in a single experiment.

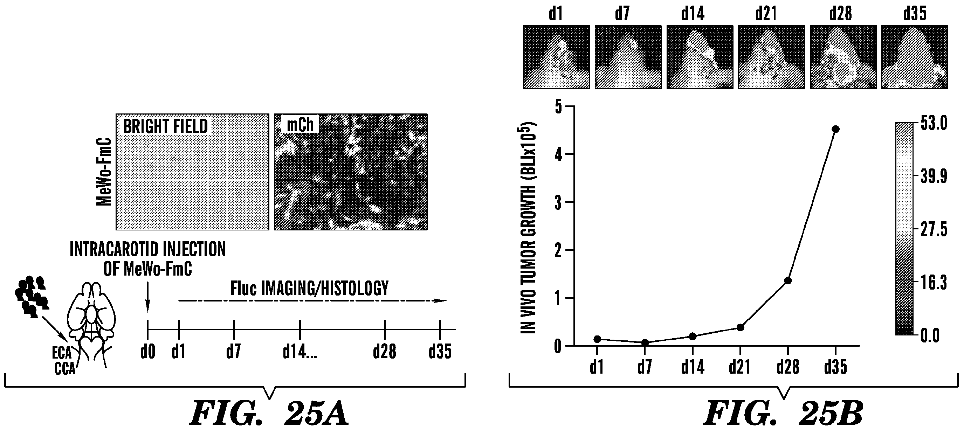

Figure 25A - Figure 25C are images and graphical representations of experimental results that indicate the viability of an in vivo imageable melanoma brain metastasis mouse model. A. Upper, representative images of MeWo-FmC cells in vitro. Lower, experiment outline. B. Representative bioluminescent images of MeWo-FmC tumor formed by intracarotid injection and plot showing the in vivo tumor growth of MeWo-FmC over time. C. Representative images of multiple melanoma brain metastatic foci and fluorescent images of metastatic foci in the brain (i and ii). Scale bar, 100um. iii and iv, photomicrograph of hematoxylin and eosin (H&E) staining of metastatic melanoma lesions in the brain and the melanoma cells with adjacent normal brain (NB). Scale bar, 200um (in iii) and 100um (in iv). v and vi, representative fluorescent images of GFAP or Ki67 immunostaining (visualized as green) on brain sections showing metastatic melanoma cells (mCherry+) surrounded by reactive astrocytes (GFAP+) and are proliferative (Ki67+). Scale bar, 100um.

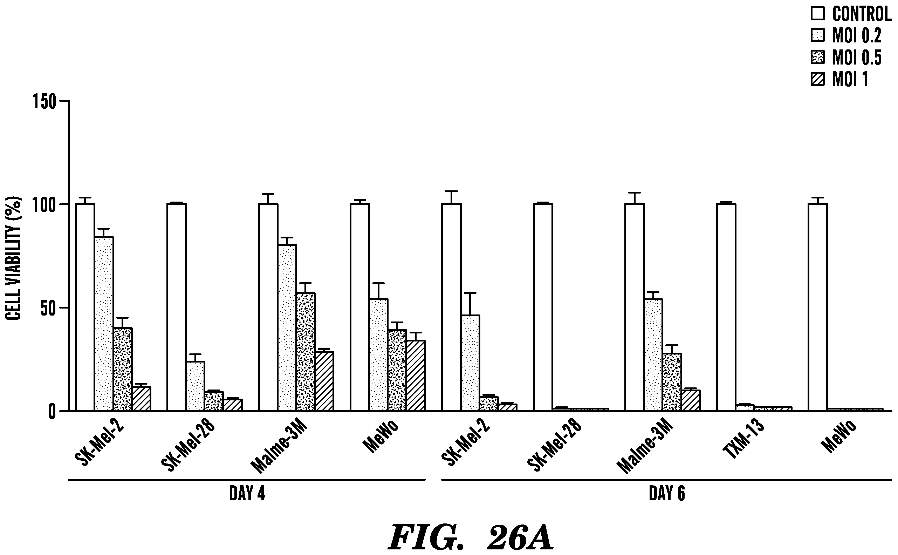

Figure 26A-Figure 26B are bar graphs and images of experimental results from the screening of melanoma lines for their sensitivities of oHSV infection and co-culture of melanoma cells with MST freshly loaded with oHSV. A. Cell viability of melanoma lines infected with oHSV at indicated MOI were analyzed at day 4 and day 6 post virus infection. B. Representative fluorescent images of melanoma cells co-cultured with MST freshly loaded with oHSV-mCh over time. Right panel, quantification of uninfected MeWo cells (GFP+), oHSV-mCh infected MST (mCherry+), and oHSV-mCh infected MeWo cells (GFP+ and mCherry+) at different time points post co-culture. Cells were counted and plotted relative to total cell numbers.

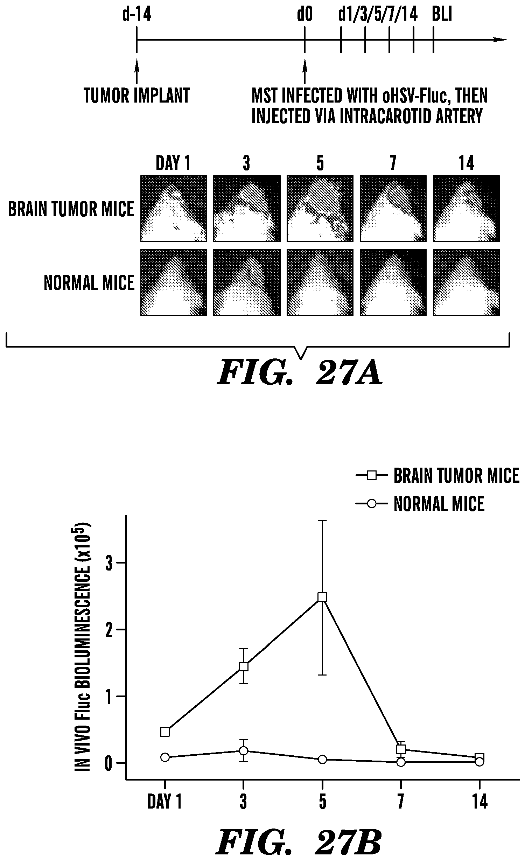

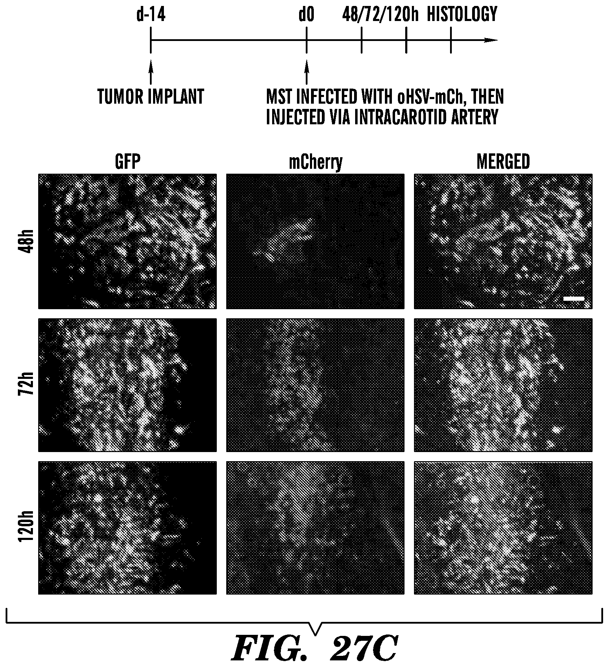

Figure 27A - Figure 27C are images and graphical representations of experimental results that indicate oHSV-mCh carried by MST can infect and amplify within tumor cells in the brain. A. Upper, outline of the experiment. Lower, representative bioluminescent images of the oHSV-Fluc injected in tumor bearing mice (brain tumor mice) or non-tumor mice (normal mice) respectively. B. Plot showing the in vivo bioluminescence of oHSV-Fluc from the two groups: brain tumor mice group and normal mice group. C. Upper, experiment outline. Lower, representative fluorescent images of MeWo-GFP and MST-oHSV-mCh in the brain at indicated time points post MST-oHSV-mCh injection. Scale bar, 100um.

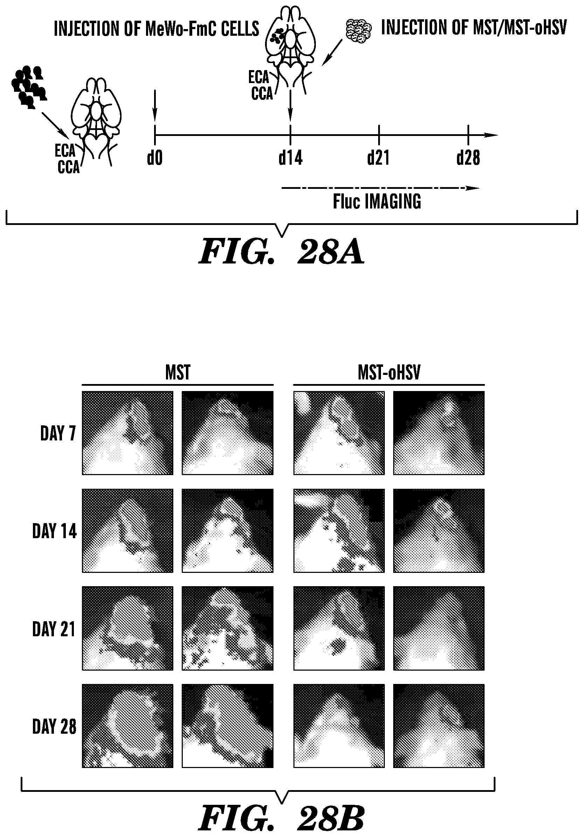

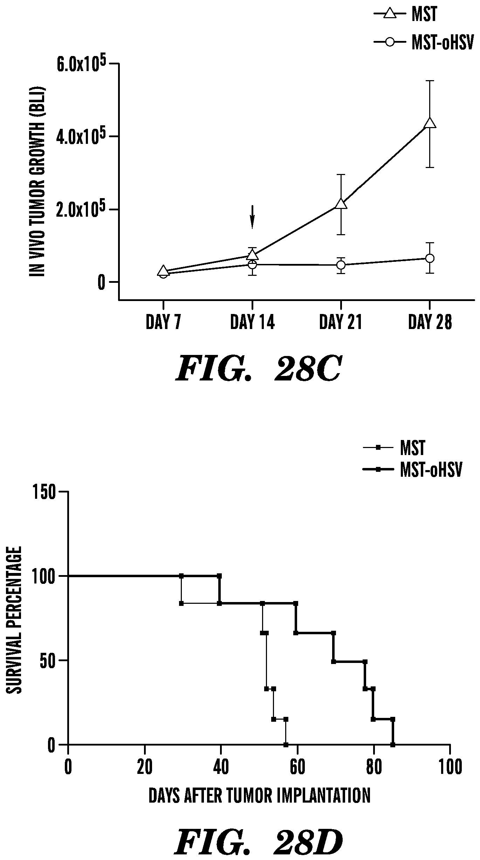

Figure 28A- Figure 28D are images and graphical representations of experimental results that indicate the in vivo therapeutic efficacy of oHSV delivered by MST in melanoma brain metastases mouse model. A. Outline of the experiment. B. Representative bioluminescent images of MST or MST loaded with oHSV injected in mice bearing melanoma brain metastases. C. Plot showing the in vivo bioluminescence of metastatic tumor growth from the two groups: MST treated group and MST-oHSV treated group. D. Kaplan-Meier survival curves of metastatic melanoma tumor bearing mice treated with MST or MST-oHSV via intracarotid injection.

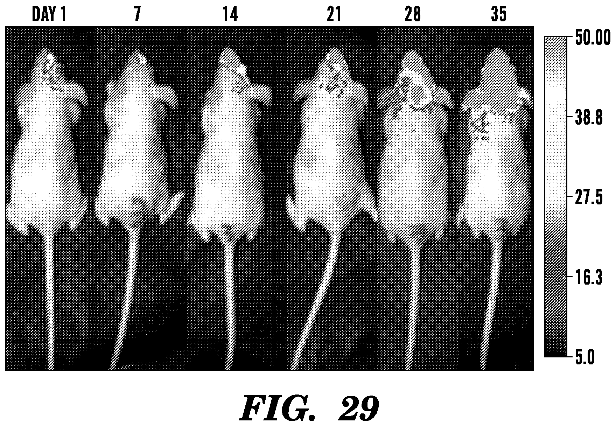

Figure 29 is an image of representative bioluminescent images of MeWo-FmC tumor formed by intracarotid injection, showing exclusive tumor growth within the mice brain.

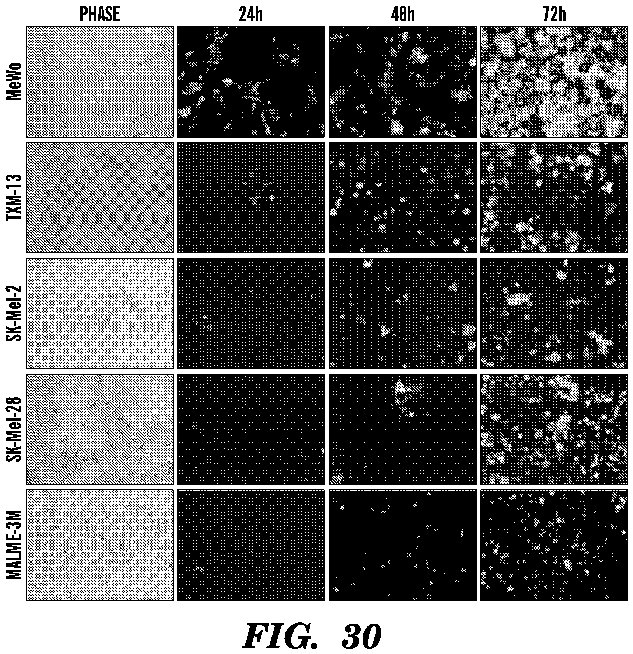

Figure 30 is a collection of images of representative phase images and fluorescent images of melanomal lines infected with oHSV-mCh at MOI 0.1 over time.

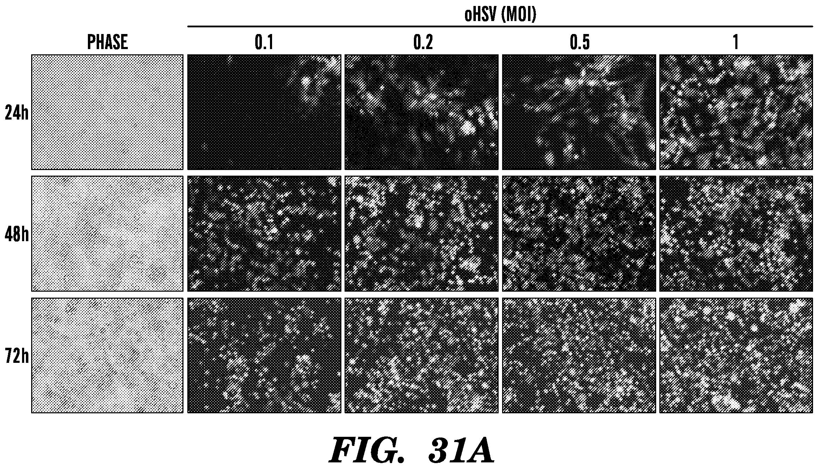

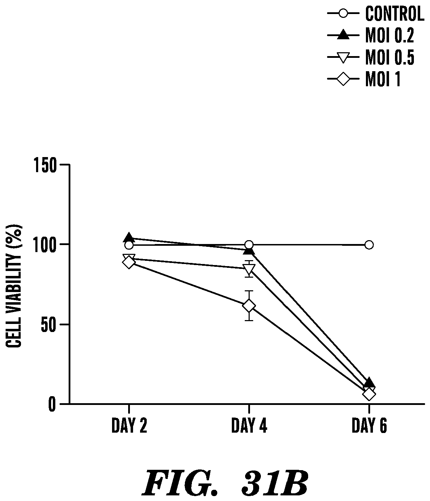

Figure 31A-Figure 31B is a collection of images and graphical representation of experimental results. A. Representative phase images and fluorescent images of MST cells infected with oHSV-mCh at different MOI over time. B. Cell viability of MST cells infected with oHSV at indicated MOI were analyzed at day 2, 4, and 6 post virus infection.

### DETAILED DESCRIPTION OF THE INVENTION

Despite their proven safety in clinical trials for highly malignant glioblastoma multiforme (GBM), the efficacy of oncolytic viruses is sub-optimal mainly due to the lack of sophisticated viral delivery methods, inability of virus to spread sufficiently within tumors and the low potency of conventional oncolytic viruses. Disclosed herein is evidence of the real time dynamics and therapeutic efficacy of human mesenchymal stem cells (MSC) loaded with oncolytic herpes simplex virus (oHSV) and its pro-apoptotic variant (oHSV-TRAIL) *in vitro* and *in vivo* mouse GBM models which mimic the more realistic clinical scenario of tumor aggressiveness and resection. Using bioluminescence and multi-color fluorescence imaging, it was shown that oHSV loaded MSC (MSC-oHSV) produce and release infectious oHSV, allow efficient GBM infection and subsequent killing *in vitro* and *in vivo,* and exert potent anti-GBM activity after direct injection to highly aggressive intracerebral GBM in mice. MSC-oHSV encapsulated in biocompatible synthetic extracellular matrix (sECM) resulted in a significant decrease in tumor growth as compared to direct injection of oHSV in the tumor resection cavity of a clinically relevant mouse model of GBM resection. To supersede oHSV resistant tumors, MSC were loaded with a novel oHSV variant, oHSV-TRAIL and it was shown that sECM encapsulated MSC-oHSV-TRAIL induces apoptosis in TRAIL and oHSV resistant GBM and significantly increases mice survival post-transplantation into the GBM tumor resection bed. This novel discovery of efficacy of MSC loaded with oHSV and its variants in mouse models that mimic the clinical scenario of GBM resection has direct clinical implications in different cancer types.

Aspects of the invention relate to a stem cell or population thereof that comprises recombinant oncolytic HSV as described herein. In one embodiment, the oHVS is infectious. In one embodiment, the stem cells produce the infectious oHSV. Although oHSV is known to infect cancer stem cells, that is not an aspect of this invention since the stem cells referred to herein are not cancer stem cells as the term is used in the art. In one embodiment, the stem cells are isolated. In one embodiment, the stem cells are isolated from a subject who is a cancer patient or is at risk for cancer (e.g., brain cancer). Typically the cells are engineered *in vitro* to contain the oHSV. As such, one aspect of the invention relates to *in vitro* stem cells or a population thereof, comprisng the oHSV, as described herein. Such stem cells are for use in the herein described methods.

### Stem Cells

Any stem cell type can be used in the instant invention. The two main stem cell types are embryonic stem cells (ES) cells and adult stem cells (i.e., somatic stem cells). Other types, such as induced pluripotent stem cells (iPSCs), are produced in the lab by reprogramming adult cells to express ES characteristics. The stem cells may be multipotent, pluripotent or totipotent. In one embodiment, the adult stem cells are mesenchymal stem cells. In one embodiment, the adult stem cells are tissue or organ specific stem cells such as neuronal stem cells, vascular stem cells, or epidermal stem cells. In one embodiemnt, the adult stem cells are mesenchymal stem cells (MSC). MSC can be obtained from a variety of sources such as bone marrow, umbilical cord blood , and adipose tissue. Common sources of stem cells are human umbilical vein endothelial cells (HUVEC), and primary human cutaneous microvascular endothelial cells (HCMEC). Analagous non-human stem cells can be obtained from similar non-human sources as well. The stem cells described herein are not considered to be cancer stem cells as the term is typically used in the art. Stem cells for use in the invention may be primary or cells that have been maintained in cell culture for an extended period. The stem cells may be obtained from any animal type (e.g., human).

In one embodiment, the stem cells are obtained or derived from a subject who is in need of thereapeutic treatment for a cell proliferative disorder in the brain (e.g., brain tumor or cancer). The subject may have the cell proliferative disorder or be at risk for the disorder.

### Oncolytic Herpes Simplex Virus (oHSV)

A variety of oHSV can be incorporated into the stem cells described herein. Oncolytic herpes simplex viruses (oHSV) are known in the art and are described, for example, in Kim et al. (1999, In: Gene Therapy of Cancer, Academic Press, San Diego, Calif., pp. 235-248), and include type 1 herpes simplex viruses and type 2 herpes simplex viruses. In one embodiment, the oHSV used in the compositions of the invention is replication-selective or replication-competent such as one of the examples described herein. In one embodiment, the oHSV is replication-incompetent.

Herpes simplex 1 type viruses are among the preferred viruses, for example the variant of HSV-1 viruses that do not express functional ICP34.5 and thus exhibit significantly less neurotoxicity than their wild type counterparts. Such variants include without limitation oHSV-R3616, one of the HSV-1 viruses described in Coukos et al., Gene Ther. Mol. Biol. 3:79-89 (1998), and Varghese and Rabkin, Cancer Gene Therapy 9:967-978 (2002). Other exemplary HSV-1 viruses include 1716, R3616, and R4009. Other replication selective HSV-1 virus strains that can be used include, e.g., R47Δ (wherein genes encoding proteins ICP34.5 and ICP47 are deleted), G207 (genes encoding ICP34.5 and ribonucleotide reductase are deleted), NV1020 (genes encoding UL24, UL56 and the internal repeat are deleted), NV1023 (genes encoding UL24, UL56, ICP47 and the internal repeat are deleted), 3616-UB (genes encoding ICP34.5 and uracil DNA glycosylase are deleted), G92A (in which the albumin promoter drives transcription of the essential ICP4 gene), hrR3 (the gene encoding ribonucleotide reductase is deleted), and R7041 (Us3 gene is deleted) and HSV strains that do not express functional ICP34.5.

oHSV for use in the methods and compositions described herein is not limited to one of the HSV-1 mutant strains described herein. Any replication-selective strain of a herpes simplex virus may be used. In addition to the HSV-1 mutant strains described herein, other HSV-1 mutant strains that are replication selective have been described in the art. Furthermore, HSV-2, mutant strains such as, by way of example, HSV-2 strains 2701 (RL gene deleted), Delta RR (ICP10PK gene is deleted), and FusOn-H2 (ICP10 PK gene deleted) can also be used in the methods and compositions described herein.

Non-laboratory strains of HSV can also be isolated and adapted for use in the invention (U.S. Patent No. 7,063,835). Furthermore, HSV-2 mutant strains such as, by way of example, HSV-2 strains HSV-2701, HSV-2616, and HSV-2604 may be used in the methods of the invention.

In one embodiment, the oHSV is G47Δ. G47Δ is a third generation virus, which contains 1) a mutation of ICP6, which targets viral deletion to tumor cells, 2) a deletion of γ34.5, which encodes ICP34.5 and blocks eIF2α phosphorylation and is the major viral determinant of neuropathogenicity, and 3) an additional deletion of the ICP47 gene and US11 promoter, so that the late gene US11 is now expressed as an immediate-early gene and able to suppress the growth inhibited properties of y34.5 mutants. Deletion of ICP47 also abrogates HSV-1 inhibition of the transporter associated with antigen presentation and MHC class 1 downregulation (Todo et al., Proc. Natl. Acad. Sci. USA, 98:6396-6401(2001)).

In one embodiment, the oHSV will comprise one or more exogenous nucleic acids encoding for one or more of the polypeptides described herein. Methods of generating an oHSV comprising such an exogenous nucleic acid are known in the art. The specific position of insertion of the nucleic acid into the oHSV genome can be determined by the skilled practitioner.

In one embodiment, the oHSV is replication-selective or replication-competent. In one embodiment, the oHSV is replication-incompetent.

The oHSV useful in the present methods and compositions are, in some embodiments, replication-selective. It is understood that an oncolytic virus may be made replication-selective if replication of the virus is placed under the control of a regulator of gene expression such as, for example, the enhancer/promoter region derived from the 5'-flank of the albumin gene (e.g. see Miyatake et al., 1997, J. Virol. 71:5124-5132). By way of example, the main transcriptional unit of an HSV may be placed under transcriptional control of the tumor growth factor-beta (TGF-β) promoter by operably linking HSV genes to the TGF-β promoter. It is known that certain tumor cells overexpress TGF-β, relative to non-tumor cells of the same type. Thus, an oHSV wherein replication is subject to transcriptional control of the TGF-β promoter is replication-selective, in that it is more capable of replicating in the certain tumor cells than in non-tumor cells of the same type. Similar replication-selective oHSV may be made using any regulator of gene expression which is known to selectively cause overexpression in an affected cell. The replication-selective oHSV may, for example, be an HSV-1 mutant in which a gene encoding ICP34.5 is mutated or deleted.

An oHSV in accordance with the present invention can further comprise other modifications in its genome. For example, it can comprise additional DNA inserted into the UL44 gene. This insertion can produce functional inactivation of the UL44 gene and the resulting lytic phenotype, or it may be inserted into an already inactivated gene, or substituted for a deleted gene. In one embodiment, the oHSV for use in the invention is under the control of an exogenously added regulator such as tetracycline (U.S. Patent No. 8,236,941), such as by engineering the virus to have a tetracycline inducible promoter driving expression of ICP27.

The oHSV may also have incorporated therein one or more promoters that impart to the virus an enhanced level of tumor cell specificity. In this way, the oHSV may be targeted to specific tumor types using tumor cell-specific promoters. The term "tumor cell-specific promoter" or "tumor cell-specific transcriptional regulatory sequence" or "tumor-specific promoter" or "tumor-specific transcriptional regulatory sequence" indicates a transcriptional regulatory sequence, promoter and/or enhancer that is present at a higher level in the target tumor cell than in a normal cell.

In one embodiment, the oHSV of the invention is engineered to place at least one viral protein necessary for viral replication under the control of a tumor-specific promoter. Or, alternatively a gene (a viral gene or exogenous gene) that encodes a cytotoxic agent can be put under the control of a tumor-specific promoter. By cytotoxic agent as used here is meant any protein that causes cell death. For example, such would include ricin toxin, diphtheria toxin, or truncated versions thereof. Also, included would be genes that encode prodrugs, cytokines, or chemokines. Such viral vectors may utilize promoters from genes that are highly expressed in the targeted tumor such as the epidermal growth factor receptor promoter (EGFR) or the basic fibroblast growth factor (bFGF) promoter, or other tumor associated promoters or enhancer elements.

### TRAIL

The oHSV virus for use in the invention may comprise a nucleic acid sequence that encodes TRAIL, or a biologically active fragment thereof, incorporated into the virus genome in expressible form. As such the oHSV serves as a vector for delivery of TRAIL to the infected cells. The use of various forms of TRAIL in the invention are envisioned, such as those described herein, including without limitation, a secreted form of TRAIL or a functional domain thereof (e.g., a secreted form of the extracellular domain), multimodal TRAIL, or a therapeutic TRAIL module, therapeutic TRAIL domain (e.g., the extracellular domain) or therapeutic TRAIL variant (examples of each of which are described in WO2012/106281), and also fragments, variants and derivatives of these, and fusion proteins comprising one of these TRAIL forms such as described herein.

TRAIL is normally expressed on both normal and tumor cells as a noncovalent homotrimeric type-II transmembrane protein (memTRAIL). In addition, a naturally occurring soluble form of TRAIL (solTRAIL) can be generated due to alternative mRNA splicing or proteolytic cleavage of the extracellular domain of memTRAIL and thereby still retaining tumor-selective pro-apoptotic activity. TRAIL utilizes an intricate receptor system comprising four distinct membrane receptors, designated TRAIL-R1, TRAIL-R2, TRAIL-R3 and TRAIL-R4. Of these receptors, only TRAIL-R1 and TRAIL-2 transmit an apoptotic signal. These two receptors belong to a subgroup of the TNF receptor family, the so-called death receptors (DRs), and contain the hallmark intracellular death domain (DD). This DD is critical for apoptotic signaling by death receptors (J. M. A. Kuijlen et al., Neuropathology and Applied Neurobiology, 2010 Vol. 36 (3), pp.168-182).

Apoptosis is integral to normal, physiologic processes that regulate cell number and results in the removal of unnecessary or damaged cells. Apoptosis is frequently dysregulated in human cancers, and recent advancements in the understanding of the regulation of programmed cell death pathways has led to the development of agents to reactivate or activate apoptosis in malignant cells. This evolutionarily conserved pathway can be triggered in response to damage to key intracellular structures or the presence or absence of extracellular signals that provide normal cells within a multicellular organism with contextual information.

Without meaning to be bound by theory, TRAIL activates the "extrinsic pathway" to apoptosis by binding to TRAIL-R1 and/or TRAIL-R2, whereupon the adaptor protein Fas-associated death domain and initiator caspase-8 are recruited to the DD of these receptors. Assembly of this "death-inducing signaling complex" (DISC) leads to the sequential activation of initiator and effector caspases, and ultimately results in apoptotic cell death. The extrinsic apoptosis pathway triggers apoptosis independently of p53 in response to pro-apoptotic ligands, such as TRAIL. TRAIL-R1 can induce apoptosis after binding non-cross-linked and cross-linked sTRAIL. TRAIL-R2 can only be activated by cross-linked sTRAIL. Death receptor binding leads to the recruitment of the adaptor FADD and initiator procaspase-8 and 10 to rapidly form the DISC. Procaspase-8 and 10 are cleaved into its activated configuration caspase-8 and 10. Caspase-8 and 10 in turn activate the effector caspase-3, 6 and 7, thus triggering apoptosis.

In certain cells, the execution of apoptosis by TRAIL further relies on an amplification loop via the "intrinsic mitochondrial pathway" of apoptosis. The mitochondrial pathway of apoptosis is a stress-activated pathway, e.g., upon radiation, and hinges on the depolarization of the mitochondria, leading to release of a variety of pro-apoptotic factors into the cytosol. Ultimately, this also triggers effector caspase activation and apoptotic cell death. This mitochondrial release of pro-apoptotic factors is tightly controlled by the Bcl-2 family of pro- and anti-apoptotic proteins. In the case of TRAIL receptor signaling, the Bcl-2 homology (BH3) only protein 'Bid' is cleaved into a truncated form (tBid) by active caspase-8. Truncated Bid subsequently activates the mitochondrial pathway.

TRAIL-R3 is a glycosylphosphatidylinositol-linked receptor that lacks an intracellular domain, whereas TRAIL-R4 only has a truncated and non-functional DD. The latter two receptors are thought, without wishing to be bound or limited by theory, to function as decoy receptors that modulate TRAIL sensitivity. Evidence suggests that TRAIL-R3 binds and sequesters TRAIL in lipid membrane microdomains. TRAIL-R4 appears to form heterotrimers with TRAIL-R2, whereby TRAIL-R2-mediated apoptotic signaling is disrupted. TRAIL also interacts with the soluble protein osteoprotegerin.

Diffuse expression of TRAIL has been detected on liver cells, bile ducts, convoluted tubules of the kidney, cardiomyocytes, lung epithelia, Leydig cells, normal odontogenic epithelium, megakaryocytic cells and erythroid cells. In contrast, none or weak expression of TRAIL was observed in colon, glomeruli, Henle's loop, germ and Sertoli cells of the testis, endothelia in several organs, smooth muscle cells in lung, spleen and in follicular cells in the thyroid gland. TRAIL protein expression was demonstrated in glial cells of the cerebellum in one study. Vascular brain endothelium appears to be negative for TRAIL-R1 and weakly positive for TRAIL-R2. With regard to the decoy receptors, TRAIL-R4 and TRAIL-R3 have been detected on oligodendrocytes and neurons.

TRAIL-R1 and TRAIL-R2 are ubiquitously expressed on a variety of tumor types. In a study on 62 primary GBM tumor specimens, TRAIL-R1 and TRAIL-R2 were expressed in 75% and 95% of the tumors, respectively. Of note, a statistically significant positive association was identified between agonistic TRAIL receptor expression and survival. Highly malignant tumors express a higher amount of TRAIL receptors in comparison with less malignant tumors or normal tissue. In general TRAIL-R2 is more frequently expressed on tumor cells than TRAIL-R1.

"Tumor necrosis factor-related apoptosis-inducing ligand" or "TRAIL" as used herein refers to the 281 amino acid polypeptide having the amino acid sequence of:
MAMMEVQGGPSLGQTCVLIVIFTVLLQSLCVAVTYVYFTNELKQMQDKYSKSGIAC FLKEDDSYWDPNDEESMNSPCWQVKWQLRQLVRKMILRTSEETISTVQEKQQNISPL VRERGPQRVAAHITGTRGRSNTLSSPNSKNEKALGRKINSWESSRSGHSFLSNLHLRN GELVIHEKGFYYIYSQTYFRFQEEIKENTKNDKQMVQYIYKYTSYPDPILLMKSARNS CWSKDAEYGLYSIYQGGIFELKENDRIFVSVTNEHLIDMDHEASFFGAFLVG (SEQ ID NO: 1), as described by, *e.g.,* NP_003801.1 , together with any naturally occurring allelic, splice variants, and processed forms thereof. Typically, TRAIL refers to human TRAIL. The term TRAIL, in some embodiments of the aspects described herein, is also used to refer to truncated forms or fragments of the TRAIL polypeptide, comprising, for example, specific TRAIL domains or residues thereof. The amino acid sequence of the human TRAIL molecule as presented in SEQ ID NO: 1 comprises an N-terminal cytoplasmic domain (amino acids 1-18), a transmembrane region (amino acids 19-38), and an extracellular domain (amino acids 39-281). The extracellular domain comprises the TRAIL receptor-binding region. TRAIL also has a spacer region between the C-terminus of the transmembrane domain and a portion of the extracellular domain This spacer region, located at the N-terminus of the extracellular domain, consists of amino acids 39 through 94 of SEQ ID NO: 1. Amino acids 138 through 153 of SEQ ID NO: 1 correspond to a loop between the β sheets of the folded (three dimensional) human TRAIL protein.

In one embodiment, the TRAIL comprises the extracellular domain of TRAIL (e.g., human trial). In one embodiment, the TRAIL is a fusion protein comprising one or more domains of TRAIL (e.g., the extracellular domain) fused to a heterologous sequence. In one embodiment, the TRAIL fusion protein further comprises a signal for secretion.

Preferably, the TRAIL protein and the nucleic acids encoding it, are derived from the same species as will be administered in the therapeutic methods described herein. In one embodiment, the nucleotide sequence encoding TRAIL and the TRAIL amino acid sequence is derived from a mammal. In one embodiment, the mammal is a human (human TRAIL). In one embodiment, the mammal is a non-human primate.

### Fragments, Variants and Derivatives of TRAIL

Fragments, variants and derivatives of native TRAIL proteins for use in the invention that retain a desired biological activity of TRAIL, such as TRAIL apoptotic activity are also envisioned for delivery by the oncolytic virus vector. In one embodiment, the biological or apoptotic activity of a fragment, variant or derivative of TRAIL is essentially equivalent to the biological activity of the corresponding native TRAIL protein. In one embodiment, the biological activity for use in determining the activity is apoptotic activity. In one embodiment, 100% of the apoptotic activity is retained by the fragment, variant or derivative. In one embodiment less than 100%, activity is retained (e.g., 95%, 90%, 85%, 80%, 75%, 70%, 65%, 60%, 55%, 50%, 45%, 40%) as compared to the full length native TRAIL. Fragments, variants or derivatives which retain less activity (e.g., 34%, 30%, 25%, 20%, 10%, etc.) may also be of value in the therapeutic methods described herein and as such are also encompassed in the invention. One measurement of TRAIL apoptotic activity by a TRAIL variant or TRAIL domain is the ability to induce apoptotic death of Jurkat cells. Assay procedures for identifying biological activity of TRAIL variants by detecting apoptosis of target cells, such as Jurkat cells, are well known in the art. DNA laddering is among the characteristics of cell death via apoptosis, and is recognized as one of the observable phenomena that distinguish apoptotic cell death from necrotic cell death. Apoptotic cells can also be identified using markers specific for apoptotic cells, such as Annexin V, in combination with flow cytometric techniques, as known to one of skill in the art. Further examples of assay techniques suitable for detecting death or apoptosis of target cells include those described in WO2012/106281.

A variety of TRAIL fragments that retain the apoptotic activity of TRAIL are known in the art, and include biologically active domains and fragments disclosed in Wiley et al. (U.S. Patent Publication 20100323399).

TRAIL variants can be obtained by mutations of native TRAIL nucleotide sequences, for example. A "TRAIL variant," as referred to herein, is a polypeptide substantially homologous to a native TRAIL, but which has an amino acid sequence different from that of native TRAIL because of one or a plurality of deletions, insertions or substitutions. "TRAIL encoding DNA sequences" encompass sequences that comprise one or more additions, deletions, or substitutions of nucleotides when compared to a native TRAIL DNA sequence, but that encode a TRAIL protein or fragment thereof that is essentially biologically equivalent to a native TRAIL protein, *i.e.,* has the same apoptosis inducing activity.

The variant amino acid or DNA sequence preferably is at least 70%, at least 75%, at least 80%, at least 85%, at least 90%, at least 91%, at least 92%, at least 93%, at least 94%, at least 95%, at least 96%, at least 97%, at least 98%, at least 99%, or more, identical to a native TRAIL sequence. The degree of homology or percent identity) between a native and a mutant sequence can be determined, for example, by comparing the two sequences using freely available computer programs commonly employed for this purpose on the world wide web.

Alterations of the native amino acid sequence can be accomplished by any of a number of known techniques known to one of skill in the art. Mutations can be introduced, for example, at particular loci by synthesizing oligonucleotides containing a mutant sequence, flanked by restriction sites enabling ligation to fragments of the native sequence. Following ligation, the resulting reconstructed sequence encodes an analog having the desired amino acid insertion, substitution, or deletion. Alternatively, oligonucleotide-directed site-specific mutagenesis procedures can be employed to provide an altered nucleotide sequence having particular codons altered according to the substitution, deletion, or insertion required. Techniques for making such alterations include those disclosed by Walder et al. (Gene 42:133, 1986); Bauer et al. (Gene 37:73, 1985); Craik (BioTechniques, January 1985, 12-19); Smith et al. (Genetic Engineering: Principles and Methods, Plenum Press, 1981); and U.S. Pat. Nos. 4,518,584 and 4,737,462.

TRAIL variants can, in some embodiments, comprise conservatively substituted sequences, meaning that one or more amino acid residues of a native TRAIL polypeptide are replaced by different residues, and that the conservatively substituted TRAIL polypeptide retains a desired biological activity, *i.e.,* apoptosis inducing activity or TRAIL apoptotic activity, that is essentially equivalent to that of the native TRAIL polypeptide. Examples of conservative substitutions include substitution of amino acids that do not alter the secondary and/or tertiary structure of TRAIL.

In other embodiments, TRAIL variants can comprise substitution of amino acids that have not been evolutionarily conserved. Conserved amino acids located in the C-terminal portion of proteins in the TNF family, and believed to be important for biological activity, have been identified. These conserved sequences are discussed in Smith et al. (Cell, 73:1349, 1993,); Suda et al. (Cell, 75:1169, 1993); Smith et al. (Cell, 76:959, 1994); and Goodwin et al. (Eur. J. Immunol., 23:2631, 1993). Advantageously, in some embodiments, these conserved amino acids are not altered when generating conservatively substituted sequences. In some embodiments, if altered, amino acids found at equivalent positions in other members of the TNF family are substituted. Among the amino acids in the human TRAIL protein of SEQ ID NO:1 that are conserved are those at positions 124-125 (AH), 136 (L), 154 (W), 169 (L), 174 (L), 180 (G), 182 (Y), 187 (Q), 190 (F), 193 (Q), and 275-276 (FG) of SEQ ID NO:1. Another structural feature of TRAIL is a spacer region (*i.e.,* TRAIL (39-94)) between the C-terminus of the transmembrane region and the portion of the extracellular domain that is believed to be important for biological apoptotic activity. In some embodiments, when the desired biological activity of TRAIL domain is the ability to bind to a receptor on target cells and induce apoptosis of the target cells substitution of amino acids occurs outside of the receptor-binding domain.

A given amino acid of a TRAIL domain can, in some embodiments, be replaced by a residue having similar physiochemical characteristics, *e.g.,* substituting one aliphatic residue for another (such as Ile, Val, Leu, or Ala for one another), or substitution of one polar residue for another (such as between Lys and Arg; Glu and Asp; or Gln and Asn). Other such conservative substitutions, *e.g.,* substitutions of entire regions having similar hydrophobicity characteristics, are well known. TRAIL polypeptides comprising conservative amino acid substitutions can be tested in any one of the assays described herein to confirm that a desired TRAIL apoptotic activity of a native TRAIL molecule is retained.

Amino acids can be grouped according to similarities in the properties of their side chains (in A. L. Lehninger, in Biochemistry, second ed., pp. 73-75, Worth Publishers, New York (1975)): (1) non-polar: Ala (A), Val (V), Leu (L), Ile (I), Pro (P), Phe (F), Trp (W), Met (M); (2) uncharged polar: Gly (G), Ser (S), Thr (T), Cys (C), Tyr (Y), Asn (N), Gln (Q); (3) acidic: Asp (D), Glu (E); (4) basic: Lys (K), Arg (R), His (H).

Alternatively, naturally occurring residues can be divided into groups based on common side-chain properties: (1) hydrophobic: Norleucine, Met, Ala, Val, Leu, Ile; (2) neutral hydrophilic: Cys, Ser, Thr, Asn, Gln; (3) acidic: Asp, Glu; (4) basic: His, Lys, Arg; (5) residues that influence chain orientation: Gly, Pro; (6) aromatic: Trp, Tyr, Phe. Non-conservative substitutions will entail exchanging a member of one of these classes for another class.

Particularly preferred conservative substitutions for use in the TRAIL variants described herein are as follows: Ala into Gly or into Ser; Arg into Lys; Asn into Gln or into His; Asp into Glu; Cys into Ser; Gln into Asn; Glu into Asp; Gly into Ala or into Pro; His into Asn or into Gln; Ile into Leu or into Val; Leu into Ile or into Val; Lys into Arg, into Gln or into Glu; Met into Leu, into Tyr or into Ile; Phe into Met, into Leu or into Tyr; Ser into Thr; Thr into Ser; Trp into Tyr; Tyr into Trp; and/or Phe into Val, into Ile or into Leu.

Any cysteine residue not involved in maintaining the proper conformation of the multimodal TRAIL agent also can be substituted, generally with serine, to improve the oxidative stability of the molecule and prevent aberrant crosslinking. Conversely, cysteine bond(s) can be added to the multimodal TRAIL agent to improve its stability or facilitate oligomerization.

### Secreted TRAIL

In one embodiment, a form of TRAIL that is secreted (secreted TRAIL or sol TRAIL) is expressed by the oHSV described herein. Various forms of secreted TRAIL can be used in the methods and compositions described herein. In one embodiment, the secreted TRAIL is the naturally occurring soluble TRAIL. (Ashkenazi A. et al., J Clin Oncol 2008; 26: 3621-30, and Kelley SK et al., J Pharmacol Exp Ther 2001; 299: 31-8). In one embodiment the naturally occurring soluble TRAIL is fused with an antibody derivative, such as scFv245 (Bremer E. et al., J Mol Med 2008; 86: 909-24; Bremer E, et al., Cancer Res 2005; 65: 3380-88; Bremer E, et al., J Biol Chem 2005; 280: 10025-33, and Stieglmaier J, et al., Cancer Immunol Immunother 2008; 57: 233-46).

Alternatively, the endogenous secretion sequence of TRAIL present on the N terminus can be replaced with the signal sequence (otherwise referred to as the extracellular domain) from Flt3 ligand and an isoleucine zipper (Shah et al., Cancer Research 64: 3236-3242 (2004); WO 2012/106281; Shah et al. Mol Ther. 2005 Jun;11(6):926-31). Other secretion signal sequences can be added to TRAIL in turn to generate a secreted TRAIL for use in the invention. For example, SEC2 signal sequence and SEC(CV) signal sequence can be fused to TRAIL (see for example U.S. Patent Publication 2002/0128438). Other secretion signal sequences may also be used and nucleotides including restriction enzyme sites can be added to the 5' or 3' terminal of respective secretion signal sequence, to facilitate the incorporation of such sequences into the DNA cassette. Such secretion signal sequences can be fused to the N-terminus or to the C-terminus.

Additionally, a linker sequence may be inserted between heterologous sequence and the TRAIL in order to preserve function of either portion of the generated fusion protein. Such linker sequences known in the art include a linker domain having the 7 amino acids (EASGGPE; SEQ ID NO: 3), a linker domain having 18 amino acids (GSTGGSGKPGSGEGSTGG; SEQ ID NO: 4). As used herein, a "linker sequence" refers to a peptide, or a nucleotide sequence encoding such a peptide, of at least 8 amino acids in length. In some embodiments of the aspects described herein, the linker module comprises at least 9 amino acids, at least 10 amino acids, at least 11 amino acids, at least 12 amino acids, at least 13 amino acids, at least 14 amino acids, at least 15 amino acids, at least 16 amino acids, at least 17 amino acids, at least 18 amino acids, at least 19 amino acids, at least 20 amino acids, at least 21 amino acids, at least 22 amino acids, at least 23 amino acids, at least 24 amino acids, at least 25 amino acids, at least 30 amino acids, at least 35 amino acids, at least 40 amino acids, at least 45 amino acids, at least 50 amino acids, at least 55 amino acids, at least 56 amino acids, at least 60 amino acids, or least 65 amino acids. In some embodiments of the aspects described herein, a linker module comprises a peptide of 18 amino acids in length. In some embodiments of the aspects described herein, a linker module comprises a peptide of at least 8 amino acids in length but less than or equal to 56 amino acids in length, *i.e.,* the length of the spacer sequence in the native TRAIL molecule of SEQ ID NO: 1. In some embodiments, the linker sequence comprises the spacer sequence of human TRAIL, *i.e.,* amino acids 39-94 of SEQ ID NO: 1, or a sequence having at least 80%, at least 85%, at least 90%, at least 95%, at least 99% identity to amino acids 39-94 of SEQ ID NO: 1.

### Signal Sequences

Secreted TRAIL may be generated by incorporation of a secretion signal sequence into the TRAIL or TRAIL fragment or derivative. As used herein, the terms "secretion signal sequence," "secretion sequence," "secretion signal peptide," or "signal sequence," refer to a sequence that is usually about 3-60 amino acids long and that directs the transport of a propeptide to the endoplasmic reticulum and through the secretory pathway during protein translation. As used herein, a signal sequence, which can also be known as a signal peptide, a leader sequence, a prepro sequence or a pre sequence, does not refer to a sequence that targets a protein to the nucleus or other organelles, such as mitochondria, chloroplasts and apicoplasts. In one embodiment, the secretion signal sequence comprises 5 to 15 amino acids with hydrophobic side chains that are recognized by a cytosolic protein, SRP (Signal Recognition Particle), which stops translation and aids in the transport of an mRNA-ribosome complex to a translocon in the membrane of the endoplasmic reticulum. In one embodiment, the secretion signal peptide comprises at least three regions: an amino-terminal polar region (N region), where frequently positive charged amino acid residues are observed, a central hydrophobic region (H region) of 7-8 amino acid residues and a carboxy-terminal region (C region) that includes the cleavage site. Commonly, the signal peptide is cleaved from the mature protein with cleavage occurring at this cleavage site.

The secretory signal sequence is operably linked to the TRAIL or TRAIL fragment or derivative such that the two sequences are joined in the correct reading frame and positioned to direct the newly synthesized polypeptide into the secretory pathway of the host cell. Secretory signal sequences are commonly positioned 5' to the nucleotide sequence encoding the polypeptide of interest, although certain secretory signal sequences can be positioned elsewhere in the nucleotide sequence of interest (see, *e.g.,* Welch et al., U.S. Pat. No. 5,037,743; Holland et al., U.S. Pat. No. 5,143,830).

In one embodiment, the secretory sequence comprises amino acids 1-81 of the following Flt3L amino acid sequence: MTVLAPAWSP NSSLLLLLLL LSPCLRGTPD CYFSHSPISS NFKVKFRELT DHLLKDYPVT VAVNLQDEKH CKALWSLFLA QRWIEQLKTV AGSKMQTLLE DVNTEIHFVT SCTFQPLPEC LRFVQTNISH LLKDTCTQLL ALKPCIGKAC QNFSRCLEVQ CQPDSSTLLP PRSPIALEAT ELPEPRPRQL LLLLLLLLPL TLVLLAAAWG LRWQRARRRG ELHPGVPLPS HP (SEQ ID NO: 2, GenBank Accession P49772), or a functional fragment thereof. In one embodiment, the signal peptide comprises amino acids 1-81 of SEQ ID NO: 2. In one embodiment, the secretory signal sequence comprises a sequence having at least 90% identity to amino acids 1-81 of SEQ ID NO: 2. In one embodiment, the secretory signal sequence consists essentially of amino acids 1-81 of SEQ ID NO: 2. In one embodiment, the secretory signal sequence consists of amino acids 1-81 of SEQ ID NO: 2.

While the secretory signal sequence can be derived from Flt3L, in other embodiments a suitable signal sequence can also be derived from another secreted protein or synthesized *de novo.* Other secretory signal sequences which can be substituted for the Flt3L signal sequence for expression in eukaryotic cells include, for example, naturally-occurring or modified versions of the human IL-17RC signal sequence, otPA pre-pro signal sequence, human growth hormone signal sequence, human CD33 signal sequence Ecdysteroid Glucosyltransferase (EGT) signal sequence, honey bee Melittin (Invitrogen Corporation; Carlsbad, Calif.), baculovirus gp67 (PharMingen: San Diego, Calif.) (US Pub. No. 20110014656). Additional secretory sequences include secreted alkaline phosphatase signal sequence, interleukin-1 signal sequence, CD-14 signal sequence and variants thereof (US Pub. No. 20100305002) as well as the following peptides and derivatives thereof: Sandfly Yellow related protein signal peptide, silkworm friboin LC signal peptide, snake PLA2, *Cyrpidina noctiluca* luciferase signal peptide, and pinemoth fibroin LC signal peptide (US Pub. No. 20100240097). Further signal sequences can be selected from databases of protein domains, such as SPdb, a signal peptide database described in Choo et al., BMC Bioinformatics 2005, 6:249, LOCATE, a mammalian protein localization database described in Sprenger et al. Nuc Acids Res, 2008, 36:D230D233, or identified using computer modeling by those skilled in the art (Ladunga, Curr Opin Biotech 2000, 1:13-18).

Selection of appropriate signal sequences and optimization or engineering of signal sequences is known to those skilled in the art (Stern et al., Trends in Cell & Molecular Biology 2007 2:1-17; Barash et al., Biochem Biophys Res Comm 2002, 294:835-842). In one embodiment, a signal sequence can be used that comprise a protease cleavage site for a site-specific protease (*e.g.*, Factor IX or Enterokinase). This cleavage site can be included between the pro sequence and the bioactive secreted peptide sequence, e.g., TRAIL domain, and the pro-peptide can be activated by the treatment of cells with the site-specific protease (US Pub. No. 20100305002).

### Leucine Zippers

The TRAIL or TRAIL fragment, derivative or variant, described herein can, in some embodiments, further comprise a leucine zipper domain sequence. As used herein, "leucine zipper domains" refer to naturally occurring or synthetic peptides that promote oligomerization of the proteins in which they are found. The leucine zipper is a super-secondary structure that functions as a dimerization domain, and its presence generates adhesion forces in parallel alpha helices. A single leucine zipper comprises multiple leucine residues at approximately 7-residue intervals, which forms an amphipathic alpha helix with a hydrophobic region running along one side. The dimer formed by a zipper domain is stabilized by the heptan repeat, designated (abcdefg)ₙ according to the notation of McLachlan and Stewart (J. Mol. Biol. 98:293; 1975), in which residues a and d are generally hydrophobic residues, with d being a leucine, which line up on the same face of a helix. Oppositely-charged residues commonly occur at positions g and e. Thus, in a parallel coiled coil formed from two helical zipper domains, the "knobs" formed by the hydrophobic side chains of the first helix are packed into the "holes" formed between the side chains of the second helix. The residues at position d (often leucine) contribute large hydrophobic stabilization energies, and are important for oligomer formation (Krystek et al., Int. J. Peptide Res. 38:229, 1991). This hydrophobic region provides an area for dimerization, allowing the motifs to "zip" together. Furthermore, the hydrophobic leucine region is absolutely required for DNA binding. Leucine zippers were originally identified in several DNA-binding proteins (Landschulz et al., Science 240:1759, 1988), and have since been found in a variety of different proteins. Among the known leucine zippers are naturally occurring peptides and derivatives thereof that dimerize or trimerize.

Examples of zipper domains are those found in the yeast transcription factor GCN4 and a heat-stable DNA-binding protein found in rat liver (C/EBP; Landschulz et al., Science 243:1681, 1989). The nuclear transforming proteins, fos and jun, also exhibit zipper domains, as does the gene product of the murine proto-oncogene, c-myc (Landschulz et al., Science 240:1759, 1988). The fusogenic proteins of several different viruses, including paramyxovirus, coronavirus, measles virus and many retroviruses, also possess zipper domains (Buckland and Wild, Nature 338:547,1989; Britton, Nature 353:394, 1991; Delwart and Mosialos, AIDS Research and Human Retrovirtises 6:703, 1990). The zipper domains in these fusogenic viral proteins are near the transmembrane region of the protein. Oligomerization of fusogenic viral proteins is involved in fusion pore formation (Spruce et al, Proc. Natl. Acad. Sci. U.S.A. 88:3523, 1991). Zipper domains have also been reported to play a role in oligomerization of heat-shock transcription factors (Rabindran et al., Science 259:230, 1993).

Examples of leucine zipper domains suitable for producing multimodal TRAIL agents include, but are not limited to, those described in PCT application WO 94/10308; U.S. Pat. No. 5,716,805; the leucine zipper derived from lung surfactant protein D (SPD) described in Hoppe et al., 1994, FEBS Letters 344:191; and Fanslow et al., 1994, Semin. Immunol. 6:267-278. In one embodiment, leucine residues in a leucine zipper domain are replaced by isoleucine residues. Such peptides comprising isoleucine can also be referred to as isoleucine zippers, but are encompassed by the term "leucine zippers" as used herein.

### Additional Nucleic Acids

The recombinant oHSV comprising TRAIL nucleic acid may further contain additional heterologous nucleic acid sequences (e.g., in expressible form), referred to herein as a second heterologous nucleic acid sequence, a third heterologous nucleic acid sequence, etc. Alternatively, the recombinant oHSV may contain no additional heterologous nucleic acid sequences.

Any desired DNA can be inserted, including DNA that encodes selectable markers, or preferably genes coding for a therapeutic, biologically active protein, such as interferons, cytokines, chemokines, or more preferably DNA coding for a prodrug converting enzyme, including thymidine kinase (Martuza et al., Science, 252:854, 1991), cytosine deamindase (U.S. Pat. No. 5,358,866), cyp450 (U.S. Pat. No. 5,688,773), and others. In one embodiment, the nucleic acid encodes a protein that inhibits tumor growth (e.g., a chemotherapeutic, growth regulatory agent) or modifies an immune response. An example of a chemotherapeutic agent is mitomicin C. In one embodiment, the nucleic acid encodes a growth regulatory molecule (e.g., one that has been lost in tumorigenesis of the tumor). Examples of such molecules without limitation proteins from the caspase family such as Caspase-9 (P55211(CASP9_HUMAN); HGNC: 15111; Entrez Gene: 8422; Ensembl: ENSG000001329067; OMIM: 6022345; UniProtKB: P552113), Caspase-8 (Q14790 (CASP8_HUMAN); 9606 [NCBI]), Caspase-7 (P55210 (CASP7_HUMAN); 9606 [NCBI]), and Caspase-3 (HCGN: 1504; Ensembl:ENSG00000164305; HPRD:02799; MIM:600636; Vega:OTTHUMG00000133681), pro-apoptotic proteins such as Bax (HGNC: 9591; Entrez Gene: 5812; Ensembl: ENSG000000870887; OMIM: 6000405; UniProtKB: Q078123), Bid (HGNC: 10501; Entrez Gene: 6372; Ensembl: ENSG000000154757; OMIM: 6019975; UniProtKB: P559573), Bad (HGNC: 9361; Entrez Gene: 5722; Ensembl: ENSG000000023307; OMIM: 6031675; UniProtKB: Q92934), Bak (HGNC: 9491; Entrez Gene: 5782; Ensembl: ENSG000000301107; OMIM: 6005165; UniProtKB: Q166113), BCL2L11 (HGNC: 9941; Entrez Gene: 100182; Ensembl: ENSG000001530947; OMIM: 6038275; UniProtKB: 0435213), p53 (HGNC: 119981; Entrez Gene: 71572; Ensembl: ENSG000001415107; OMIM: 1911705; UniProtKB: P046373), PUMA (HGNC: 178681; Entrez Gene: 271132; Ensembl: ENSG000001053277; OMIM: 6058545; UniProtKB: Q96PG83; UniProtKB: Q9BXH13), Diablo/SMAC (HGNC: 215281; Entrez Gene: 566162; Ensembl: ENSG000001840477; OMIM: 6052195; UniProtKB: Q9NR283). In one embodiment, the nucleic acid encoes an immunomodulatory agent (e.g, immunostimulatory transgenes), including, without limitation, Flt-3 ligand, HMBG1, calreticulin, GITR ligand, interleukin-12, interleukin-15, interleukin-18, or CCL17.

The exogenous nucleic acids can be inserted into the oHSV by the skilled practitioner. In one embodiment, the exogenous nucleic acid is inserted into the thymidine kinase (TK) gene of the viral genome, or replacing the deleted TK gene (see for example, U.S. Patent No. 5,288,641, for insertion of exogenous nucleic acid into HSV). When the virus comprises a second exogenous nucleic acid, the nucleic acid preferably encodes an anti-oncogenic or oncolytic gene product. The gene product may be one (e.g. an antisense oligonucleotide) which inhibits growth or replication of only the cell infected by the virus, or it may be one (e.g. thymidine kinase) which exerts a significant bystander effect upon lysis of the cell infected by the virus.

### Encapsulation

The stem cells described herein may further be encapsulated in a synthetic extracellular matrix (sECM) or biodegradable hydrogel. Encapsulation of cells as the term is used herein refers to immobilization of cells within biocompatible, semipermeable membranes. Encapsulation of stem cells is described in Encapsulated Stem Cells for Cancer Therapy (Khalid Shah, Biomatter. Jan 1, 2013; 3(1): e24278). The encapsulation of cells allows the delivery of molecules of interest for a longer period of time as cells can release the molecules at the tumor site, especially if they hone to a tumor site as has been shown, and further can protect the oHSV from the host immune system. Due to their ability to provide a physiologic environment that promotes cell survival and prevent immune response while permitting easy in vivo transplantation and cell retention, biodegradable hydrogels and synthetic extracellular matrix (sECM) to encapsulate stem cells have been utilized (Burdick et al. Adv Mater. 2011;23H41-56; Allison et al., Hyaluronan: a powerful tissue engineering tool. Tissue Eng. 2006;12:2131-40). A variety of biomaterials such as alginate, hyaluronic acid, agarose and other polymers have been used for encapsulation and can be used in the invention disclosed herein.

Hyaluronic acid (HA) is a non-sulfated, linear polysaccharide with the repeating disaccharide, β-1,4-D-glucuronic acid-β-1,3-N-acetyl-Dglucosamine. HA is ubiquitous and highly hydrated polyanion and an essential component of the extracellular matrix (ECM); its structural and biological properties mediate cellular signaling, wound repair, morphogenesis and matrix organization (Prestwich, J Control Release. 2011;155:193-9). Although HA and its derivatives have been clinically used in the past, it has become recognized as an important building block for the creation of new biomaterials for use in cell therapy (Prestwich, J Cell Biochem. 2007;101:1370-83; Prestwich , Acc Chem Res. 2008;41:139-48; Shu et al., Biomaterials. 2003;24:3825-34) . Chemical modification of HA alters its material and biological properties (Shu et al., Biomacromolecules. 2002;3:1304-11), and targets three functional groups: the glucuronic acid carboxylic acid, the primary and secondary hydroxyl groups, and the N-acetyl group (following deamidation). The chemical, mechanical, and biological criteria for clinical and preclinical biomaterials are design constraints that must be incorporated into the biomaterial design (Prestwich, J Cell Biochem. 2007;101:1370-83; Vanderhooft et al., Biomacromolecules. 2007;8:2883-9). HA-based synthetic extracellular matrices (sECMs) have been developed for use in drug evaluation and regenerative medicine (Vanderhooft et al., Macromol Biosci. 2009;9:20-8). These sECMs were based on modification of the carboxylate groups of glycosaminoglycans (GAGs) and proteins such as gelatin using hydrazides containing disulfides (Pan et al., J Neurosci Res. 2009;87:3207-20; Sayyar et al., J Tissue Eng. 2012;3:2041731412462018). More importantly, in vivo injectable cell suspensions in the sECM macro monomers can be crosslinked with cytocompatible bifunctional polyethylene glycol (PEG) derived crosslinkers (Park et al., Gene Ther. 2002;9:613-24). The mechanical properties and rates of biodegradation could be altered by several varying parameters (Teng et al.,Proc Natl Acad Sci U S A. 2002;99:3024-9): (1) molecular weight of starting HA employed; (2) percentage of thiol modification; (3) concentrations of thiolated HA and thiolated gelatin; (4) molecular weight of the crosslinker polyethylene glycol diacrylate (PEGDA); and (5) ratio of thiols to acrylates. Living hydrogels allow control of gel composition and mechanics, and permit incorporation of cells and a wide variety of small molecules, large molecules, nanoparticles, and microparticles (Shu et al., Biomacromolecules. 2002;3:1304-11).

### Pharmaceutical Compositions

Another aspect of the invention relates to a pharmaceutical composition used to deliver the isolated stem cell or population described herein to the body of the subject at the appropriate site for treatment. The pharmaceutical composition comprises the active agent (the stem cells) and a pharmaceutically acceptable carrier. The stem cells within the composition may further be encapsulated as described herein. As used here, the term "pharmaceutically acceptable" refers to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

As used here, the term "pharmaceutically-acceptable carrier" means a pharmaceutically-acceptable material, composition or vehicle, such as a liquid or solid filler, diluent, excipient, manufacturing aid (e.g., lubricant, talc magnesium, calcium or zinc stearate, or steric acid), or solvent encapsulating material, involved in carrying or transporting the subject compound from one organ, or portion of the body, to another organ, or portion of the body. Each carrier must be "acceptable" in the sense of being compatible with the other ingredients of the formulation and not injurious to the patient. Some examples of materials which can serve as pharmaceutically-acceptable carriers include: (1) sugars, such as lactose, glucose and sucrose; (2) starches, such as corn starch and potato starch; (3) cellulose, and its derivatives, such as sodium carboxymethyl cellulose, methylcellulose, ethyl cellulose, microcrystalline cellulose and cellulose acetate; (4) powdered tragacanth; (5) malt; (6) gelatin; (7) lubricating agents, such as magnesium stearate, sodium lauryl sulfate and talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol (PEG); (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) pH buffered solutions; (21) polyesters, polycarbonates and/or polyanhydrides; (22) bulking agents, such as polypeptides and amino acids (23) serum component, such as serum albumin, HDL and LDL; (22) C₂-C₁₂ alcohols, such as ethanol; and (23) other non-toxic compatible substances employed in pharmaceutical formulations. Wetting agents, coloring agents, release agents, coating agents, sweetening agents, flavoring agents, perfuming agents, preservative and antioxidants can also be present in the formulation. The terms such as "excipient", "carrier", "pharmaceutically acceptable carrier" or the like are used interchangeably herein.

For injection, the active ingredients of the pharmaceutical composition may be formulated in aqueous solutions, preferably in physiologically compatible buffers such as Hank's solution, Ringer's solution, or physiological salt buffer

### Methods of Treatment

Another instance the disclosure relates to a method for treating a proliferative disorder in a subject's brain (e.g., brain tumor/cancer). The method comprises administering a therapeutically effective amount of a population of the isolated stem cells comprising the infectious oHSV described herein to the subject. The population is administered as part of a pharmaceutical composition described herein. Administration is by a route that results in contact of the cells with the cancer cells in the brain of the subject to thereby deliver the oHSV to those cancer cells. The stem cells that carry the oHSV are kept in a state that allows their use as a carrier of the oHSV. The oHSV replicate in the cells and/or are released into the environment of the stem cells to thereby infect the brain cancer cells in the subject. The method may further comprise obtaining stem cells (non-cancer) from the subject and manipulating them to contain the oHSV, and then delivering those stem cells back into the patient. Sources of the stem cells from the subject are described herein (e.g.,bone marrow, adipose tissue).

In one embodiment the proliferative disorder is a brain cancer, brain tumor, or intracranial neoplasm. Primary cancers, when metastasized to the brain, can be treated by the herein described methods. Cancers that arise originally in the brain are also encompassed by the invention. Such cancers include, without limitation, glioblastoma (GBM), astrocytoma, meningiomas, and neuroblastoma.

Intracranial neoplasms or cancers can arise from any of the structures or cell types present in the CNS, including the brain, meninges, pituitary gland, skull, and even residual embryonic tissue. The overall annual incidence of primary brain tumors in the United States is 14 cases per 100,000. The most common primary brain tumors are meningiomas, representing 27% of all primary brain tumors, and glioblastomas, representing 23% of all primary brain tumors (whereas glioblastomas account for 40% of malignant brain tumor in adults). The methods of treatment described herein are appropriate for use on subjects with such conditions.

### Administration

The pharmaceutical composition is administered by a route that results in direct contact of the stem cells or of the oHSV produced from the stem cells with brain tumor cells of the subject. In one embodiment, this is accomplished by injection of a pharmaceutical composition comprising the stem cells into a brain tumor or into a brain tumor resection cavity of the subject (e.g. for a subject with a primary brain tumor). In one embodiment, the composition is administered for systemic delivery to the brain such as through injection into the intracarotid artery (e.g., for a subject with a secondary metastatic tumor in the brain). The secondary metastatic tumor may results from any form of cancer that can metastasize to the brain, examples of which are provided herein. In one embodiment, the secondary metastatic tumor arises from a melanoma. The pharmaceutical composition comprising the stem cells is administered in a manner compatible with the dosage formulation, and in a therapeutically effective amount. The quantity to be administered and timing depends on the subject to be treated, capacity of the subject's system to utilize the active ingredient, and degree of therapeutic effect desired. Precise amounts of active ingredient required to be administered depend on the judgment of the practitioner and will be determined for each individual.

Unless otherwise defined herein, scientific and technical terms used in connection with the present application shall have the meanings that are commonly understood by those of ordinary skill in the art. Further, unless otherwise required by context, singular terms shall include pluralities and plural terms shall include the singular.

It should be understood that this invention is not limited to the particular methodology, protocols, and reagents, etc., described herein and as such may vary. The terminology used herein is for the purpose of describing particular embodiments only, and is not intended to limit the scope of the present invention, which is defined solely by the claims.

Other than in the operating examples, or where otherwise indicated, all numbers expressing quantities of ingredients or reaction conditions used herein should be understood as modified in all instances by the term "about." The term "about" when used to described the present invention, in connection with percentages means ±1%.

In one respect, the present invention relates to the herein described compositions, and respective component(s) thereof, as essential to the invention, yet open to the inclusion of unspecified elements, essential or not ("comprising). In some embodiments, other elements to be included in the description of the composition or respective component thereof are limited to those that do not materially affect the basic and novel characteristic(s) of the invention ("consisting essentially of'). This applies equally to compositions and components therein. In other embodiments, the inventions, compositions, and respective components thereof, described herein are intended to be exclusive of any element not deemed an essential element to the component, composition or method ("consisting of').

The present description disloses the subject matter defined in any one of the following numbered paragraphs.
1. An isolated stem cell or population thereof comprising infectious recombinant oncolytic herpes simplex virus (oHSV).
2. The isolated stem cell or population thereof of paragraph 1 that is a non-cancer stem cell.
3. The isolated stem cell or population thereof of any one of paragraphs 1 or 2 that is human.
4. The isolated stem cell or population thereof of any one of paragraphs 1-3 that is selected from the group consisting of a mesenchymal stem cell (MSC), a neuronal stem cell, and an induced pluripotent stem cell.
5. The isolated stem cell or population thereof of paragraph 4, wherein the MSC is derived from bone marrow, umbilical cord blood, or adipose tissue.
6. The isolated stem cell or population thereof of any one of paragraphs 1-5 wherein the oncolytic HSV is engineered to be inducible by addition of an exogenous factor.
7. The isolated stem cell or population thereof of paragraph 6 wherein the oncolytic HSV is engineered to have a tetracycline inducible promoter driving ICP27 expression.
8. The isolated stem cell or population thereof of any one of paragraphs 1-7 wherein the oncolytic HSV is engineered to comprise a nucleic acid sequence encoding tumor necrosis factor-related apoptosis-inducing ligand (TRAIL) or a biologically active fragment thereof, in expressible form.
9. The isolated stem cell or population thereof of paragraph 8, wherein the TRAIL is a secreted form of TRAIL (S-TRAIL).
10. The isolated stem cell or population thereof of any one of paragraphs 1-9, wherein the oHSV is selected from the group consisting of G207, G47Δ HSV-R3616, 1716, R3616, and R4009.
11. The isolated stem cell or population thereof of any one of paragraphs 8-10, wherein the TRAIL is a TRAIL fusion protein.
12. The isolated stem cell or population thereof of any one of paragraphs 8-11, wherein the TRAIL is regulated by the HSV immediate early 4/5 promoter.
13. The isolated stem cell or population thereof of any one of paragraphs 8-12, wherein the virus contains an additional exogenous nucleic acid in expressible form.
14. The isolated stem cell or population thereof of any one of paragraphs 8-12 wherein the virus contains no additional exogenous nucleic acids.
15. The isolated stem cell or population thereof of any one of paragraphs 1-14 that is encapsulated in a synthetic extracellular matrix (sECM).
16. A pharmaceutical composition comprising the isolated stem cell or population thereof of any one of paragraphs 1-15, and a pharmaceutically acceptable carrier.
17. A method of treating brain cancer in a subject, comprising administering the pharmaceutical composition of paragraph 13 to the subject to thereby contact cancer cells in the brain of the subject with oHSV.
18. The method of paragraph 17, wherein the brain cancer is a primary brain cancer.
19. The method of paragraph 18, wherein the primary brain cancer is malignant glioblastoma multiforme (GBM).
20. The method of paragraph 17, wherein the brain cancer is a secondary metastatic cancer in the brain.
21. The method of paragraph 20, wherein the secondary metastatic cancer is melanoma.
22. The method of any one of paragraphs 17- 21, wherein administration is by injection into a tumor resection cavity.
23. The method of any one of paragraphs 17- 21, wherein administration is by intracarotid artery injection.

The invention is further illustrated by the following examples, which should not be construed as further limiting.

### EXAMPLES

### Example 1A: Human MSC loaded with different variants of multi-mechanistic oHSV have anti-tumor effects in mouse models of intact and resected GBMs

In this study we loaded human MSC with oHSV (MSC-oHSV) and explored the dynamics of oHSV loaded MSC (MSC-oHSV) in real time *in vitro* and *in vivo,* extensively comparing direct oHSV injection with MSC mediated delivery of oHSV in malignant models of GBMs in mice. Using novel diagnostic and armed oHSV mutants, we then tested the efficacy of MSC-oHSV encapsulated in biocompatible synthetic extracellular matrix (sECM) in clinically applicable mouse resection models which more accurately reflect the current clinical setting which involves GBM tumor resection as standard treatment. To supersede oHSV resistant tumors, we loaded MSC with a pro-apoptotic oHSV variant (oHSV-TRAIL) and tested their efficacy in TRAIL and oHSV resistant GBMs.

### Results

### MSC as a cellular delivery vehicle for oHSV

To assess whether MSC are capable of serving as a cellular delivery vehicle for oHSV, we first studied oHSV replication and release of infectious viral particles in human MSC *in vitro.* Human MSC were infected with an oHSV mutant in which cDNA encoding the mCherry fluorescent protein was placed under an immediate-early promoter of HSV (oHSV-mCh) (Cheema et al, 2013 submitted). Infection of MSC with oHSV-mCh resulted in exponential amplification of virus during the first 24 hours (Figure 1A), reaching peak virus yields at about 72 hours post-infection with a viral burst size of -500 (production of 500 infectious virus particles in average per cell). This correlated with marker protein mCherry expression and resulted in decreasing MSC survival *in vitro* over a time course of 5 days post oHSV infection (Figure 1B-E). To assess the survival of oHSV-mCherry loaded MSC (MSC-oHSV-mCh) *in vivo,* MSC were first engineered to express a bimodal fluorescent and bioluminescent fused protein, GFP-firefly luciferase (Fluc) fusion protein (GFl). MSC-GFl were then loaded with oHSV-mCh, implanted into the brains of SCID mice and MSC survival was monitored by changes in Fluc activity *in vivo.* The viability of uninfected MSC control decreased over time, but the viability decline in MSC-oHSV-mCh was more rapid (Figure 1F). Brain sections of mice at 24, 48 and 120 hours after oHSV-mCh injection showed virus-loaded, mCh+ MSC (Figure 1G), which rounded off due to cytopathic effect (CPE) as virus amplification and mCh expression intensified (Figure 1H), eventually resulted in cell lysis with mCh+ cell debris (Figure 1I).

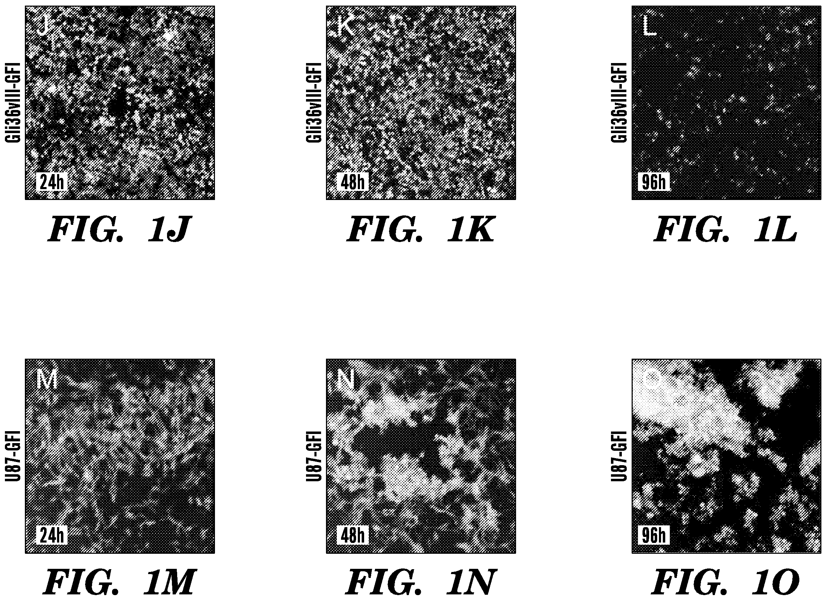

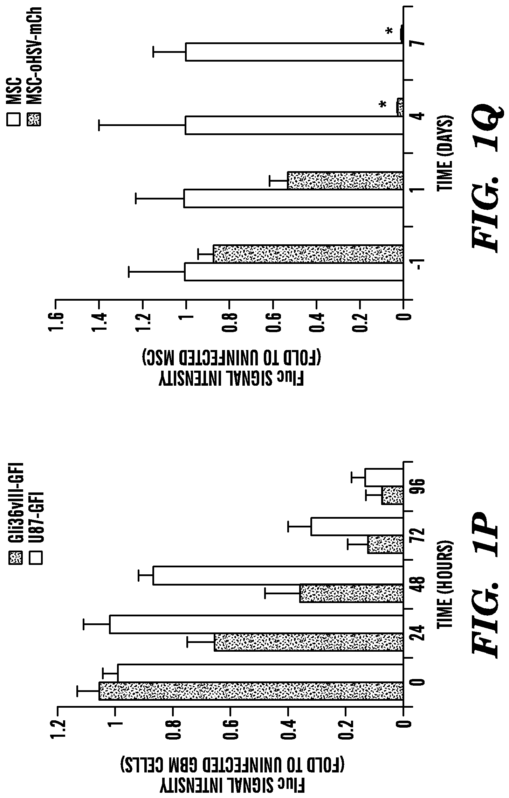

To assess the oncolytic activity of MSC-oHSV-mCh in GBM cells, we utilized human GBM cell lines Gli36vIII (highly proliferating) and U87 (intermediately proliferating) engineered to express the diagnostic marker GFl, Gli36vIII-GFl and U87-GFl. A direct correlation between GBM-GFl cell number, Fluc signal intensity and GFP-positive cells was seen *in vitro* within the ranges tested (Figure 6). Release of oHSV-mCh from MSC resulted in the infection of engineered GBM cells and spread among them leading to extensive infection and oncolysis as illustrated by merged images of green and red fluorescence photomicrographs taken at 24, 48 and 96 hours showing infected GBM cells in yellow (Figure 1J-O). *In vitro,* co-culture of these GBM lines with MSC-oHSV-mCh (3% of total cell number) resulted in drastic cell killing of both highly proliferating Gli36vIII-GFl (Figure 1J-L, P) and intermediately proliferating U87-GFl (Figure 1M-O, P) GBM cells. Next, we tested whether different ratios of MSC-oHSV-mCh to GBMs in co-culture would influence the killing of a panel of GBMs with varying sensitivity to oHSV. A significantly increased killing was seen in oHSV sensitive (U87, U251 and U373, Gli36vIII,) and oHSV resistant (LN229, U138 and LN319) (Figure 7). *In vivo,* Fluc bioluminescence imaging (BLI) on mice bearing Gli36vIII-GFl tumors showed the growth dynamics of that highly aggressive GBM line over 14 days (Figure 8A). Despite the aggressiveness of this GBM tumor, the intratumoral injection of MSC-oHSV-mCh in mice bearing Gli36vIII-GFl tumors resulted in a significant tumor volume reduction as compared to the control MSC injection, (2.2% ±0.7% of relative tumor volume remaining) after 4 days of MSC-oHSV-mCh treatment (Figure 1Q). A side by side comparison of concentrated oHSV-mCh (2.5x10⁷ pfu) vs MSC-oHSV-mCh (2x10⁵ cells infected at MOI 15) upon direct injection to established intracerebral Gli36vIII-GFI tumors showed that MSC-oHSV-mCh provided more potent effect than oHSV-mCh although the oncolytic effect of MSC delivered oHSV set in later than the concentrated oHSV-mCh (Figure 8B). These results reveal that oHSV-mCh loaded MSC effectively produce oHSV progeny which results in effective killing of GBM cells *in vitro* as well as in established GBMs *in vivo.*

### Dynamics of oHSV infection and oncolysis in vivo

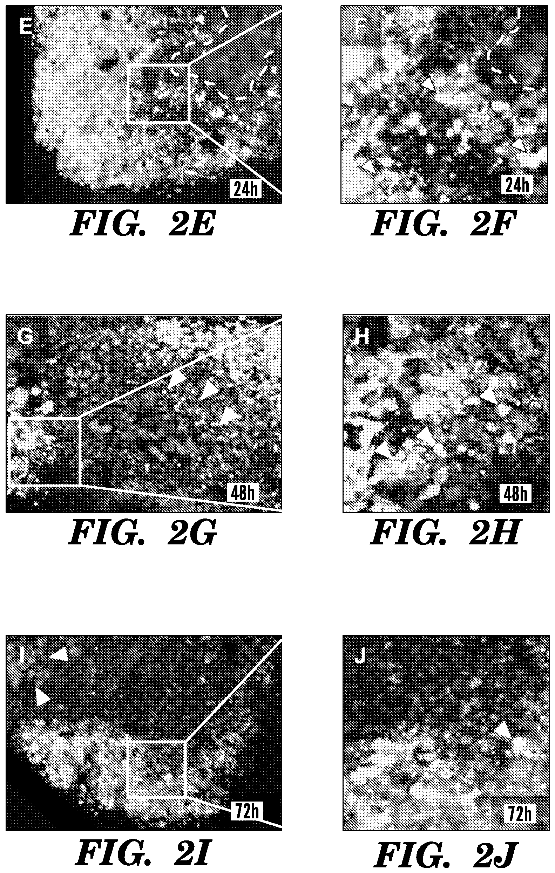

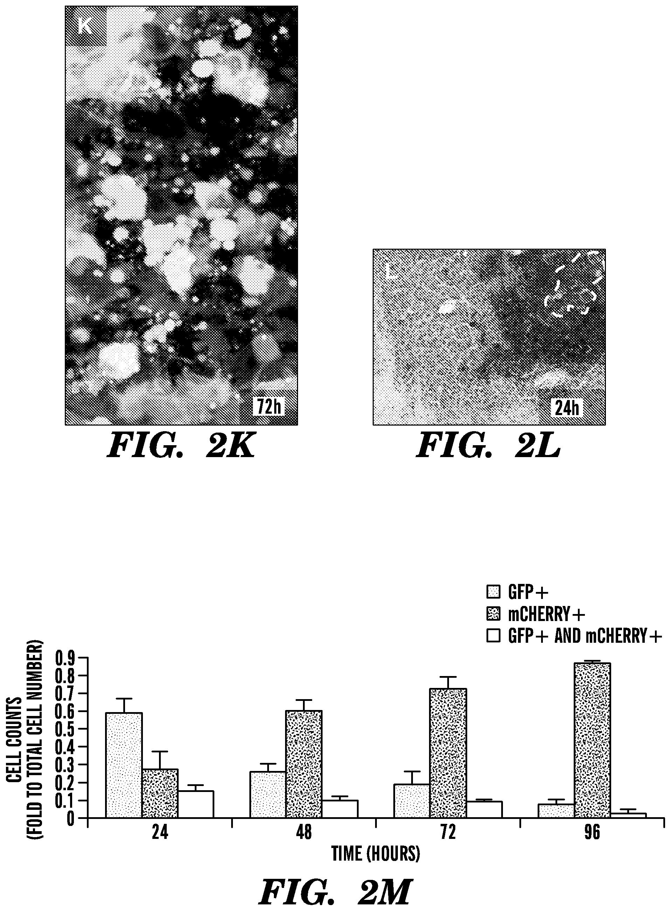

In order to investigate the dynamics of oHSV spread and GBM cell killing mediated by oHSV loaded MSC, we performed histological studies using multi-color fluorescence imaging *in vivo.* Mice bearing established highly proliferating Gli36vIII-GFl tumors were treated with MSC-oHSV-mCh and brain sections were collected at 24, 48, 72 and 96 hours post MSC-oHSV-mCh implantation were analyzed with confocal microscopy and histochemistry. Fluorescence imaging of serial brain sections showed rapid spread of oHSV-mCh emanating from MSC-oHSV-mCh implantation site at 24 h, 48 h and 72 h with concomitant shrinkage of Gli36vIII-GFl tumor area (green) within 96 hours (Figure 2A-D). Higher magnification fluorescence imaging at 24 hours revealed the emergence of a significant number of yellow cells (GFP+ mCherry+) around the MSC implantation site, confirming infection of tumor cells (Figure 2E-F, white arrowheads). oHSV amplification and spread penetrating into tumor tissue was seen at 48 hours and rounded tumor cells showing weakened GFP expression could be observed suggesting widespread cytopathic effect (Figure 2G, black arrowheads). At 48 and 72 hours post implantation MSC-oHSV-mCh retained strong expression of mCherry capable of serving as oHSV factory (white arrowheads in Figure 2B, I). After 72 hours the forefront of mCherry+ area extended to near tumor periphery, leaving vast areas of mCh+ cell debris behind and considerably reduced areas of GFP+ virus-free tumor (Figure 2C, I). Continuing rounds of tumor infection occurred at the borders between the mCherry+ and GFP+ regions as shown by GFP+ mCherry+ (visualized as yellow) tumor cells for at least 72 hours post implantation (Figure 2F, H, J, white arrowheads) with high resolution photomicrographs showing infected GBM cells at varying stages of initial infection, cytopathic effect and final cell lysis (Figure 2K). X-gal staining on adjacent brain sections revealed that an area of cells positive for oHSV reporter lacZ was almost exactly superimposable on the combined mCherry+ and mCherry+GFP+ (visualized as yellow) area on the corresponding fluorescence images, confirming that mCherry+ and mCherry+ GFP+ cells are oHSV-infected (Figure 2L). Quantification of the fluorescent imaging results revealed a continuous increase of oHSV-mCh-infected cells (visualized as red) and a concurrent decrease of unimpaired tumor cells (visualized as green), with the most dramatic changes in terms of tumor infection and virus replication taking place within the first 48 hours post MSC-oHSV-mCh implantation (Figure 2M). Our multi-color fluorescence imaging thus reveals the dynamic process of oHSV infection and tumor destruction mediated by oHSV-loaded MSC *in vivo.*

### MSC-mediated delivery of oHSV in a mouse model of GBM resection

We have recently developed a clinically relevant mouse model of GBM resection and shown the synthetic extracellular matrix (sECM) encapsulation of therapeutic MSC allows retention of a significant number of MSC in the GBM tumor resection cavity resulting in higher therapeutic efficacy [25]. Based on these studies, we first assessed whether sECM encapsulation of oHSV loaded MSC influences their capability to deliver and release oHSV. *In vitro* co-culture of sECM encapsulated MSC-oHSV-mCh with U87-GFl GBM cells significantly reduced GBM cell viability over time compare with sECM encapsulated oHSV-mCh (Figure 9A-B) indicating that sECM encapsulation of MSC-oHSV-mCh allow the release of oHSV from sECM. In order to assess the effects of sECM encapsulated MSC-oHSV *in vivo,* we first sought to determine whether delivery of oHSV by MSC increases oHSV persistence and oncolytic activity in the tumor in a clinically relevant model of GBM resection when compared to direct injection of concentrated oHSV. We utilized an *in vivo* imageable oHSV mutant bearing firefly luciferase (oHSV-Fluc) which we previously developed [12]. sECM encapsulated MSC loaded with oHSV-Fluc (MSC-oHSV-Fluc, 3x10⁶ pfu when loading) led to significantly increased expression of oHSV mediated Fluc when compared to conventional direct injection of purified oHSV-Fluc (1x10⁸ pfu) in the tumor resection cavity of the pre-established Gli36vIII-GFP tumors (13,2 ± 1,82 times higher Fluc expression in the MSC-oHSV-Fluc group in comparison to purified oHSV-Fluc group) (Figure 3A). These results show that sECM encapsulated oHSV loaded MSC are retained for a significantly longer time than the oHSV alone in the tumor resection cavity.

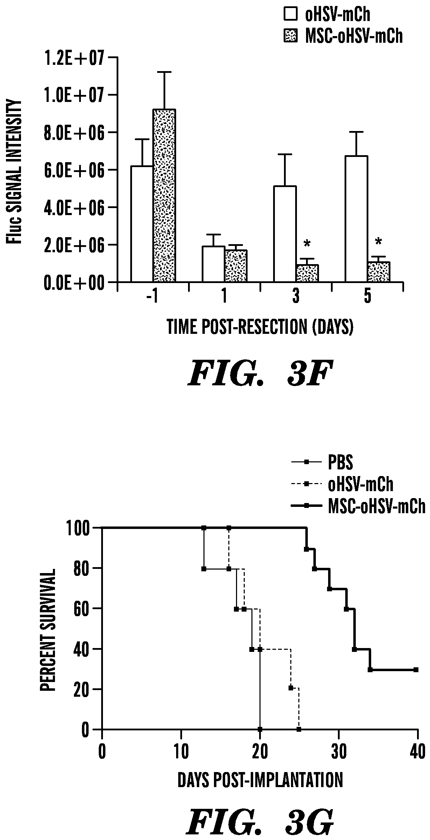

In order to compare the therapeutic efficacy of sECM encapsulated MSC-oHSV-mCh with direct injection of oHSV-mCh into the resection cavity in a mouse model of GBM resection, mice bearing established Gli36vIII-GFl tumors (Figure 3B-C) underwent subtotal GBM resection (Figure 3D). Mice bearing resected tumors were treated with sECM-encapsulated MSC-oHSV-mCh (Figure 3E) or purified oHSV-mCh. A quick relapse of tumor, attaining pre-resection levels of tumor burden (Fluc signal) 5 days post treatment was seen in mice treated with purified oHSV-mCh (Figure 3F). In contrast, mice treated with sECM-encapsulated MSC-oHSV-mCh showed a significant suppression of tumor growth (Figure 3F). This anti-GBM activity almost doubled median survival time in sECM-encapsulated MSC-oHSV-mCh group (36.1 days), compared to sECM-encapsulated MSC group (17.8 days), and purified oHSV-mCh group (19.4 days) (p <0.05; Figure 3G). These results demonstrate that encapsulated MSC-oHSV results in significantly increased anti-GBM efficacy compared to direct injection of purified oHSV in a preclinical model of GBM resection possibly due to long lasting production of oHSV in the vicinity of GBM deposits.

### MSC-mediated delivery of an armed oHSV mutant

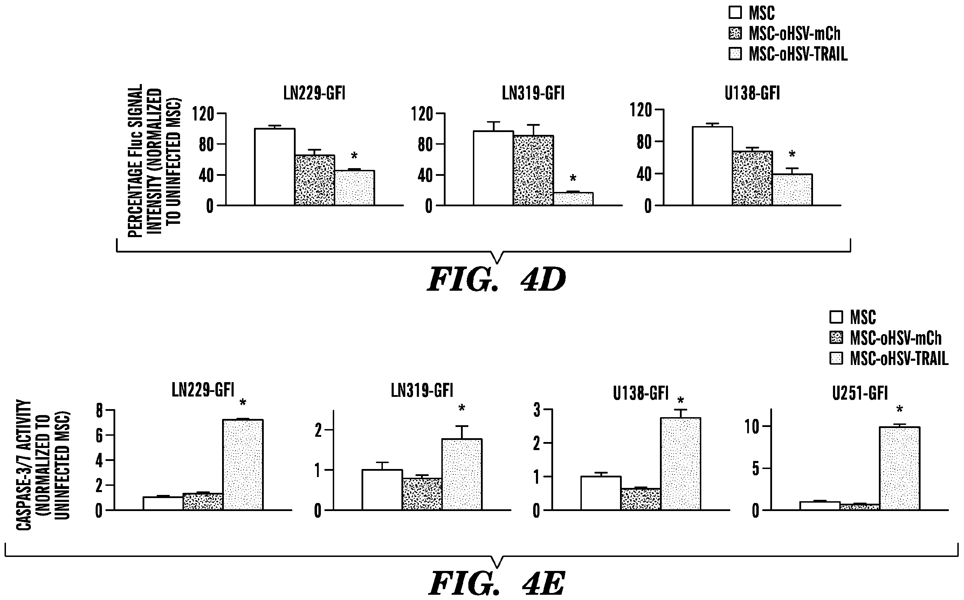

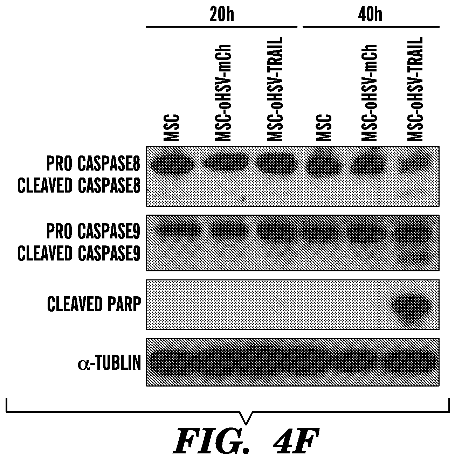

We have recently created an armed oHSV mutant encoding secretable TRAIL (oHSV-TRAIL) and shown that it targets a broad spectrum of GBM lines including oHSV resistant and TRAIL resistant lines [12]. To develop MSC loaded oHSV therapies for a broad spectrum of GBMs, we next investigated whether MSC loaded with oHSV-TRAIL could target both oHSV and TRAIL resistant GBM lines. In order to assess the utility of MSC to deliver oHSV-TRAIL, we loaded MSC with oHSV-TRAIL (MSC-oHSV-TRAIL). oHSV-TRAIL released from MSC exponentially amplified during the first 36 hours and reached a plateau 48 hours post-infection (Figure 4A). Similar to MSC-oHSV-mCh, a time dependent decrease in MSC viability was seen in MSC-oHSV-TRAIL over 120 hours (Figure 4B). Time course ELISA on MSC-oHSV-TRAIL confirmed the release of S-TRAIL into the culture media over time, reaching 200ng/ml from 10⁶ MSC 48 hours post infection (Figure 4C). In order to confirm functionality and efficacy of S-TRAIL secreted by MSC-oHSV-TRAIL, we performed co-culture assays of MSC-oHSV-mCh and MSC-oHSV-TRAIL with different GBM lines (LN229, LN319, U138, U251) that are either fully or semi-resistant to TRAIL and have low susceptibility to oHSV mediated oncolysis [12]. All the GBM lines were engineered to express GFl as a diagnostic marker (Figure 6). MSC-oHSV-TRAIL resulted in significantly greater cell killing of LN229-GFl, LN319-GFl or U138-GFl GBM than MSC-oHSV-mCh after 3-day co-culture with GBM cells (Figure 4D; Figure 10A-C). Co-culture of these TRAIL resistant GBM lines with MSC-oHSV-TRAIL but not with MSC-oHSV-mCh activated caspase-3/7, revealing that oHSV-TRAIL mediates apoptosis in GBMs (Figure 4E). To further confirm that MSC-oHSV-TRAIL killing of GBMs was apoptosis mediated, oHSV and TRAIL resistant LN229 GBM cells and MSC, MSC-oHSV or MSC-oHSV-TRAIL were plated in the lower and upper chamber of transwell inserts, respectively, and incubated for 40 hours. Western blotting analysis of LN229 GBM cell lysate showed a significant increase in cleaved caspase-8, cleaved caspase-9 and cleaved PARP in MSC-oHSV-TRAIL treatment group as compared to controls (Figure 4F). These results show that MSC loaded with the armed oHSV mutant encoding secretable TRAIL (oHSV-TRAIL) effectively produce oHSV-TRAIL progeny and induce apoptosis mediated killing of both oHSV- and TRAIL-resistant GBM.

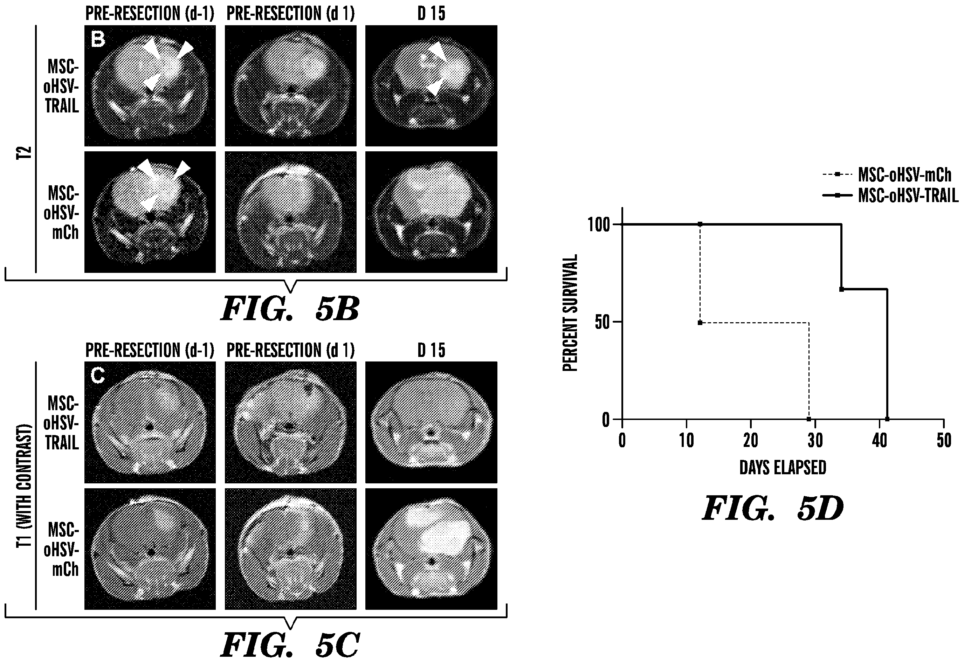

To test *in vivo* efficacy of MSC-oHSV-TRAIL in a clinically relevant GBM mouse model, mice bearing established LN229-GFl tumors underwent GBM resection followed by injection of sECM-encapsulated MSC, MSC-oHSV-mCh or MSC-oHSV-TRAIL. Bioluminescence imaging revealed rapid tumor re-growth after implantation of MSC as well as MSC-oHSV-mCh, indicating that this tumor model is resistant to oHSV therapy. In contrast, relapse of LN229-GFI tumors was significantly suppressed by the treatment with MSC-oHSV-TRAIL (Figure 5A). T2-weighted magnetic resonance imaging (MRI) showed a localized high signal intensity area at the site of MSC-oHSV-TRAIL injection on day 1 post-resection, which persisted for 2 weeks (Figure 5B). T1-weighted MRI with contrast confirmed the efficacy by sECM-encapsulated MSC-oHSV-TRAIL as it revealed sustained tumor regression after treatment (Figure 5C). In contrast, the mice treated with sECM encapsulated MSC-oHSV-mCh had massive tumor regrowth, clearly identified by its strong tumor enhancement (Figure 5C). This anti-GBM activity by MSC-oHSV-TRAIL resulted in significant prolongation of mice survival in the sECM-encapsulated MSC-oHSV-TRAIL treated group (median survival time 41 days) as compared to the MSC-oHSV-mCh treated group (median survival time 29 days; p <0.05 (Figure 5D). These results demonstrate that MSC can serve as a robust cellular delivery vehicle for oHSV armed with pro-apoptotic molecule, and when applied within sECM to a clinically relevant mouse model of GBM resection, this treatment modality targets resistant GBM, resulting in significantly increased animal survival.

### Discussion

In this study we explored the dynamics of diagnostic oHSV mutants, oHSV-mCh and oHSV-Fluc delivered by MSC (MSC-oHSV) in real time *in vitro* and *in vivo* in mouse models of GBMs. We also tested the efficacy of sECM encapsulated MSC-oHSV and its pro-apoptotic variant MSC-oHSV-TRAIL in clinically applicable mouse models that represent clinical scenarios of tumor resection and resistance. We show that MSC release oHSV over an extended period of time resulting in widespread tumor cell killing both *in vitro* and in established tumors *in vivo* thus allowing the understanding of both carrier cell fate as well as timing and dynamics of viral spread and oncolysis *in vivo.* sECM encapsulated stem cells loaded with oHSV and oHSV-TRAIL delayed tumor regrowth and significantly increased survival of mice bearing GBMs with varying resistance to oHSV and TRAIL after their transplantation into the resection cavity after surgical debulking.

Among the novel therapies developed for treatment of GBM, the use of oncolytic viruses holds substantial promise as majority of the eleven clinical trials conducted to date have proven that these viruses do not present any safety issue when injected directly into the brain [26, 27]. However, it has also become clear that viral delivery and patient anti-viral immune response remain two of the biggest issues that need to be solved in order to turn oncolytic viruses into an effective GBM therapy [26]. The procedure used in clinical trials to deliver oncolytic viruses has been direct injection of unshielded virus into the resection cavity and surrounding brain parenchyma. However this delivery method creates certain issues, such as clearance of injected virus by the influx of cerebrospinal fluid, elimination of virus by neutralizing antibodies or quick uptake by healthy brain parenchyma. In an effort to circumvent the issues dampening the current oHSV trials in GBM, we sought to develop a cell-based strategy to deliver oHSV that takes into account the challenges found in a clinical scenario of GBM resection. We have previously shown that both human NSC and MSC can home to tumors in the brain, can effectively deliver therapeutic proteins on site resulting in significant therapeutic efficacy. To our knowledge, this is the first study that uses oHSV loaded MSC for GBM therapy and attempts to develop a delivery approach for clinical models of GBM resection. The use of MSC as delivery vehicles opposed to NSC has major advantages in that they can be easily isolated from patients and grown in culture, have high metabolic activity and are readily transducible with integrating vectors [28, 29]. In this study we show that MSC can be used as a delivery vehicle for oHSV and show the dynamics of viral spread using oHSV mutants encoding diagnostic proteins and confocal fluorescent microscopy, and the efficacy of MSC-oHSV in clinically relevant GBM tumor models. Employing oHSV mutants bearing diagnostic proteins and combining bioluminescence imaging with serial histological analysis of brain sections by confocal microscopy, we were able to decipher the dynamic process of oHSV therapy of GBM and demonstrate that tumor regression is clearly correlated with viral spread and subsequent tumor cell oncolysis. Our results reveal that the robust changes in virus spread and oncolysis occur during the initial 48 hours after MSC-oHSV implantation, which may be crucial for overall therapeutic success. Comparison of therapeutic activity between MSC-oHSV and naked oHSV revealed that both potently induced tumor volume reduction. However, MSC-oHSV treatment resulted in superior efficacy on day 7 while purified oHSV only initially suppressed the tumors (Figure 8B). This difference may be associated with the different dynamics of virus production *in situ,* spread, and clearance after injection of MSC-oHSV and purified oHSV.

We have previously shown that encapsulation of stem cells in biodegradable sECM is a promising approach toward successful stem cell based therapy post-GBM resection [25]. Biodegradable sECMs ensure retention of therapeutic stem cells in the resection cavity and increase their viability as post-procedural secondary bleeding, inflammatory immune responses and influx of cerebrospinal fluid cause considerable physical and cellular stress [30]. Most of the experiments in this study were performed on Gli36vIII-GFI, which is an extremely proliferative GBM line and supports poorer oHSV replication rates than other GBM lines such as U87-GFI [12]. We showed that Gli36vIII-GFI can be successfully targeted with MSC-oHSV encapsulated in sECM *in vivo* despite its aggressive and difficult to treat nature. Utilizing a diagnostic oHSV variant, oHSV-Fluc, and real time BLI, we show that sECM-encapsulated MSC loaded with oHSV when transplanted in the tumor resection cavity released oHSV that retained at higher amounts and for a longer period in the brain when compared to conventional direct injection of purified oHSV. This persistence of oHSV when delivered via sECM encapsulated MSC results in suppression of tumor growth and significantly increased survival of animals treated as compared to oHSV alone.

We observed that the majority of Gli36vIII-GFl tumors, which were resected and treated with MSC-oHSV, eventually showed tumor relapse that should have originated from the tumor deposits which had escaped the therapy (Figure 3G). This is not surprising considering that the GBM line used for these studies, Gli36vIII, is a highly aggressive GBM line, with a median animal survival of only 14 days after implantation of 1x10⁵ cells (Figure 8D). Also, it was a part of our study design to leave about 20% of tumor mass un-resected to mimic the frequent clinical situations in which total GBM resection is not possible due to tumor-infiltration into functionally important brain areas [31, 32]. Therefore, large tumor residues can outgrow the oHSV spread emanating from the resection cavity, although oHSV have the capability of spreading among GBM cells and penetrating deeper into the tumor mass. MSC with tumor tracking capabilities have the potential to reach such invasive tumor deposits and deliver oHSV, but the oHSV toxicity of MSC and immature MSC death appear to be an obstacle. Control of viral replication in cellular vehicles may be applicable to address this issue. Recently a novel oHSV, KTR27, which has a tetracycline inducible promoter driving ICP27, one the critical immediately early genes for the replication of HSV has been developed. This oHSV can only replicate in the presence of tetracycline [33, 34] and offers a potential to load the MSC with KTR27, and activate the replication of the virus via tetracycline administration when MSC have migrated out of sECM post implantation in the tumor resection cavity. Tet-regulatable systems are likely to enter clinical trials in the years to come as they have been successfully used in a large variety of preclinical studies and could prove to be a powerful therapeutic tool in the future [34, 35].

We previously showed that oHSV susceptibility varies among GBM lines and some lines are resistant to oHSV mediated oncolysis, possibly due to factors such as a slower cell cycle, insufficient receptor expression and intact interferon response. This implies that patient GBM tumors have heterogenous responsiveness to oHSV and suggests the need to develop oHSV strategies that target a broad spectrum of GBM tumors. To improve antitumor efficacy of oHSV, some groups have armed oHSV with antiangiogenic and immunomodulatory molecules [36, 37]. In our previously published study we engineered an armed oHSV mutant encoding for secretable TRAIL and showed its ability to successfully target GBM lines that are both less permissive to oHSV mediated oncolysis and also resistant to TRAIL [12]. In the current study, we assessed the feasibility of using oHSV-TRAIL loaded MSC and showed that MSC are capable of amplifying oHSV-TRAIL, producing secretable TRAIL and inducing caspase mediated apoptosis in GBM lines non-permissive to oHSV and resistant to TRAIL. Finally, we showed that these results translate into *in vivo* efficacy and increase survival in a GBM resection model. Using MRI, we showed that contrast enhancement in T1-weighted image, which typically is a sign of tumor with leaky vasculature, was nearly undetectable in mice 15 days post MSC-oHSV-TRAIL treatment. This was in line with our BLI data, which showed only small remaining tumor residues compared to the animals treated with MSC-oHSV-mCh. Increased signal intensity area in T2-weighted image persisted around the original tumor implantation site after MSC-oHSV-TRAIL therapy, suggesting the existence of edema possibly associated with ongoing inflammatory or immune responses induced by oHSV-TRAIL.

In summary, our findings demonstrate the feasibility and impact of MSC delivery of oncolytic virotherapy in clinical scenarios of GBM resection, underlining the translatability of this approach. Moreover our results suggest that the stem cell based delivery of oHSV can overcome the problem associated with the current clinical practice involving direct oncolytic virus injection into resection cavities, which have produced minimal therapeutic effect. To our knowledge, this is the first report that describes a stem cell-based oHSV therapy against sensitive and multi-resistant GBM lines in a clinically relevant tumor resection model in mice. Finally, we show that MSC can be employed to target multi-resistant GBMs by loading them with a pro-apoptotic variant of oHSV, oHSV-TRAIL. Thus our results have direct implications for designing future clinical trials using oncolytic viruses for GBM therapy. Moreover, since different oHSV mutants have been widely used for the treatment of other malignancies as well [18, 37, 38], this study will have impact on the development of viral delivery systems in other solid tumors, such as liver, prostate, ovarian, breast and lung cancer.

### Materials and Methods

Parental and Engineered Cell lines: Human bone marrow-derived mesenchymal stem cells (MSC) (kindly provided by David Prockop, Tulane University, New Orleans) were grown in Alpha-MEM (Invitrogen/GIBCO) with 16.5% FBS 2- 4 mM L-glutamine and 100 U/mL penicillin and 100µg/mL streptomycin (P/S). GBM cell lines, Gli36vIII (Gli36 cells expressing EGFRvIII, a constitutively active variant of EGFR), U87, U251, LN319, U138, U251 and LN229 cells were cultured in DMEM supplemented with 10% FBS and P/S. MSC and GBM cell lines, LN229 and Gli36-vIII cells were transduced with LV-GFP-Fluc (GFl) or LV-GFP at MOI=2 in medium containing protamine sulfate (2 µg/ml). All cells were visualized by fluorescence microscopy for GFP expression 36 hours post transduction. Lentiviral packaging was performed by transfection of 293T cells as previously described [39].

Recombinant oncolytic herpes simplex viruses and viral growth assay. Recombinant oHSV vectors, G47Δ-TRAIL (oHSV-TRAIL) and G47Δ-Fluc (oHSV-Fluc) used in this study are described previously [12, 40-43]. G47Δ-TRAIL carries S-TRAIL cDNA driven by the IE4/5 immediate early promoter of HSV and G47Δ-Fluc carries firefly luciferase cDNA driven by cytomegalovirus immediate early promoter. oHSV-mCherry was generated by cloning mCherry cDNA under the IE4/5 immediate early promoter of HSV using the same BAC technique and the shuttle plasmid as with G47Δ-TRAIL. All the recombinant oHSVs express E.coli lacZ driven by endogenous ICP6 promoter. For viral production, MSC were infected with oHSV-mCherry (oHSV-mCh) or oHSV-TRAIL at MOI=15 in 24-well plates. After virus infection, media was replaced and viral titers from cell extracts and conditioned media at 12, 18, 24, 48, 72 and 96 hours post infection were determined by plaque assay on Vero cells (American Type Culture Collection) as described previously [12]. All experiments were performed in triplicates.

*In vitro* bioluminescence assays. For *in vitro* viability studies of oHSV-loaded MSC, cells were plated in 96-well plates (2x10³/well) and after 24h infected with oHSV for 4 hours at MOI=15. Cell viability was measured over 5 days by determining the aggregate cell metabolic activity using an ATP-dependent luminescent reagent (CellTiter-Glo, Promega, Madison, WI, USA) as described previously [29].

Cell viability and caspase activation in co-culture experiments. To determine the therapeutic effect of oHSV-loaded MSC in co-culture with GBM, GBM cells expressing GF1 (5x10³/well) and 5% MSC-oHSV-mCh or 10% MSC-oHSV-TRAIL were plated in a 96 well plate. The viability of GBM cells was assessed after 3 days by measuring the Fluc activity of GBM cells as described previously [44]. Caspase 3/7 activity of GBM cells in co-culture with 5% MSC-oHSV-mCh or 5% MSC-oHSV-TRAIL was determined at day 2 after plating the cells by using DEVD-aminoluciferin (CaspaseGlo 3/7, Promega) according to manufacturer's instructions. For encapsulation experiment, the sECM components, Hystem and Extralink (Glycosan Hystem-C, Biotime Inc.), were reconstituted according to the manufacturer's protocol. MSC cells (4x10⁴), MSC cells infected with oHSV-mCh for 4 hours at MOI 15 (4x10⁴) or the same amount of purified oHSV-mCh (6x10⁵ pfu) were resuspended in Hystem (10µl) and the matrix cross-linker (3µl) was added. sECM encapsulated MSC, purified oHSV-mCh or MSC-oHSV-mCh were plated in 24 wells and U87-GF1 cells (2x10⁴) were added to the plate and Fluc signal was measured at day 1, 4 and 6. All experiments were performed in triplicates.

Western blotting analysis and ELISA: For Western blotting analysis, LN229 GBM cells were co-cultured with MSC-oHSV in transwell plates Twenty four hours post plating, LN229 cells (5x10⁵/well) in 6-well plates, MSC-oHSV-mCh or MSC-oHSV-TRAIL were plated in 6-well transwell inserts (BD Biosciences). After 20 or 40 hours of incubation, transwell inserts were removed and LN229 cells were lysed with NP40 buffer supplemented with protease (Roche) and phosphatase inhibitors (Sigma). Twenty µg of harvested proteins from each lysate were resolved on 10% SDS-PAGE, and immunoblotted with antibodies against caspase-8 (Cell Signaling), caspase-9 (Stressgen), cleaved-PARP (Cell Signaling) or α-tubulin (Sigma); and detected by chemiluminescence after incubation with HRP-conjugated secondary antibodies (Santa Cruz). For assessment of TRAIL secretion from MSC-oHSV-TRAIL, 2x10⁴ MSC were infected with oHSV-TRAIL at MOI 15, and washed twice with PBS 4 hours post infection. The concentrations of TRAIL in the conditioned media collected at 0, 12, 24 and 48 hours post infection were determined by ELISA using a TRAIL Immunoassay Kit (Biosource International, Camarillo, CA) with recombinant hTRAIL expressed in *E. coli* as a standard [45].

Viability of oHSV-loaded MSC *in vivo*: To assess viability of MSC-oHSV, MSC-GF1 (1x10⁵/mouse; n=3) or MSC-GF1 infected with oHSV-mCh (1x10⁵/mouse; n=5) were stereotactically implanted (right striatum, 2.5-mm lateral from bregma and 2.5-mm deep) into the brains of SCID mice (6 weeks of age, Charles River Laboratories, Wilmington, MA). Bioluminescence imaging for Fluc activity was performed as described [29] and mice were sacrificed at 24, 48 and 120 hours post implantation to obtain brain sections for immunohistochemical analysis.

*In vivo* experiments in intact GBMs: To assess therapeutic effect of MSC-oHSV *in vivo,* Gli36vIII-GFl cells (5x10⁴/mouse) were stereotactically implanted into the brains of SCID mice (n=12) as described [29]. Tumor bearing mice were injected with MSC (1x10⁵/mouse, n=3), oHSV-mCh (5 ul of 5x10⁹ pfu/ml, n=3) or MSC-oHSV-mCh (2x10⁵/mouse, n=6) intratumorally at the same coordinate as the tumor cell implantation. Mice were followed for changes in tumor volumes by Fluc bioluminescence imaging (BLI) as described previously [29]. To obtain brain sections for immunohistochemical analysis, we performed the same experiment as above and mice were successively sacrificed over a period of 7 days. All *in vivo* procedures were approved by the Subcommittee on Research Animal Care at MGH.

*In vivo* experiments in resected GBMs: To assess the efficacy of MSC- oHSV in a mouse model of tumor resection, a cranial window was created over the original implantation site for tumor debulking using a SZX10 stereo microscope system (Olympus) for fluorescence guided surgery. One week later Gli36vIII-GFP (5x10⁴/mouse) were stereotactically implanted (right striatum, 2.5-mm lateral from bregma and 0.5-mm deep) into the brains of 10 mice and tumor debulking was performed 7 days post implantation as previously described [30, 46]. For MSC encapsulation, MSC-oHSV (2x10⁵) were resuspended in Hystem™ (Sigma Aldrich) (7µl) and the matrix cross-linker (3µl) was added. sECM encapsulated MSC-oHSV-Fluc (2x10⁵, n=5) or naked/purified oHSV-Fluc (10⁸ pfu in 10µl of volume) in mice (n=5) were injected into the resection cavity. Mice were serially imaged for Fluc activity over 12 days as described [25]. For survival studies, mice bearing Gli36vIII-GFl GBM tumors (n=20) underwent tumor debulking and were treated with sECM encapsulated MSC (2x10⁵/mouse, n=5), purified oHSV-mCh (10 ul of 10⁹ pfu/ml, n=5) or sECM encapsulated MSC-oHSV-mCh (2x10⁵/mouse, n=10). Mice were imaged for Fluc activity as well as followed for survival and sacrificed when neurological symptoms became apparent. For oHSV-TRAIL *in vivo* studies, LN229-GFl GBM cells (5x10⁵ cells/mouse) were implanted (n=12) and 21 days later tumor debulking was performed as described above, followed by injection of sECM-encapsulated MSC (2x10⁵, n=4), sECM-encapsulated MSC-oHSV-mCh (2x10⁵, n=4) or sECM-encapsulated MSC-oHSV-TRAIL (2x10⁵, n=4). Using another set of mice (n=12) we performed the same experiment with sECM-encapsulated MSC-oHSV-mCh (n=6) or sECM-encapsulated MSC-oHSV-TRAIL (n=6) to follow mice survival. Bioluminescence and MR imaging were performed over a period of 9 and 15 days, respectively. All *in vivo* procedures were approved by the Subcommittee on Research Animal Care at MGH.

Tissue processing and immunohistochemistry. Mice were perfused by pumping ice-cold 4% paraformaldehyde (PFA) directly into the heart and the brains were fixed in 4% PFA and frozen sections were obtained for H&E staining, immunohistochemistry and confocal microscopic analysis. 5-Bromo-4-choloro-3-indolyl-β-D-galactopyranoside (X-gal) staining was performed to identify lacZ-expressing infected cells as described previously [12].

Statistical analysis. Data were analyzed by Student t-test when comparing 2 groups. Data were expressed as mean ± standard deviations *in vitro* studies and standard errors *in vivo* studies and differences were considered significant at p<0.05. Kaplan-Meier analysis was used for mouse survival studies and the groups were compared using the log-rank test.

### References for Example 1A and Background section

1. Wen, P.Y. and S. Kesari, Malignant gliomas in adults. N Engl J Med, 2008. 359(5): p. 492-507.
2. Johnson, D.R. and S.M. Chang, Recent medical management of glioblastoma. Adv Exp Med Biol, 2012. 746: p. 26-40.
3. Johannessen, T.C. and R. Bjerkvig, Molecular mechanisms of temozolomide resistance in glioblastoma multiforme. Expert Rev Anticancer Ther, 2012. 12(5): p. 635-42.
4. Barr, J.G. and P.L. Grundy, The effects of the NICE Technology Appraisal 121 (Gliadel and temozolomide) on survival in high-grade glioma. Br J Neurosurg, 2012. 26(6): p. 818-22.
5. Aghi, M. and R.L. Martuza, Oncolytic viral therapies - the clinical experience. Oncogene, 2005. 24(52): p. 7802-16.
6. Liu, T.C., E. Galanis, and D. Kirn, Clinical trial results with oncolytic virotherapy: a century of promise, a decade of progress. Nat Clin Pract Oncol, 2007. 4(2): p. 101-17.
7. Markert, J.M., et al., Conditionally replicating herpes simplex virus mutant, G207 for the treatment of malignant glioma: results of a phase I trial. Gene Ther, 2000. 7(10): p. 867-74.
8. Wakimoto, H., et al., Human glioblastoma-derived cancer stem cells: establishment of invasive glioma models and treatment with oncolytic herpes simplex virus vectors. Cancer Res, 2009. 69(8): p. 3472-81.
9. Varghese, S. and S.D. Rabkin, Oncolytic herpes simplex virus vectors for cancer virotherapy. Cancer Gene Ther, 2002. 9(12): p. 967-78.
10. Hoffmann, D. and O. Wildner, Comparison of herpes simplex virus- and conditionally replicative adenovirus-based vectors for glioblastoma treatment. Cancer Gene Ther, 2007. 14(7): p. 627-39.
11. Todo, T., "Armed" oncolytic herpes simplex viruses for brain tumor therapy. Cell Adh Migr, 2008. 2(3): p. 208-13.
12. Tamura, K., et al., Multimechanistic Tumor Targeted Oncolytic Virus Overcomes Resistance in Brain Tumors. Mol Ther, 2012.
13. Markert, J.M., et al., Phase Ib trial of mutant herpes simplex virus G207 inoculated pre-and post-tumor resection for recurrent GBM. Mol Ther, 2009. 17(1): p. 199-207.
14. Harrow, S., et al., HSV1716 injection into the brain adjacent to tumour following surgical resection of high-grade glioma: safety data and long-term survival. Gene Ther, 2004. 11(22): p. 1648-58.
15. Rampling, R., et al., Toxicity evaluation of replication-competent herpes simplex virus (ICP 34.5 null mutant 1716) in patients with recurrent malignant glioma. Gene Ther, 2000. 7(10): p. 859-66.
16. Papanastassiou, V., et al., The potential for efficacy of the modified (ICP 34.5(-)) herpes simplex virus HSV1716 following intratumoural injection into human malignant glioma: a proof of principle study. Gene Ther, 2002. 9(6): p. 398-406.
17. Garcia-Castro, J., et al., Treatment of metastatic neuroblastoma with systemic oncolytic virotherapy delivered by autologous mesenchymal stem cells: an exploratory study. Cancer Gene Ther, 2010. 17(7): p. 476-83.
18. Coukos, G., et al., Use of carrier cells to deliver a replication-selective herpes simplex virus-1 mutant for the intraperitoneal therapy of epithelial ovarian cancer. Clin Cancer Res, 1999. 5(6): p. 1523-37.
19. Komarova, S., et al., Mesenchymal progenitor cells as cellular vehicles for delivery of oncolytic adenoviruses. Mol Cancer Ther, 2006. 5(3): p. 755-66.
20. Raykov, Z., et al., Carrier cell-mediated delivery of oncolytic parvoviruses for targeting metastases. Int J Cancer, 2004. 109(5): p. 742-9.
21. Jevremovic, D., et al., Use of blood outgrowth endothelial cells as virus-producing vectors for gene delivery to tumors. Am J Physiol Heart Circ Physiol, 2004. 287(2): p. H494-500.
22. Crittenden, M., et al., Pharmacologically regulated production of targeted retrovirus from T cells for systemic antitumor gene therapy. Cancer Res, 2003. 63(12): p. 3173-80.
23. Tyler, M.A., et al., Neural stem cells target intracranial glioma to deliver an oncolytic adenovirus in vivo. Gene Ther, 2009. 16(2): p. 262-78.
24. Yong, R.L., et al., Human bone marrow-derived mesenchymal stem cells for intravascular delivery of oncolytic adenovirus Delta24-RGD to human gliomas. Cancer Res, 2009. 69(23): p. 8932-40.
25. Kauer, T.M., et al., Encapsulated therapeutic stem cells implanted in the tumor resection cavity induce cell death in gliomas. Nat Neurosci, 2012. 15(2): p. 197-204.
26. Zemp, F.J., et al., Oncolytic viruses as experimental treatments for malignant gliomas: using a scourge to treat a devil. Cytokine Growth Factor Rev, 2010. 21(2-3): p. 103-17.
27. Wollmann, G., K. Ozduman, and A.N. van den Pol, Oncolytic virus therapy for glioblastoma multiforme: concepts and candidates. Cancer J, 2012. 18(1): p. 69-81.
28. Pereboeva, L., et al., Approaches to utilize mesenchymal progenitor cells as cellular vehicles. Stem Cells, 2003. 21(4): p. 389-404.
29. Sasportas, L.S., et al., Assessment of therapeutic efficacy and fate of engineered human mesenchymal stem cells for cancer therapy. Proc Natl Acad Sci U S A, 2009. 106(12): p. 4822-7.
30. Kauer, T.M., et al., Encapsulated therapeutic stem cells implanted in the tumor resection cavity induce cell death in gliomas. Nat Neurosci, 2011.
31. Kong, D.S., et al., Preservation of quality of life by preradiotherapy stereotactic radiosurgery for unresectable glioblastoma multiforme. J Neurosurg, 2006. 105 Suppl: p. 139-43.
32. Lesimple, T., et al., Topotecan in combination with radiotherapy in unresectable glioblastoma: a phase 2 study. J Neurooncol, 2009. 93(2): p. 253-60.
33. Yao, F., et al., Highly efficient regulation of gene expression by tetracycline in a replication-defective herpes simplex viral vector. Mol Ther, 2006. 13(6): p. 1133-41.
34. Yao, F., et al., Development of a regulatable oncolytic herpes simplex virus type 1 recombinant virus for tumor therapy. J Virol, 2010. 84(16): p. 8163-71.
35. Chaturvedi, A.K., et al., Validation of the tetracycline regulatable gene expression system for the study of the pathogenesis of infectious disease. PLoS One, 2011. 6(5): p. e20449.
36. Zhang, W., et al., Bevacizumab with angiostatin-armed oHSV increases antiangiogenesis and decreases bevacizumab-induced invasion in U87 glioma. Mol Ther, 2012. 20(1): p. 37-45.
37. Castelo-Branco, P., et al., Oncolytic herpes simplex virus armed with xenogeneic homologue of prostatic acid phosphatase enhances antitumor efficacy in prostate cancer. Gene Ther, 2010. 17(6): p. 805-10.
38. Li, J., et al., Treatment of breast cancer stem cells with oncolytic herpes simplex virus. Cancer Gene Ther, 2012. 19(10): p. 707-14.
39. Shah, K., et al., Bimodal viral vectors and in vivo imaging reveal the fate of human neural stem cells in experimental glioma model. J Neurosci, 2008. 28(17): p. 4406-13.
40. Fukuhara, H., et al., Triple gene-deleted oncolytic herpes simplex virus vector double-armed with interleukin 18 and soluble B7-1 constructed by bacterial artificial chromosome-mediated system. Cancer Res, 2005. 65(23): p. 10663-8.
41. Kuroda, T., et al., Flip-Flop HSV-BAC: bacterial artificial chromosome based system for rapid generation of recombinant herpes simplex virus vectors using two independent site-specific recombinases. BMC Biotechnol, 2006. 6: p. 40.
42. Saeki, Y., et al., Herpes simplex virus type 1 DNA amplified as bacterial artificial chromosome in Escherichia coli: rescue of replication-competent virus progeny and packaging of amplicon vectors. Hum Gene Ther, 1998. 9(18): p. 2787-94.
43. Yamamoto, S., et al., Imaging immediate-early and strict-late promoter activity during oncolytic herpes simplex virus type 1 infection and replication in tumors. Gene Ther, 2006. 13(24): p. 1731-6.
44. Corsten, M.F., et al., MicroRNA-21 knockdown disrupts glioma growth in vivo and displays synergistic cytotoxicity with neural precursor cell delivered S-TRAIL in human gliomas. Cancer Res, 2007. 67(19): p. 8994-9000.
45. Kock, N., et al., Tumor therapy mediated by lentiviral expression of shBcl-2 and S-TRAIL. Neoplasia, 2007. 9(5): p. 435-42.
46. Hingtgen, S., et al., Real-time multi-modality imaging of glioblastoma tumor resection and recurrence. J Neurooncol, 2013. 111(2): p. 153-61.

### Example 1B: Stem Cells Loaded with Multi-mechanistic oHSV Variants for Brain Tumor Therapy

(Example 1B describes an updated interpretation of some of the data shown in Figures 1-10 referred to in Example 1A. As such, some of Figure 1- Figure 10 are referred to in both Example 1A and Example IB.)

Glioblastoma multiforme (GBM) is the most common brain tumor in adults and despite great advances in its molecular understanding it remains one of the most difficult to treat malignancies (1). Although GBM tumor resection constitutes an important therapeutic intervention, standard treatment with radiation and temozolomide chemotherapy post-tumor resection only provide modest clinical benefits (2, 3). Previous studies attempting to use local therapy with clinically approved Gliadel wafers, polyanhydride wafers containing the chemotherapeutic agent BCNU, in the cavity of resected GBM, have been shown to have limited therapeutic benefit (4). Oncolytic viruses have shown great potential in treating tumors in preclinical studies(5-8) Oncolytic Herpes Simplex Virus (oHSV) is inherently neurotropic and one of the most promising candidates for GBM therapy (5, 9, 10).

Although phase I and Ib clinical trials using oHSV for GBMs post-resection have shown anti-tumor activity, clinical response rates have been sub-optimal (7, 11-14). This could be partly due to the secondary bleeding caused by the surgical intervention and influx of cerebrospinal fluid into the resection cavity rinsing out injected virus(15, 16). To improve delivery of viral therapeutics and circumvent antiviral immunity, a number of studies have explored the possibility of using infected cells as delivery vehicles for oncolytic viruses (17-23). Mesenchymal stem cells (MSC) have shown great promise in this respect and several studies have employed MSC for delivery of oncolytic adenoviruses to GBM (17, 19, 23, 24). Although promising, these studies have been limited by their inability to explore the therapeutic efficacy of MSC loaded oncolytic viruses that could be translated into clinics for treatment of GBM patients. In our previous studies, we employed biodegradable synthetic extracellular matrix (sECMs) that are based on a thiol-modified hyaluronic acid (HA) and a thiol reactive cross-linker (polyethylene glycol diacrylate) and shown that sECM encapsulation enhances retention and the therapeutic potential of engineered stem cells within the resection cavity (25).

In this study we loaded human MSC with oHSV (MSC-oHSV) and explored the dynamics of MSC-oHSV in real time *in vitro* and *in vivo* in resected GBM models. Using novel armed oHSV mutants, we then tested the efficacy of oHSV and a pro-apoptotic oHSV variant (oHSV-TRAIL) loaded MSC encapsulated in biocompatible sECM in clinically applicable mouse models which more accurately reflect the current clinical setting of GBM tumor aggressiveness, resistance and resection.

### Results

### MSC as a cellular delivery vehicle for oHSV

To assess whether MSC are capable of serving as a cellular delivery vehicle for oHSV, we first studied oHSV replication and release of infectious viral particles in human MSC *in vitro.* Human MSC were infected with a G47Δ-based recombinant oHSV in which cDNA encoding the mCherry fluorescent protein is placed under the IE4/5 immediate-early promoter of HSV (oHSV-mCh) (29). Infection of MSC with oHSV-mCh resulted in exponential amplification of virus during the first 24 hours (Figure 11A), which was associated with marker protein mCherry expression and resulted in decreasing MSC survival *in vitro* (Figure 11B-E). To assess the survival of oHSV-mCherry loaded MSC (MSC-oHSV-mCh) *in vivo,* MSC were first engineered to express a bimodal fluorescent and bioluminescent fused protein, GFP-firefly luciferase (Fluc) fusion protein (GF1). A significant decrease in cell viability was seen MSC-GFI loaded oHSV-mCh implanted into the brains of mice as compared to the controls (p=0.0057; Figure 11F). Brain sections of mice at different time points after oHSV-mCh injection showed cytopathic effect of virus-loaded, mCh+ MSC as virus amplification and mCh expression intensified, eventually resulting in cell lysis with mCh+ cell debris (Figure 11G-I).

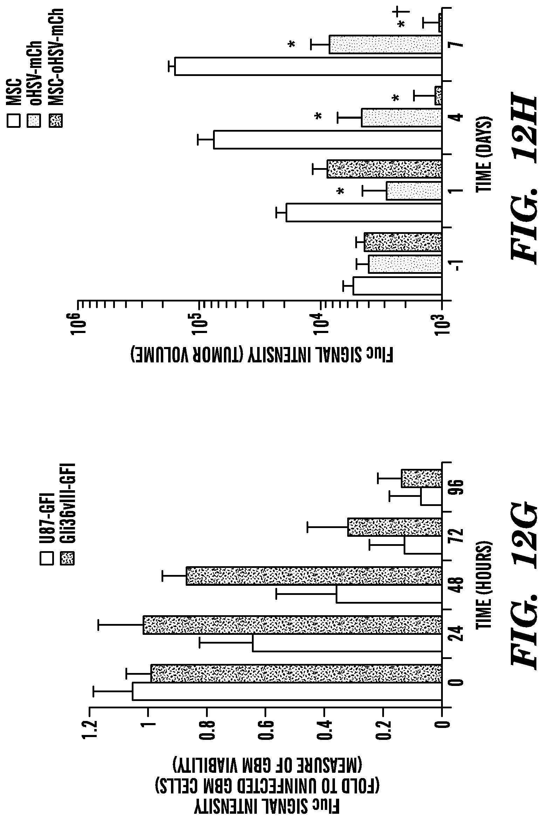

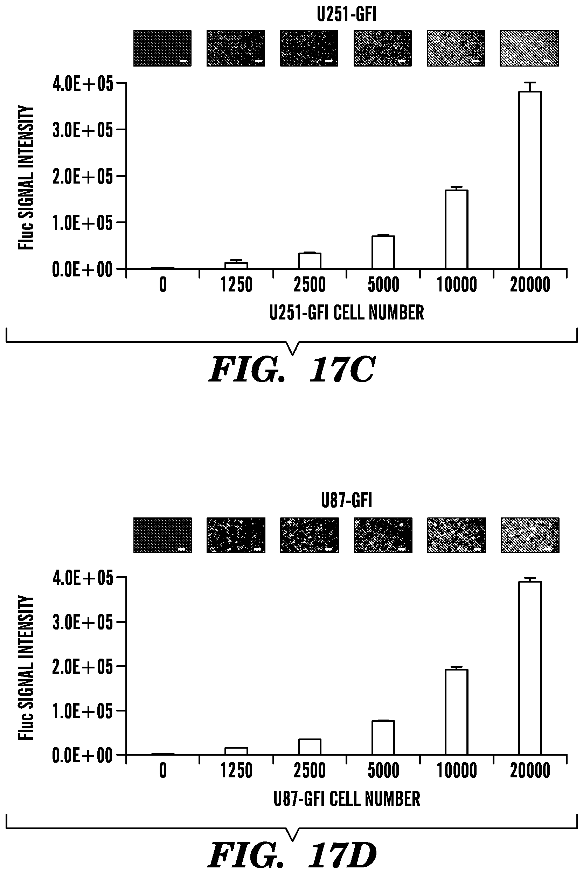

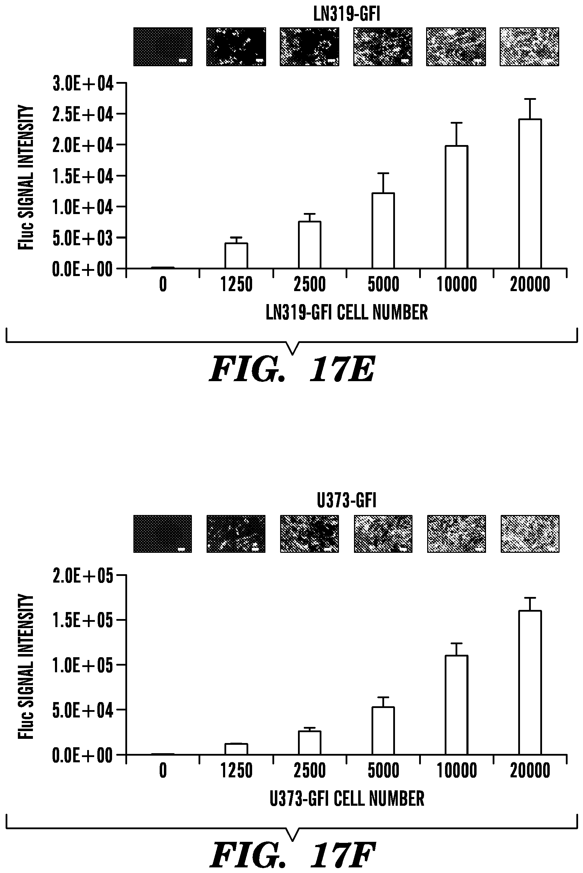

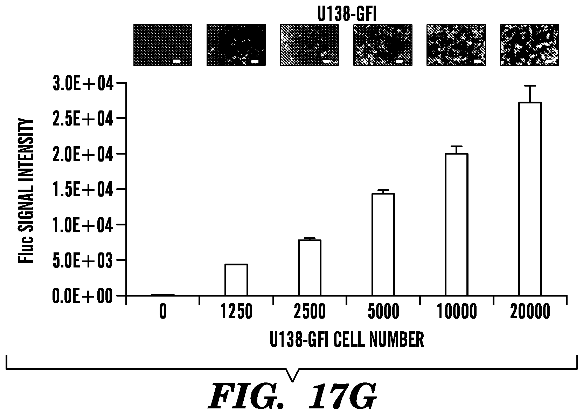

To assess the oncolytic activity of MSC-oHSV-mCh in GBM cells, we utilized human GBM cell lines Gli36vIII (highly proliferating) and U87 (intermediately proliferating) engineered to express the diagnostic marker GF1, Gli36vIII-GFl and U87-GFl. A direct association between GBM-GFl cell numbers, Fluc signal intensity and GFP-positive cells were seen *in vitro* within the ranges tested (Figure 17). Release of oHSV-mCh from MSC resulted in the infection of engineered GBM cells and spread among them leading to extensive oncolysis (Figure 12A-F). *In vitro,* co-culture of these GBM lines with MSC-oHSV-mCh resulted in drastic cell killing of both highly proliferating Gli36vIII-GFl (Figure 12A-C,G) and intermediately proliferating U87-GFl (Figure 12D-F, G) GBM cells. Next, MSC-oHSV-mCh co-cultured with GBMs resulted in a significantly increased killing of oHSV sensitive (U87, U251, U373 and Gli36vIII,) and oHSV resistant (LN229, U138 and LN319) GBM cells (Figure 18). Despite the aggressiveness of Gli36vIII-GFl GBM *in vivo* (Figure 19), intratumoral injection of oHSV-mCh or MSC-oHSV-mCh in mice bearing Gli36vIII-GFl tumors resulted in a significant tumor volume reduction as compared to the control MSC injection (oHSV-mCh= 95% ± 2%, p<0.001; MSC-oHSV-mCh= 99.4% ±0.5%, p<0.001) (Figure 12H). Interestingly, we observed significantly more potent antitumor effect in MSC-oHSV-mCh than concentrated oHSV-mCh group (87.5% tumor volume reduction in MSC-oHSV-mCh compared to oHSV-mCh, p=0.049) (Figure 12H). In order to test if systemic delivery of MSC-oHSV-mCh could be used to treat intracranial tumors, Gli36vIII tumor bearing mice were treated intravenously with MSC-FmC. BLI imaging revealed that intravenously injected MSC did not to home to the tumors in the brain and got trapped in the lungs (Figure 20). These results reveal that oHSV-mCh loaded MSC effectively produce oHSV progeny, which results in effective killing of GBM cells *in vitro* as well as in established GBMs *in vivo.*

### Dynamics of oHSV infection and oncolysis in vivo

In order to investigate the dynamics of oHSV spread and GBM cell killing mediated by oHSV loaded MSC, mice bearing established highly proliferating Gli36vIII-GFl tumors were treated with MSC-oHSV-mCh. Multi-color fluorescence imaging of serial brain sections showed rapid spread of oHSV-mCh emanating from MSC-oHSV-mCh implantation site with concomitant shrinkage of Gli36vIII-GFl tumor area (visualized as green) within 96 hours (Figure 13A-D). At 24 hours, a significant number of yellow cells (GFP+ mCherry+) emerged around the MSC implantation site, confirming infection of tumor cells (Figure 13E-F, white arrowheads). oHSV amplification and spread penetrating into tumor tissue was seen at 48 hours and rounded tumor cells showing weakened GFP expression was observed indicating widespread cytopathic effect (Figure 13G, black arrowheads). After 72 hours the forefront of mCherry+ area extended to near tumor periphery, leaving vast areas of mCh+ cell debris behind and considerably reduced areas of GFP+ virus-free tumor (Figure 13C, I). Continuing rounds of tumor infection occurred at the borders between the mCherry+ and GFP+ regions as shown by GFP+ mCherry+ (visualized as yellow) tumor cells for at least 72 hours post implantation (Figure 13F, H, J, white arrowheads) with infected GBM cells at varying stages of initial infection, cytopathic effect and final cell lysis (Figure 13K). X-gal staining on adjacent brain sections revealed that an area of cells positive for oHSV reporter lacZ was almost exactly superimposable on the combined mCherry+ and mCherry+GFP+ (visualized as yellow) area confirming that mCherry+ and mCherry+ GFP+ cells are oHSV-infected (Figure 13L). Quantification of the fluorescent imaging results revealed a continuous increase of oHSV-mCh-infected cells (visualized as red) and a concurrent decrease of unimpaired tumor cells (visualized as green), with the most dramatic changes of tumor infection and virus replication taking place within the first 48 hours post MSC-oHSV-mCh implantation (Figure 13M). Our multi-color fluorescence imaging thus revealed the dynamic process of oHSV infection and tumor destruction mediated by oHSV-loaded MSC *in vivo.*

### MSC-mediated delivery of oHSV in a mouse model of GBM resection

We have recently developed a clinically relevant mouse model of GBM resection and shown the sECM encapsulation of therapeutic MSC allows retention of a significant number of MSC in the GBM tumor resection cavity resulting in higher therapeutic efficacy (30). Based on these studies, we first assessed the production and release of oHSV-mCh from sECM encapsulated MSC. *In vitro* sECM-MSC-oHSV-mCh produced oHSV-mCh during the first 24 hours and reached a plateau 36 hours post-infection (Figure 21A). Further, co-culture of sECM encapsulated MSC-oHSV-mCh with U87-GFl GBM cells significantly reduced GBM cell viability over time compare with sECM encapsulated oHSV-mCh (p=0.0041) and sECM-MSC (p=0.0017) (Figure 21B-C). We then sought to determine whether oHSV delivery by sECM encapsulated MSC increases oHSV persistence and oncolytic activity in a clinically relevant model of GBM resection when compared to direct injection of concentrated oHSV. We utilized an *in vivo* imageable version of G47Δ recombinant oHSV in which cDNA encoding firefly luciferase (Fluc) is placed under the cytomegalovirus immediate early promoter (oHSV-Fluc) (31). sECM encapsulated MSC loaded with oHSV-Fluc (MSC-oHSV-Fluc, 3x10⁶ pfu when loading) led to significantly increased expression of Fluc when compared to conventional direct injection of purified oHSV-Fluc (1x10⁸ pfu) in the tumor resection cavity of the pre-established Gli36vIII-GFP tumors (p=0.039) (Figure 4A). These results show that sECM encapsulated oHSV loaded MSC allows significantly longer persistence of oHSV infection than the oHSV alone tumor resection cavity.

In order to compare the therapeutic efficacy of sECM encapsulated MSC-oHSV-mCh with direct injection of oHSV-mCh into the resection cavity, mice bearing established Gli36vIII-GFl tumors (Figure 14B-C) underwent subtotal GBM resection (Figure 14D) and were treated with sECM-encapsulated MSC-oHSV-mCh (Figure 14E) or purified oHSV-mCh. A significant suppression of tumor growth (oHSV-mCh vs sECM-MSC-oHSV-mCh p=0.0039) and increased median survival time was seen in mice treated with sECM-MSC-oHSV-mCh as compared to the mice treated with purified oHSV-mCh.(MSC-oHSV-mCh group:36.1 days;, MSC group: 17.8 days; and purified oHSV-mCh group; 9.4 days) (p<0.001 with Gehan-Breslow-Wilcoxin test; Figure 14F-G). We also assessed the viral yield of intratumorally transplanted MSC-oHSV-mCh by X-gal staining of serially collected brain sections. oHSV-mCh initially produced by MSC (Day 1) infected neighboring GBM tumor cells, which was followed by further oHSV-mCh propagation in tumor cells (Day 3) (Figure 22A-B). Furthermore, brain sections from mice treated with MSC-oHSV-mCh did not show evidence of oHSV infection in peritumoral normal brain (neurons and astrocytes) 12 days post treatment (Figure 22C), confirming the safety of this approach. These results demonstrate that encapsulated MSC-oHSV results in an increased anti-GBM efficacy compared to direct injection of purified oHSV in a preclinical model of GBM resection possibly due to long lasting production of oHSV in the vicinity of GBM deposits.

### MSC-mediated delivery of an armed oHSV mutant

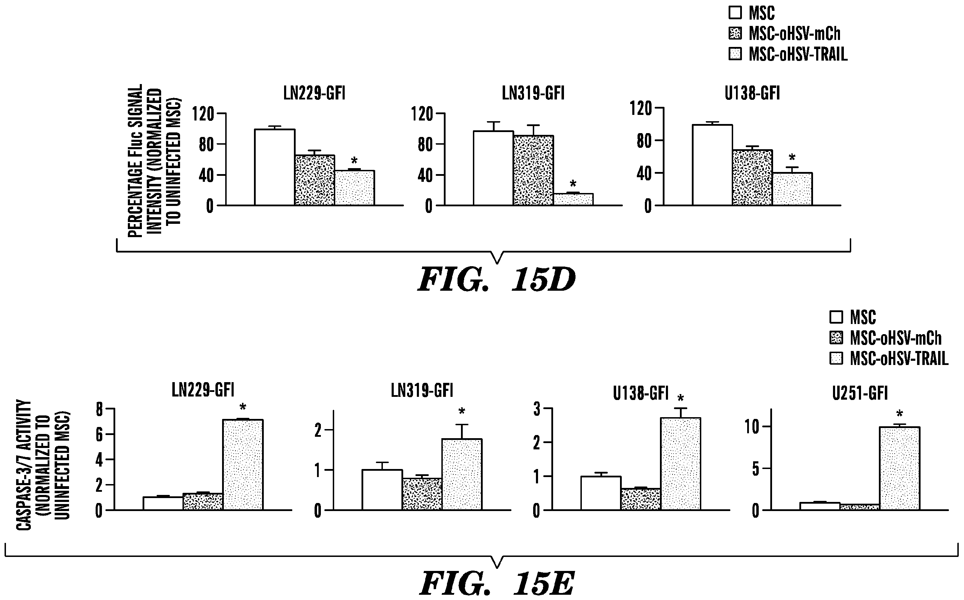

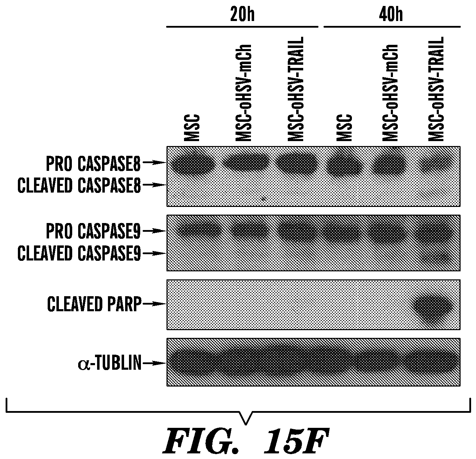

We recently created an armed version of G47Δ recombinant oHSV in which cDNA encoding secretable TRAIL is placed under the IE4/5 immediate-early promoter of HSV (oHSV-TRAIL) and showed that it targets a broad spectrum of GBM lines including oHSV resistant and TRAIL resistant lines (31). To develop MSC loaded oHSV therapies for a broad spectrum of GBMs, we next investigated whether MSC loaded with oHSV-TRAIL could target both oHSV and TRAIL resistant GBM lines. oHSV-TRAIL released from MSC loaded oHSV-TRAIL (MSC-oHSV-TRAIL) exponentially amplified during the first 36 hours and reached a plateau 48 hours post-infection (Figure 15A). Similar to MSC-oHSV-mCh, a time dependent decrease in MSC viability was seen in MSC-oHSV-TRAIL over 120 hours (Figure 15B). Time course ELISA on MSC-oHSV-TRAIL confirmed the release of S-TRAIL into the culture media over time (Figure 15C). As compared to MSC-oHSV-mCh or MSC-TRAIL treatment, MSC-oHSV-TRAIL treatment resulted in significantly greater cell killing when co-cultured with different engineered GBM lines that are either fully or semi-resistant to TRAIL and have low susceptibility to oHSV mediated oncolysis (31) (Figure 15D; Fig 23A-C; Figure 24A). GBM cell killing by MSC-oHSV-TRAIL was mediated by activated caspase-3/7 (Figure 15E; Figure 24B). Western blotting analysis of LN229 GBM cell lysates obtained from transwell inserts culture showed a significant increase in cleaved caspase-8, cleaved caspase-9 and cleaved PARP in MSC-oHSV-TRAIL treatment group as compared to controls (Figure 15F). These results show that MSC loaded with the armed oHSV mutant encoding secretable TRAIL effectively produce oHSV-TRAIL progeny and induce apoptosis mediated killing of both oHSV- and TRAIL-resistant GBM.

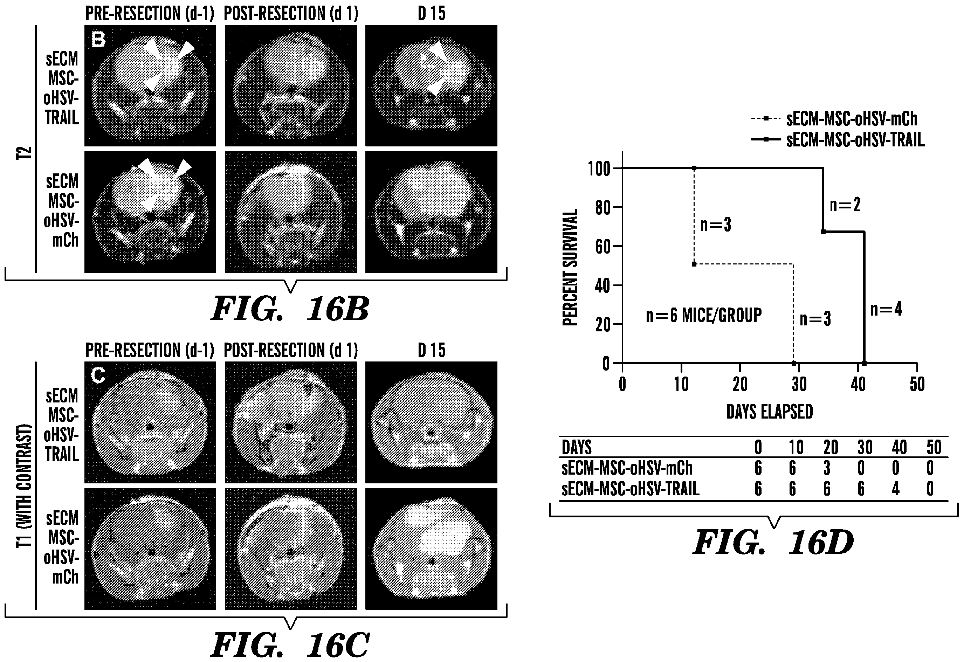

To test *in vivo* efficacy of MSC-oHSV-TRAIL in a clinically relevant GBM mouse model, mice bearing established LN229-GFl tumors underwent GBM resection followed by injection of sECM-encapsulated MSC, MSC-oHSV-mCh or MSC-oHSV-TRAIL. A suppression in the relapse of LN229-GFI tumors was seen in the sECM-MSC-oHSV-TRAIL treated as compared to the controls (Figure 16A). T2-weighted magnetic resonance imaging (MRI) showed a localized high signal intensity area at the site of sECM-MSC-oHSV-TRAIL injection on day 1 post-resection, which persisted for 2 weeks (Figure 16B). T1-weighted MRI with contrast confirmed the efficacy by sECM-encapsulated MSC-oHSV-TRAIL as it revealed sustained tumor regression after treatment as compared to the controls (Figure 16C). This anti-GBM activity by sECM-MSC-oHSV-TRAIL resulted in significant prolongation of median survival time of mice (38.6 days) as compared to the sECM-MSC-oHSV-mCh treated group (20.5 days; p <0.001 with Chi-square contingency test, Figure 16D). These results demonstrate that MSC can serve as a robust cellular delivery vehicle for oHSV armed with pro-apoptotic molecule, and when applied within sECM to a clinically relevant mouse model of GBM resection, this treatment modality targets resistant GBM, resulting in significant survival benefit (p=0.038).

### Discussion

In this study we show the dynamics of diagnostic oHSV mutants, oHSV-mCh and oHSV-Fluc delivered by MSC (MSC-oHSV) in real time *in vitro* and *in vivo* in mouse models of GBMs. We also show the efficacy of sECM encapsulated MSC-oHSV and its pro-apoptotic variant MSC-oHSV-TRAIL in clinically applicable mouse models that represent clinical scenarios of tumor resection and resistance.

In an effort to circumvent the issues dampening the current oHSV trials in GBM, we sought to develop a cell-based strategy to deliver oHSV that takes into account the challenges found in a clinical scenario of GBM resection. We have previously shown that both human NSC and MSC can home to tumors in the brain, can effectively deliver therapeutic proteins on site resulting in significant therapeutic efficacy (26, 32, 33). The use of MSC as delivery vehicles opposed to NSC has major advantages in that they can be easily isolated from patients and grown in culture and have high metabolic activity (26, 34). Employing oHSV mutants bearing diagnostic proteins and combining bioluminescence imaging, our results revealed that the robust changes in virus spread and oncolysis occurred during the initial 48 hours after MSC-oHSV implantation, which may be crucial for overall therapeutic success. Comparison of therapeutic activity between MSC-oHSV and naked oHSV revealed that both potently induced tumor volume reduction. However, MSC-oHSV treatment resulted in superior efficacy that may be associated with the different dynamics of virus production *in situ,* spread, and clearance after injection of MSC-oHSV and purified oHSV.

We have previously shown that encapsulation of stem cells in biodegradable sECM is a promising approach toward successful stem cell based therapy post-GBM resection (30). Most of the experiments in this study were performed on Gli36vIII-GFI, which is an extremely proliferative GBM line and supports poorer oHSV replication rates than other GBM lines such as U87-GFI (31). We showed that Gli36vIII-GFI can be successfully targeted with MSC-oHSV encapsulated in sECM *in vivo* despite its aggressive and difficult-to-treat nature. Utilizing a diagnostic oHSV variant, oHSV-Fluc, and real time BLI, we show that sECM-encapsulated MSC loaded with oHSV when transplanted in the tumor resection cavity released oHSV for a longer period in the brain when compared to conventional direct injection of purified oHSV. This persistence of oHSV when delivered via sECM encapsulated MSC results in suppression of tumor growth and significantly increased survival of animals treated as compared to oHSV alone.

We previously showed that oHSV susceptibility varies among GBM lines and some lines are resistant to oHSV mediated oncolysis (31). This implies that patient GBM tumors have heterogenous responsiveness to oHSV and suggests the need to develop oHSV strategies that target a broad spectrum of GBM tumors. In our previously published study we engineered an armed oHSV mutant encoding secretable TRAIL and showed its ability to successfully target GBM lines that are both less permissive to oHSV mediated oncolysis and also resistant to TRAIL (31). In the current study, we assessed the feasibility of using oHSV-TRAIL loaded MSC and showed that MSC are capable of amplifying oHSV-TRAIL, producing secretable TRAIL and inducing caspase mediated apoptosis in GBM lines non-permissive to oHSV and resistant to TRAIL.

In this study preclinical GBM models were used that are based on conventional GBM cell lines, which may not represent the phenotypic and genotypic hallmarks of GBM (35). Future work will establish that MSC loaded with oHSV retain their efficacy in mouse models generated with patient-derived tumor initiating cells that mirror the human disease and provide the challenge of tumor invasion (36). Although MSC are known to be non-immunogenic following transplantation (37), it would be ideal to use a patient's own MSC or reprogrammed induced pluripotent cells loaded with oHSV and its variants (38). We envisage that, after the neurosurgical removal of the main tumor mass, the patient's own reprogrammed cells or MSC loaded with different variants of oHSV tailored to the molecular profile of the tumor, will be encapsulated in sECM and used in patients post GBM-resection.

In summary, our findings demonstrate the feasibility and impact of MSC delivery of oncolytic virus in clinical scenarios of GBM resection, underlining the translatability of this approach. Stem cell based delivery of oHSV can overcome the problems associated with the current clinical practice involving direct oncolytic virus injection into resection cavities, which have produced minimal therapeutic effect. Thus our results have direct implications for designing future clinical trials using oncolytic viruses for GBM therapy. Since different oHSV mutants have been widely used for the treatment of different cancer types (18, 39, 40), this study will have an impact on the development of viral delivery systems in other solid tumors, such as liver, prostate, ovarian, breast and lung cancer.

### Materials and Methods

Parental and engineered cell lines. Human bone marrow-derived mesenchymal stem cells (MSC) (kindly provided by David Prockop, Tulane University, New Orleans) and growth as previously described (26). Gli36vIII (Gli36 cells expressing EGFRvIII, a constitutively active variant of EGFR), U87, U251, LN319, U138, U251 and LN229 GBM cell lines were obtained from the American Type Culture Collection (ATCC, Manassas, VA) and grown as described previously (27). MSC and GBM cell lines, LN229 and Gli36-vIII cells were transduced with LV-GFP-Fluc (GFl) or LV-GFP at MOI=2 in medium containing protamine sulfate (2 µg/ml). All cells were visualized by fluorescence microscopy for GFP expression 36 hours post transduction. Lentiviral packaging was performed by transfection of 293T cells as previously described (28).

Recombinant oncolytic herpes simplex viruses and viral growth assay. G47Δ-TRAIL carries S-TRAIL cDNA driven by the IE4/5 immediate early promoter of HSV and G47Δ-Fluc carries firefly luciferase cDNA driven by cytomegalovirus immediate early promoter. oHSV-mCherry was generated by cloning mCherry cDNA under the IE4/5 immediate early promoter of HSV using the same BAC technique and the shuttle plasmid as with G47Δ-TRAIL (29). All the recombinant oHSVs express E.coli lacZ driven by endogenous ICP6 promoter.

*In vivo* mouse experiments. Female SCID mice (6-8 weeks old) obtained from Charles River laboratories (Wilmington, MA) were used in three different *in vivo* experiments. All the animal care procedures were approved by the Subcommittee on Research Animal Care at MGH.

To assess cell viability of MSC-oHSV, MSC-GF1 (mice; n=3) or MSC-GF1 infected with oHSV-mCh (n=5), MSC were stereotactically implanted into the brains of mice and bioluminescence imaging was performed as described in the Supplementary Methods (shown below).

To assess the therapeutic effects of MSC-oHSV, Gli36vIII-GFl cells were stereotactically implanted into the brains of SCID mice (n=12) and tumor bearing mice were injected with MSC (n=3), oHSV-mCh (n=3) or MSC-oHSV-mCh (n=6) intratumorally at the same coordinate as the tumor cell implantation. Mice were followed for changes in tumor volumes by Fluc bioluminescence imaging (BLI).

To assess the efficacy of MSC-oHSV or MSC-oHSV-TRAIL in a mouse model of tumor resection, a cranial window was created over the original implantation site for tumor debulking One week later, Gli36vIII-GFl or LN229-GFl were stereotactically implanted into the brains of 10 mice and tumor debulking was performed 7 days (Gli36vIII-GFl) or 21 days (LN229-GFl) post implantation as described in the Supplementary Materials (shown below). sECM encapsulated MSCs or naked/purified oHSV were injected into the resection cavity (mice: n=5). Mice were serially imaged for Fluc activity as described in the Supplementary Methods (shown below).

For survival studies, mice bearing Gli36vIII-GFl GBM tumors (n=20) underwent tumor debulking and were treated with sECM encapsulated MSC (n=5), purified oHSV-mCh (n=5) or sECM encapsulated MSC-oHSV-mCh (n=10). Mice were imaged for Fluc activity as well as followed for survival and sacrificed when neurological symptoms became apparent. For oHSV-TRAIL *in vivo* studies, LN229-GFl GBM cells were implanted (n=12) and 21 days later tumor debulking was performed followed by injection of sECM-encapsulated MSC-oHSV-mCh (n=6) or sECM-encapsulated MSC-oHSV-TRAIL (n=6) and mice were followed for survival.

Statistical analysis. Data were analyzed by Student t-test when comparing 2 groups. Data were expressed as mean ± standard deviations *in vitro* studies and standard errors *in vivo* studies. Differences were considered statistically significant at p<0.05. Kaplan-Meier analysis was used for mouse survival studies and the groups were compared using Gehan-Breslow-Wilcoxin test, or the Chi-square contingency test. All the statistical tests were two-sided.

### References (Example 1B only)

1. Wen PY, Kesari S. Malignant gliomas in adults. N Engl J Med 2008;359(5):492-507.
2. Johnson DR, Chang SM. Recent medical management of glioblastoma. Adv Exp Med Biol 2012;746:26-40.
3. Johannessen TC, Bjerkvig R. Molecular mechanisms of temozolomide resistance in glioblastoma multiforme. Expert Rev Anticancer Ther 2012;12(5):635-42.
4. Barr JG, Grundy PL. The effects of the NICE Technology Appraisal 121 (Gliadel and temozolomide) on survival in high-grade glioma. Br J Neurosurg 2012;26(6):818-22.
5. Aghi M, Martuza RL. Oncolytic viral therapies - the clinical experience. Oncogene 2005;24(52):7802-16.
6. Liu TC, Galanis E, Kirn D. Clinical trial results with oncolytic virotherapy: a century of promise, a decade of progress. Nat Clin Pract Oncol 2007;4(2):101-17.
7. Markert JM, Medlock MD, Rabkin SD, et al. Conditionally replicating herpes simplex virus mutant, G207 for the treatment of malignant glioma: results of a phase I trial. Gene Ther 2000;7(10):867-74.
8. Wakimoto H, Kesari S, Farrell CJ, et al. Human glioblastoma-derived cancer stem cells: establishment of invasive glioma models and treatment with oncolytic herpes simplex virus vectors. Cancer Res 2009;69(8):3472-81.
9. Varghese S, Rabkin SD. Oncolytic herpes simplex virus vectors for cancer virotherapy. Cancer Gene Ther 2002;9(12):967-78.
10. Hoffmann D, Wildner O. Comparison of herpes simplex virus- and conditionally replicative adenovirus-based vectors for glioblastoma treatment. Cancer Gene Ther 2007;14(7):627-39.
11. Markert JM, Liechty PG, Wang W, et al. Phase Ib trial of mutant herpes simplex virus G207 inoculated pre-and post-tumor resection for recurrent GBM. Mol Ther 2009;17(1):199-207.
12. Harrow S, Papanastassiou V, Harland J, et al. HSV1716 injection into the brain adjacent to tumour following surgical resection of high-grade glioma: safety data and long-term survival. Gene Ther 2004;11(22):1648-58.
13. Rampling R, Cruickshank G, Papanastassiou V, et al. Toxicity evaluation of replication-competent herpes simplex virus (ICP 34.5 null mutant 1716) in patients with recurrent malignant glioma. Gene Ther 2000;7(10):859-66.
14. Papanastassiou V, Rampling R, Fraser M, et al. The potential for efficacy of the modified (ICP 34.5(-)) herpes simplex virus HSV1716 following intratumoural injection into human malignant glioma: a proof of principle study. Gene Ther 2002;9(6):398-406.
15. Mohyeldin A, Chiocca EA. Gene and viral therapy for glioblastoma: a review of clinical trials and future directions. Cancer J 2012;18(1):82-8.
16. Kaur B, Chiocca EA, Cripe TP. Oncolytic HSV-1 virotherapy: clinical experience and opportunities for progress. Curr Pharm Biotechnol 2012;13(9):1842-51.
17. Garcia-Castro J, Alemany R, Cascallo M, et al. Treatment of metastatic neuroblastoma with systemic oncolytic virotherapy delivered by autologous mesenchymal stem cells: an exploratory study. Cancer Gene Ther 2010;17(7):476-83.
18. Coukos G, Makrigiannakis A, Kang EH, et al. Use of carrier cells to deliver a replication-selective herpes simplex virus-1 mutant for the intraperitoneal therapy of epithelial ovarian cancer. Clin Cancer Res 1999;5(6):1523-37.
19. Komarova S, Kawakami Y, Stoff-Khalili MA, et al. Mesenchymal progenitor cells as cellular vehicles for delivery of oncolytic adenoviruses. Mol Cancer Ther 2006;5(3):755-66.
20. Raykov Z, Balboni G, Aprahamian M, et al. Carrier cell-mediated delivery of oncolytic parvoviruses for targeting metastases. Int J Cancer 2004;109(5):742-9.
21. Jevremovic D, Gulati R, Hennig I, et al. Use of blood outgrowth endothelial cells as virus-producing vectors for gene delivery to tumors. Am J Physiol Heart Circ Physiol 2004;287(2):H494-500.
22. Crittenden M, Gough M, Chester J, et al. Pharmacologically regulated production of targeted retrovirus from T cells for systemic antitumor gene therapy. Cancer Res 2003;63(12):3173-80.
23. Tyler MA, Ulasov IV, Sonabend AM, et al. Neural stem cells target intracranial glioma to deliver an oncolytic adenovirus in vivo. Gene Ther 2009;16(2):262-78.
24. Yong RL, Shinojima N, Fueyo J, et al. Human bone marrow-derived mesenchymal stem cells for intravascular delivery of oncolytic adenovirus Delta24-RGD to human gliomas. Cancer Res 2009;69(23):8932-40.
25. Kauer TM, Figueiredo JL, Hingtgen S, et al. Encapsulated therapeutic stem cells implanted in the tumor resection cavity induce cell death in gliomas. Nat Neurosci 2011.
26. Sasportas LS, Kasmieh R, Wakimoto H, et al. Assessment of therapeutic efficacy and fate of engineered human mesenchymal stem cells for cancer therapy. Proc Natl Acad Sci U S A 2009;106(12):4822-7.
27. Martinez-Quintanilla J, Bhere D, Heidari P, et al. Therapeutic efficacy and fate of bimodal engineered stem cells in malignant brain tumors. Stem Cells 2013;31(8):1706-14.
28. Shah K, Hingtgen S, Kasmieh R, et al. Bimodal viral vectors and in vivo imaging reveal the fate of human neural stem cells in experimental glioma model. J Neurosci 2008;28(17):4406-13.
29. Cheema TA, Wakimoto H, Fecci PE, et al. Multifaceted oncolytic virus therapy for glioblastoma in an immunocompetent cancer stem cell model. Proc Natl Acad Sci U S A 2013;110(29):12006-11.
30. Kauer TM, Figueiredo JL, Hingtgen S, et al. Encapsulated therapeutic stem cells implanted in the tumor resection cavity induce cell death in gliomas. Nat Neurosci 2012;15(2):197-204.
31. Tamura K, Wakimoto H, Agarwal AS, et al. Multimechanistic Tumor Targeted Oncolytic Virus Overcomes Resistance in Brain Tumors. Mol Ther 2012.
32. Shah K, Bureau E, Kim DE, et al. Glioma therapy and real-time imaging of neural precursor cell migration and tumor regression. Ann Neurol 2005;57(1):34-41.
33. Corsten MF, Shah K. Therapeutic stem-cells for cancer treatment: hopes and hurdles in tactical warfare. Lancet Oncol 2008;9(4):376-84.
34. Pereboeva L, Komarova S, Mikheeva G, et al. Approaches to utilize mesenchymal progenitor cells as cellular vehicles. Stem Cells 2003;21(4):389-404.
35. Lee J, Kotliarova S, Kotliarov Y, et al. Tumor stem cells derived from glioblastomas cultured in bFGF and EGF more closely mirror the phenotype and genotype of primary tumors than do serum-cultured cell lines. Cancer Cell 2006;9(5):391-403.
36. Wakimoto H, Mohapatra G, Kanai R, et al. Maintenance of primary tumor phenotype and genotype in glioblastoma stem cells. Neuro Oncol 2012;14(2):132-44.
37. Shah K. Mesenchymal stem cells engineered for cancer therapy. Adv Drug Deliv Rev 2012;64(8):739-48.
38. Somoza RA, Rubio FJ. Cell therapy using induced pluripotent stem cells or somatic stem cells: this is the question. Curr Stem Cell Res Ther 2012;7(3):191-6.
39. Li J, Zeng W, Huang Y, et al. Treatment of breast cancer stem cells with oncolytic herpes simplex virus. Cancer Gene Ther 2012;19(10):707-14.
40. Castelo-Branco P, Passer BJ, Buhrman JS, et al. Oncolytic herpes simplex virus armed with xenogeneic homologue of prostatic acid phosphatase enhances antitumor efficacy in prostate cancer. Gene Ther 2010;17(6):805-10.

### Supplemental Materials and Methods

oHSV viral growth assay. MSC were infected with oHSV-mCh or oHSV-TRAIL at MOI=15 in 24-well plates (2x10⁴ cells/well). After virus infection, cells were washed with PBS, media was replaced and cells and media collected at 12, 18, 24, 48, 72 and 96 hours post infection. Samples were subject to three cycles of freeze/thaw and viral titers were determined by plaque assay on Vero cells (American Type Culture Collection) To quantify the viral yield from MSC-oHSV-mCh encapsulated in sECM, MSC were infected with oHSV-mCh, washed with PBS, encapsulated in sECM and plated in 24-well plates (2x10⁴ cells/well) and the viral titer was determined as described above. All experiments were performed in triplicates.

*In vitro* bioluminescence assays. For *in vitro* viability studies of oHSV-loaded MSC, cells were plated in 96-well plates (2x10³/well) and after 24h infected with oHSV for 4 hours at MOI=15. Cell viability was measured over 5 days by determining the aggregate cell metabolic activity using an ATP-dependent luminescent reagent (CellTiter-Glo, Promega, Madison, WI, USA) according to manufacturer's instructions.

Cell viability and caspase activation in co-culture experiments. To determine the therapeutic effect of oHSV-loaded MSC in co-culture with GBM, GBM cells expressing GFl (5x10³/well) and 5% MSC-TRAIL, 5% MSC-oHSV-mCh or 10% MSC-oHSV-TRAIL were plated in a 96 well plate. The viability of GBM cells was assessed after 3 days by measuring the Fluc activity of GBM cells Specifically, the culture medium was replaced with 250 µl of fresh medium containing 1 mg/ml of d-luciferin (Biotium).The plates were incubated for 10 min at room temperature and Fluc activity was imaged using a liquid nitrogen charged cooled device (CCD) (Roper Scientific, Trenton, NJ). Caspase 3/7 activity of GBM cells in co-culture with 5% MSC-TRAIL, 5% MSC-oHSV-mCh or 5% MSC-oHSV-TRAIL was determined at day 2 after plating the cells by using DEVD-aminoluciferin (CaspaseGlo 3/7, Promega) according to manufacturer's instructions. For encapsulation experiment, the sECM components, Hystem and Extralink (Glycosan Hystem-C, Biotime Inc.), were reconstituted according to the manufacturer's protocol. MSC cells (4x10⁴), MSC cells infected with oHSV-mCh for 4 hours at MOI 15 (4x10⁴) or the same amount of purified oHSV-mCh (6x10⁵ pfu) were resuspended in Hystem (10µl) and the matrix cross-linker (3µl) was added. sECM encapsulated MSC, purified oHSV-mCh or MSC-oHSV-mCh were plated in 24 wells and U87-GF1 cells (2x10⁴) were added to the plate and Fluc signal was measured at day 1, 4 and 6. All experiments were performed in triplicates and repeated two times.

Western blotting analysis and ELISA. For Western blotting analysis, LN229 GBM cells were co-cultured with MSC-oHSV in transwell plates Twenty four hours post plating, LN229 cells (5x10⁵/well) in 6-well plates, MSC-oHSV-mCh or MSC-oHSV-TRAIL were plated in 6-well transwell inserts (BD Biosciences). After 20 or 40 hours of incubation, transwell inserts were removed and LN229 cells were lysed with NP40 buffer supplemented with protease (Roche) and phosphatase inhibitors (Sigma). Twenty µg of harvested proteins from each lysate were resolved on 10% SDS-PAGE, and immunoblotted with antibodies against caspase-8 (Cell Signaling), caspase-9 (Stressgen), cleaved-PARP (Cell Signaling) or α-tubulin (Sigma); and detected by chemiluminescence after incubation with HRP-conjugated secondary antibodies (Santa Cruz). For assessment of TRAIL secretion from MSC-oHSV-TRAIL, 2x10⁴ MSC were infected with oHSV-TRAIL at MOI 15, and washed twice with PBS 4 hours post infection. The concentrations of TRAIL in the conditioned media collected at 0, 12, 24 and 48 hours post infection were determined by ELISA using a TRAIL Immunoassay Kit (Biosource International, Camarillo, CA) with recombinant hTRAIL expressed in *E. coli* as a standard [1]. All experiments were performed in triplicates and repeated two times.

Viability of oHSV-loaded MSC *in vivo.* To assess viability of MSC-oHSV, MSC-GFl (1x10⁵/mouse; n=3) or MSC-GFl infected with oHSV-mCh (1x10⁵/mouse; n=5) were stereotactically implanted (right striatum, 2.5-mm lateral from bregma and 2.5-mm deep) into the brains of SCID mice (6 weeks of age, Charles River Laboratories, Wilmington, MA). Bio luminescence imaging for Fluc activity was performed 10 min. after mice were given intraperitoneal (i.p.) injection of D-luciferin (1.5 mg/25 g body weight; prepared in 150 µL saline) using a cryogenically cooled high efficiency CCD camera system (Roper Scientific, Trenton, NJ) and mice were sacrificed at 24, 48 and 120 hours post implantation to obtain brain sections for immunohistochemical analysis.

*In vivo* experiments in intact GBMs. To assess therapeutic effect of MSC-oHSV *in vivo,* Gli36vIII-GFl cells (5x10⁴/mouse) were stereotactically implanted into the brains of SCID mice (n=12) as described [2]. Tumor bearing mice were injected with MSC (1x10⁵/mouse, n=3), oHSV-mCh (5 µl of 5x10⁹ pfu/ml, n=3) or MSC-oHSV-mCh (2x10⁵/mouse, n=6) intratumorally at the same coordinate as the tumor cell implantation. Mice were followed for changes in tumor volumes by Fluc bioluminescence imaging (BLI) as described previously [2]. To obtain brain sections for immunohistochemical analysis, we performed the same experiment as above and mice were successively sacrificed over a period of 7 days. To assess the efficacy of MSC-oHSV delivered intravenously, Gli36vIII-GFP tumor bearing mice were treated intravenously (tail vein injection of MSC engineered to express FmC; 1x10⁵/mouse, n=3) and the fate of MSC-FmC was assessed by BLI imaging over time.

*In vivo* experiments in resected GBMs. To assess the efficacy of MSC-oHSV or MSC-oHSV-TRAIL in a mouse model of tumor resection, a cranial window was created over the original implantation site for tumor debulking using a SZX10 stereo microscope system (Olympus) for fluorescence guided surgery. One week later, Gli36vIII-GFl (5x10⁴/mouse) or LN229-GFl (5x10⁵ cells/mouse) were stereotactically implanted (right striatum, 2.5-mm lateral from bregma and 0.5-mm deep) into the brains of 10 mice and tumor debulking was performed 7 days (Gli36vIII-GFl) or 21 days (LN229-GFl) post implantation as previously described [3]. For MSC encapsulation, MSC, MSC-oHSV-Fluc, MSC-oHSV-mCh or MSC-TRAIL (2x10⁵) were resuspended in Hystem (7µl) and the matrix cross-linker (3µl) was added. sECM encapsulated MSCs or naked/purified oHSV (10⁸ pfu in 10µl of volume) were injected into the resection cavity (n=5). Mice were serially imaged for Fluc activity as described above.

Persistence of oHSV in resected GBMs. To allow the persistence of oHSV in mouse model of tumor resection, a cranial window was created and Gli36vIII-GFP (5x10⁴/mouse) were stereotactically implanted into the brains of 10 mice and tumor debulking was performed 7 days post implantation as previously described [3, 4]. sECM encapsulated MSC-oHSV-Fluc (2x10⁵, n=5) or naked/purified oHSV-Fluc (10⁸ pfu in 10µl of volume) were injected into the resection cavity. Mice were serially imaged for Fluc activity over 12 days as described above.

Mice survival in resected GBMs. For survival studies, mice bearing Gli36vIII-GFl GBM tumors (n=20) underwent tumor debulking and were treated with sECM encapsulated MSC (2x10⁵/mouse, n=5), purified oHSV-mCh (10ul of 10⁹ pfu/ml, n=5) or sECM encapsulated MSC-oHSV-mCh (2x10⁵/mouse, n=10). Mice were imaged for Fluc activity as well as followed for survival and sacrificed when neurological symptoms became apparent. For oHSV-TRAIL *in vivo* studies, LN229-GFl GBM cells (5x10⁵ cells/mouse) were implanted (n=12) and 21 days later tumor debulking was performed followed by injection of sECM-encapsulated MSC-oHSV-mCh (2x10⁵, n=6) or sECM-encapsulated MSC-oHSV-TRAIL (2x10⁵, n=6) and mice were followed for survival.

Tissue processing and immunohistochemistry. Mice were perfused by pumping ice-cold 4% paraformaldehyde (PFA) directly into the heart and the brains were fixed in 4% PFA and frozen sections were obtained for H&E staining, immunohistochemistry and confocal microscopic analysis. To identify lacZ-expressing infected cells, X-gal staining was performed by incubating brain sections with a mixture of 5-Bromo-4-choloro-3-indolyl-β-D-galactopyranoside (1 mg/ml), Potassium ferricyanide (5 mM) Potassium ferrocyanide trihydrate (5 mM), and MgCl₂ (2 mM) for 5 hours at 37°C For NeuN and GFAP staining to identify neurons and astrocytes, respectively, sections were incubated with primary antibodies for NeuN (Millipore, 1: 200) or GFAP (Sigma, 1: 400). After using the Vectastain Elite ABC kit (Vector) following manufacturer's instruction, immunopositivity was visualized with diaminobenzidine (Dako).

### References for Supplemental Materials and Methods

1. Kock N, Kasmieh R, Weissleder R, et al. Tumor therapy mediated by lentiviral expression of shBcl-2 and S-TRAIL. Neoplasia 2007;9(5):435-42.
2. Sasportas LS, Kasmieh R, Wakimoto H, et al. Assessment of therapeutic efficacy and fate of engineered human mesenchymal stem cells for cancer therapy. Proc Natl Acad Sci U S A 2009;106(12):4822-7.
3. Kauer TM, Figueiredo JL, Hingtgen S, et al. Encapsulated therapeutic stem cells implanted in the tumor resection cavity induce cell death in gliomas. Nat Neurosci 2011.
4. Hingtgen S, Figueiredo JL, Farrar C, et al. Real-time multi-modality imaging of glioblastoma tumor resection and recurrence. J Neurooncol 2013;111(2):153-61.

### Example 2: Targeting metastatic brain tumors with engineered stem cell carrying oncolytic herpes simplex virus

Metastatic brain tumors are the most frequent neoplasms of adult's central nervous system, and are ten times more common than primary brain tumors (1), which most commonly originate from lung cancer, breast cancer or skin cancer (melanoma). Among these, melanoma has the highest propensity to metastasize to the brain, occurring in over 50% of all melanoma patients with advanced disease (2, 3). The frequency of tumor incidence in the brain is rising, due to better treatment strategies of the primary tumor as well as improved clinical diagnosis. Mortality rates of both lung and breast cancer have decreased (4); yet, there continues to be an increase in the melanoma death rate, with brain metastases contributing to half of all melanoma-related deaths (5). The clinical presentation of melanoma brain metastases differs from that of breast or lung cancer brain metastasis as there is a higher incidence of multiple intracranial lesions and a greater hemorrhagic tendency (6, 7). Therefore, many melanoma patients with brain metastases are not candidates for either surgical resection or stereotactic radiosurgery despite the potential survival benefits (8). Even with optimal treatment, however, patients with melanoma brain metastases have a median survival of only 3-6 months (9). As such, to develop new and efficient therapeutic approaches targeting melanoma brain metastases is urgently needed.

Development and preclinical testing of new cancer therapies is limited by the scarcity of in vivo models that authentically reproduce tumor growth and metastatic progression. Most of previous studies have utilized either sub-cutaneous or intracranial injection of tumor cells directly into the brain parenchyma, which does not mimic the actual clinical settings of melanoma brain metastases, such as initial arrest of tumor cells at the brain capillaries, extravasation, continuation of perivascular position, vessel co-option, micrometastatic growth, and macro metastatic growth. In order to mimic a majority of the steps of metastatic colonization and blood vessel interactions, we have successfully created an in vivo imageable mouse model of melanoma brain metastases by intracarotid injection of malignant patient derived melanoma cells, which were previously isolated from the metastatic sites of patient with advanced melanoma. In order to real-time track brain tumor growth in vivo, we engineered the melanoma cells to express both fluorescent protein mCherry and bioluminescent protein Fluc. Further, using non-invasive bioluminescent imaging (BLI), we then assessed the temporal and spatial distribution of the metastases in the mouse brain. This new mouse model will provide us a unique and valuable platform to test existing and novel therapeutic approaches.

Oncolytic viruses have shown great potential in treating tumors in preclinical studies (10). Among them, oncolytic herpes simplex viruses (oHSV) are inherently neurotropic (11), tumor selective (12) and have shown promising therapeutic efficacy in treating advanced melanomas in pre-clinical studies (13). This has led to the use of oHSV in clinical trials in melanoma patients (14). Although these results are promising for extracranial melanomas, there are no studies focused on melanoma brain metastases, which is the major cause for melanoma-related mortality. To target multiple melanoma lesions with oHSV in the brain parenchymal, systemic delivery of virus will be required. However, virus neutralization, sequestration and inefficient extravasation are major barriers for efficient oHSV delivery via the bloodstream (12). Here, the unique ability of adult stem cells to home to intracranial pathologies (15) and to package and efficiently deliver oHSV (16) makes them ideal candidates for targeting melanoma metastatic lesions in the brain. In this study, we aim to test the therapeutic efficacy of systemically delivered stem cells carrying oHSV in an in vivo imageable mouse model of melanoma brain metastases.

### Results

### Characterization of melanoma brain metastases in vivo

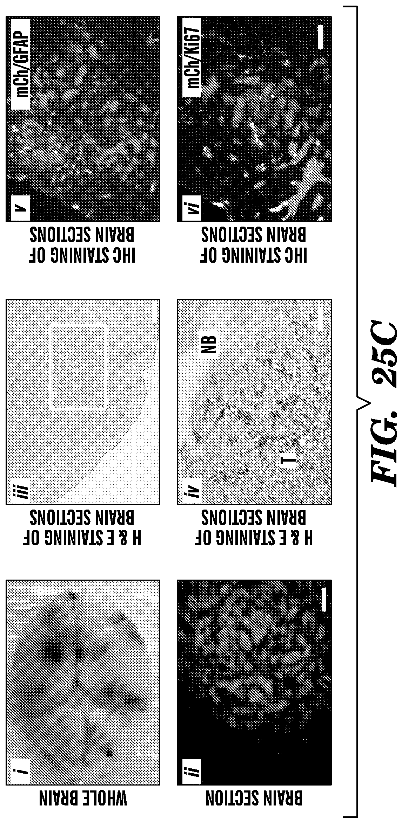

To establish an in vivo animal model that can recapitulate the steps of metastatic progression and be imaged noninvasively, we engineered MeWo cells, which were previously isolated from metastatic sites of patient with advanced melanoma, to express firefly luciferase (Fluc) and mCherry using a bicistronic lentiviral vector (referred as MeWo-FmC, Figure 25A). In order to mimic a majority of the steps of metastatic colonization and blood vessel interactions, we injected MeWo-FmC cells via intracarotid artery in the immuno-compromised mice to build up the mouse model of melanoma brain metastases. Non-invasive bioluminescence imaging (BLI) on tumor bearing mice revealed exclusive brain metastases and the exponentially growth of metastatic tumor in the brain 3 weeks after tumor injection (Figure 25B, Figure 29). Numerous pigmented metastatic tumor deposits were found within the brain parenchymal and the fluorescent images confirmed the presence of MeWo-FmC cells within macrometastatic foci (Figure 25C, i and ii). Photomicrograph of hematoxylin and eosin (H&E) staining of metastatic melanoma lesions in the brain revealed the invasion of melanoma cells into adjacent normal brain (Figure 25C, iii and iv). Further immunohistochemistry results showed GFAP or Ki67 immunostaining on cryostat brain sections from tumor bearing mice, indicating that metastatic melanoma cells (mCherry+) surrounded by reactive astrocytes (GFAP+) and are still proliferative (Ki67+, Figure 25C, v and vi). Here, our results showed that the intracarotid injection of melanoma brain metastatic tumor cells resulted in the formation of a clinically-relevant mouse model that resembles the multi-foci metastases observed in the clinics.

### Dynamics of oHSV infection and oncolysis in vitro

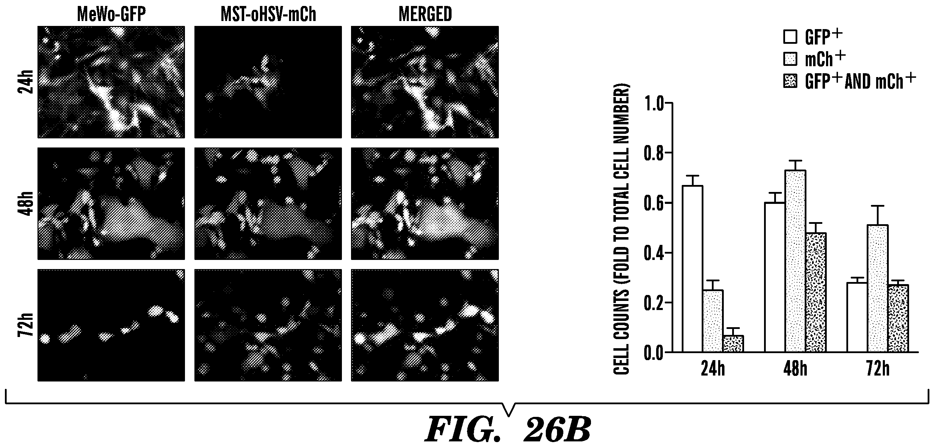

To investigate the dynamics of oHSV spread and oHSV-mediated cell killing in melanoma cells, we first screened multiple melanoma lines for their sensitivity to oHSV infection and oncolysis. We choose malignant human melanoma lines (MeWo, TXM-13, MALME-3M, SK-mel-2, and SK-mel-28) for the further experiment, considering both the metastatic capabilities and the status of BRAF and NRAS, two most common mutated genes found in melanoma patients. In order to visualize the spread and amplification of oHSV within melanoma cells, we engineered a G47delta-based recombinant oHSV in which cDNA encoding the mCherry fluorescent protein is placed under the IE4/5 immediate-early promoter of HSV (oHSV-mCh). Infection of multiple melanoma lines with oHSV-mCh even at MOI=0.1 resulted in smooth spread of oHSV among those tumor cells over time (Figure 26A). In addition, cell viabilities measured 4 and 6 days post oHSV infection at different MOI, revealing the robust cell killing effect achieved in most of the melanoma lines (Figure 26B and C), while regardless of their heterogenous genetic background and different metastatic capabilities. The results thus indicated that oHSV targeted a broad spectrum of malignant metastatic melanoma lines and had a robust cell-killing effect.

### MST as a cellular delivery vehicle for oHSV

To target multiple metastatic lesions in the brain, here we used MST cell as a cell carrier for on-site delivering oHSV. The unique ability of adult stem cells to home to intracranial pathologies (15) and to package and efficiently deliver oHSV (16) makes them ideal candidates for this purpose. To assess the spread of oHSV-mCh within MST and the survival of oHSV-mCh-loaded MST (MST-oHSV-mCh) in vitro, MST was infected with oHSV-mCh at different MOI, fluorescent images were collected over time and cell viability was measured as well. The increasing expression of marker protein mCherry within MST over time indicated that both the spread and amplification of oHSV-mCh were relative quick and efficient (Figure 30A). Cell viability of MST loaded with oHSV at different MOI over time showed that MST can survive at least 4 days post infection (Figure 30B), justifying the use of MST for in vivo delivery of oHSV. To further evaluate the oncolytic activity of MST-oHSV-mCh in melanoma cells, we used human malignant melanoma cells MeWo engineered to express green fluorescent protein (MeWo-GFP). Co-culture experiment of MeWo-GFP and MST freshly loaded with oHSV-mCh (MOI=1) showed that, the release of oHSV-mCh from MST resulted in the infection of adjacent MeWo-GFP cells and spread of oHSV-mCh among melanoma cells (representative as both GFP+ and mCherry+), leading to extensive oncolysis and the dramatically decreased tumor cell number (Figure 27A and B). These results suggested that oHSV can be carried by MST and killed the adjacent tumor cells upon release from MST.

### MST-mediated delivery of oHSV in a mouse model of melanoma brain metastases

In order to visualize the activity and dynamics of oHSV delivered by MST in vivo, we engineered oHSV to express Fluc (oHSV-Fluc), freshly loaded MST with oHSV-Fluc (MST-oHSV-Fluc) and intracarotid injection of MST-oHSV-Fluc in both brain tumor bearing mice and normal mice, followed by BLI imaging for tracking the oHSV in vivo. In tumor bearing mice, Fluc signal can be detected as early as day 1 post MST-oHSV-Fluc injection, with a significant increase from day 1 to day 3 and the peak signal achieved at day 5 (Figure 28A and B). However, in normal mice injected with MST-oHSV-Fluc, Fluc signal slightly increased at day 3 while was lost in the following days. It is thus indicated that oHSV carried by MST was able to amplify and sustain for couple of days in the brain of tumor bearing mice instead of normal mice. To further confirm the presence of oHSV within brain tumor lesions and study the infection of oHSV into adjacent melanoma cells after release from lysed MST in vivo, mice bearing MeWo-GFP brain tumors were intracarotidly injected with MST-oHSV-mCh, then sacrificed at different time points for cryostat brain sections. Multicolor fluorescence imaging of serial brain sections showed a rapid spread of oHSV-mCh emanating from a small population of MST-oHSV-mCh within melanoma deposits in the brain (Figure 28C).

We then sought to determine the therapeutic efficacy of the novel MST-oHSV virotherapy administered via intracarotid artery in a mouse model of melanoma brain metastases (Figure 29A). Representative BLI images of MST or MST-oHSV treated tumor-bearing mice showed a dramatic remission of metastatic tumor burden in the brain (Figure 29B and C). In addition, there is a significant survival benefit achieved by MST-oHSV treatment in mice bearing melanoma brain metastases (Figure 29D). These results demonstrate that MST can serve as a robust cellular delivery vehicle for therapeutic oHSV, and when applied via intracarotid artery to a clinically relevant mouse model of melanoma brain metastases, this treatment modality targets the metastatic deposits in the brain, resulting in a statistically significantly survival benefit.

### Discussion

In current study, we first developed and extensively characterized an in vivo imageable model of melanoma brain metastases that displays various features of brain metastases in advanced melanoma patients. Based on the metastatic brain tumor model, we then developed and tested the therapeutic efficacy of Multi-stem cell based virotherapy. Our results demonstrated that 1), MST is an ideal cell carrier for oHSV in vivo delivery due to its innate capability of homing to intracranial pathological lesions and ability of packaging oHSV; 2), oHSV can be delivered on-site upon the release from MST's oncolysis, infect adjacent tumor cells and kill them; 3), administration of MST-oHSV therapy via circulation can successfully target multiple tumor lesions, suppress metastatic brain tumor growth and achieve significant survival benefits in a mouse model of melanoma brain metastases.

Most of previous studies have utilized either sub-cutaneous or intracranial injection of tumor cells directly into the brain parenchyma, which does not mimic the actual clinical settings of metastatic melanoma. In order to mimic a majority of the steps of metastatic colonization and blood vessel interactions, we have successfully created an in vivo imageable mouse model of melanoma brain metastases by intracarotid injection of metastatic melanoma cells, which were previously isolated from the metastatic sites of patient with advanced melanoma. Instead of intracardic injection, intracarotid injection is able to bypass pulmonary circulation, therefore successfully avoid the trap of tumor cells in the lung. In an effort to develop the mouse model of melanoma brain metastases, we first screened multiple malignant human melanoma lines isolated from different metastatic sites, lymph node (MeWo), brain (TXM-13), skin (SK-mel-2), and lung (MALME-3M), for their capability of forming exclusive brain metastases after intracarotid artery injection in immuno-compromised mice (data not shown). We finally focus on using MeWo cells for the in vivo metastatic model since the exclusive brain metastases formed 100% in the mice administered MeWo cells. The engineering of metastatic melanoma cells with both Fluc and mCherry expression made it is possible to track the in vivo tumor growth of brain metastases in real time and visualize the interaction of metastatic cells with brain microenvironment.

The benefits of using oHSV based therapy to treat melanoma brain metastases include 1), most of human malignant melanoma lines completely respond to the robust oncolysis effects of oHSV regardless of the status of BRAF, NRAS and PTEN, which are main genes linked with advanced melanoma; and 2) oHSV only replicates within tumor cells while sparing normal cells. oHSV based therapies are currently undergoing clinical trials for various types of cancers.

To circumvent the issue dampening the current oHSV trials in clinics and further develop novel, safe and efficient strategy targeting hard-to-access metastatic tumor deposits in the brain, we used MST as a robust cellular delivery vehicle for oHSV. MST is a biologic product that is manufactured from human stem cells obtained from adult bone marrow or other tissue sources. Unlike other cell types, after isolation from a qualified donor, MST may be expanded on a large scale for future clinical use and stored in frozen form until needed. Cells obtained from a single donor require no genetic modification and may be used to produce banks yielding hundreds of thousands to millions of doses of MST product - an amount far greater than other stem cell types can achieve. Thus, the use of MST as delivery vehicles here has major advantages in that they can be easily obtained with great amount and most importantly, they do not form teratomas after implantation in mouse brain (data not shown).

Here, we showed the dynamics of diagnostic oHSV, oHSV-mCh, and oHSV-Fluc delivered by MST in real time in vitro in co-culture experiment and *in vivo* in a mouse model of melanoma brain metastases. Furthermore, we showed the efficacy of MST-oHSV in mouse model of melanoma brain metastases that represent clinical scenarios of circulated tumor cells, tumor cell extravasation, and survival/outgrowth of evading metastatic tumor cells within brain parenchymal. We believe that the new MST-oHSV therapy can be further test in other types of metastatic cancers, especially for those hard-to-access tumor deposits.

### References for Example 2

1. Maher EA, Mietz J, Arteaga CL, DePinho RA, & Mohla S (2009) Brain metastasis: opportunities in basic and translational research. Cancer research 69(15):6015-6020.
2. Fidler IJ, Schackert G, Zhang RD, Radinsky R, & Fujimaki T (1999) The biology of melanoma brain metastasis. Cancer metastasis reviews 18(3):387-400.
3. Sampson JH, Carter JH, Jr., Friedman AH, & Seigler HF (1998) Demographics, prognosis, and therapy in 702 patients with brain metastases from malignant melanoma. Journal of neurosurgery 88(1): 11-20.
4. Kohler BA, et al. (Annual report to the nation on the status of cancer, 1975-2007, featuring tumors of the brain and other nervous system. Journal of the National Cancer Institute 103(9):714-736.
5. Chamberlain MC (Brain metastases: a medical neuro-oncology perspective. Expert review of neurotherapeutics 10(4):563-573.
6. Byrne TN, Cascino TL, & Posner JB (1983) Brain metastasis from melanoma. Journal of neuro-oncology 1(4):313-317.
7. Bindal RK, Sawaya R, Leavens ME, & Lee JJ (1993) Surgical treatment of multiple brain metastases. Journal of neurosurgery 79(2):210-216.
8. Eigentler TK, et al. (Number of metastases, serum lactate dehydrogenase level, and type of treatment are prognostic factors in patients with brain metastases of malignant melanoma. Cancer 117(8):1697-1703.
9. Zakrzewski J, et al. (Clinical variables and primary tumor characteristics predictive of the development of melanoma brain metastases and post-brain metastases survival. Cancer 117(8):1711-1720.
10. Liu TC, Galanis E, & Kirn D (2007) Clinical trial results with oncolytic virotherapy: a century of promise, a decade of progress. Nature clinical practice. Oncology 4(2):101-117.
11. Aghi M & Martuza RL (2005) Oncolytic viral therapies - the clinical experience. Oncogene 24(52):7802-7816.
12. Russell SJ, Peng KW, & Bell JC (2012) Oncolytic virotherapy. Nature biotechnology 30(7):658-670.
13. MacKie RM, Stewart B, & Brown SM (2001) Intralesional injection of herpes simplex virus 1716 in metastatic melanoma. Lancet 357(9255):525-526.
14. Sivendran S, Pan M, Kaufman HL, & Saenger Y (2010) Herpes simplex virus oncolytic vaccine therapy in melanoma. Expert opinion on biological therapy 10(7):1145-1153.
15. Shah K (2012) Mesenchymal stem cells engineered for cancer therapy. Advanced drug delivery reviews 64(8):739-748.
16. Duebgen M, et al. (2014) Stem cells loaded with multimechanistic oncolytic herpes simplex virus variants for brain tumor therapy. Journal of the National Cancer Institute 106(6).

### Materials and Methods

Cell lines. MeWo, SK-MEL-2, SK-MEL-28,TXM-13 and MALME-3M melanoma cells were kindly provided by Dr. David Fisher (MGH, Boston) and cultured in DMEM (MeWo and MALME-3M) or RPMI (SK-MEL-2 and SK-MEL-28) supplemented with 10% FBS and 1% Penicillin-Streptomycin. MST are human stem cells derived from adult bone marrow and were grown in Alpha-MEM (Invitrogen/GIBCO) with 16.5% FBS, 2-4mM L-glutamine and penicillin/streptomycin.

Engineered viral vectors, viral packaging and transduction of tumor cells. Lentiviral construct, Pico2-Fluc.mCherry, was gifted from Dr. Andrew Kung (Dana Farber Cancer Institute; Boston, MA) and Lentiviral packaging was performed by transfection of 293T cells as previously described (Bagci-Onder *et al*., 2011). Pico2-Fluc.GFP was constructed by replacing mCherry with GFP. MeWo cells were transduced at a multiplicity of infection (MOI) of 5 in medium containing protamine sulfate (10 µg/ml). All cells were visualized by fluorescence microscopy for GFP or mCherry expression to confirm transduction. oHSV-mCherry was generated by cloning mCherry cDNA under the IE4/5 immediate early promoter of HSV using the same BAC technique and the shuttle plasmid as with G47Δ-TRAIL. All the recombinant oHSVs express E.coli lacZ driven by endogenous ICP6 promoter.

Cell viability assays. The effect of oHSV on tumor cell viability was measured using CellTiterGlo (Promega, Madison, WI, USA) 4 and 6 days post virus infection. All experiments were performed in triplicate.

Co-cultures of MST and melanoma cells. MST cells were freshly loaded with oHSV-mCh (MOI=1) for 2 hrs and then co-cultured with MeWo-GFP cells at 1:1 ratio on 24-well (0.5x10⁵/well; Costar) in MST medium. MeWo cells were then assessed for both the infection of oHSV-mCh and the cell lysis caused by oHSV infection via counting the GFP+ cell number.

Melanoma brain metastases mouse model. Athymic nude female mice (6∼8 weeks of age, Charles River Laboratories, Wilmington, MA) were anesthetized with ketamine-xylazine and an incision was made to expose the right carotid artery. Using 8-0 suture, both common and internal carotid arteries were temporally ligated and a catheter connected to a 1ml syringe was inserted into the external carotid artery to inject tumor cells. Two hundred thousand MeWo cells suspended in 100 ul PBS were slowly injected through the catheter. The external artery was then permanently ligated under the dissecting scope (Olympus, SZX10) using fine surgical tools and blood circulation was restored by releasing both common and internal carotid blood flow. Mice were imaged for tumor cell presence a few days later and then periodically for tumor progression by bioluminescence. All *in vivo* procedures were approved by the Subcommittee on Research Animal Care at Massachusetts General Hospital.

In vivo mouse experiments. Female SCID mice (6-8 weeks old) obtained from Charles River laboratories (Wilmington, MA) were used in three different *in vivo* experiments. 1) To track the fate of oHSV delivered by MST and the dynamics of oHSV spread in vivo, MST cells were freshly infected with oHSV-Fluc for 2 hrs and 200,000 MST-oHSV-Fluc cells were intracarotidly injected into the brains of either normal mice (without brain tumor, n=3) or brain tumor mice (100,000 MeWo-GFP cells were stereotactically implanted into the mice brain 14 days prior to MST injection, n=3). The fate of oHSV virus within the brain was later assessed by the dynamics of Fluc bioluminescence over time. 2) To directly visualize the distribution of oHSV delivered by MST within brain tumor deposits, 200,000 modified MST cells (carrying oHSV-mCherry) were intracarotidly injected into the mice which bearing brain tumors as described above. Virus distribution was assessed by immunofluorescence on brain sections obtained after MST-oHSV-mCh injection (48h, 72h, and 120h). Briefly, mice were sacrificed and brains were dissected as described. Fourteen µM sections were assessed for GFP and mCherry expression representing tumor cells and oHSV, respectively. Higher magnification images were acquired with Olympus Digital Imaging Software (CellSens). Detailed section analysis was performed using Confocal microscopy (LSM Pascal, Zeiss). 3) To test the therapeutic effects of MST-oHSV, MeWo-FmC cells were intracarotidly injected into SCID mice (n=12) and the metastatic tumor growth in the brain was further confirmed by Fluc bioluminescence imaging. Two weeks later, the mice bearing metastatic brain tumors were treated with either MST (n=6) or MST-oHSV (n=6) via intracarotid artery administration. Mice were followed for changes in brain tumor volumes by BLI as well as followed for survival.

Statistical analysis. Data were analyzed by Student *t*-test when comparing two groups and ANOVA when comparing more than two groups. Data were plotted as mean ± SEM and differences were considered significant at *P* < 0.05. Survival curves were compared using the log-rank test. Analyses were done using GraphPad Prism 5.01.

## Claims

1. A composition for use in treating a secondary multifocal metastatic cancer in the brain, wherein the composition comprises an isolated non-cancer stem cell selected from the group consisting of a mesenchymal stem cell (MSC), a neuronal stem cell, and an induced pluripotent stem cell, or population thereof, comprising infectious recombinant oncolytic herpes simplex virus (oHSV), wherein the composition is administered via intracarotid injection.

2. The composition for use according to claim 1, wherein the isolated non-cancer stem cell or population thereof is human.

3. The composition for use according to claim 1, wherein the MSC is derived from bone marrow, umbilical cord blood, or adipose tissue.

4. The composition for use according to claim 1, wherein the oncolytic HSV is engineered to be inducible by addition of an exogenous factor, and optionally wherein the oncolytic HSV is engineered to have a tetracycline inducible promoter driving ICP27 expression.

5. The composition for use according to claim 1, wherein the oncolytic HSV is engineered to comprise a nucleic acid sequence encoding tumor necrosis factor-related apoptosis-inducing ligand (TRAIL) or a biologically active fragment thereof, in expressible form, and optionally wherein the TRAIL is a secreted form of TRAIL (S-TRAIL).

6. The composition for use according to claim 1, wherein the oHSV is selected from the group consisting of G207, G47Δ HSV-R3616, 1716, R3616, and R4009.

7. The composition for use according to claim 5, wherein the TRAIL is a TRAIL fusion protein; and/or wherein the TRAIL is regulated by the HSV immediate early 4/5 promoter.

8. The composition for use according to claim 1, wherein the virus contains an additional exogenous nucleic acid in expressible form; or wherein the virus contains no additional exogenous nucleic acids.

9. The composition for use according to claim 1, that is encapsulated in a synthetic extracellular matrix (sECM).

10. A pharmaceutical composition for use according to claim 1 comprising the composition of any of claims 1 to 9, and a pharmaceutically acceptable carrier.

## Patentansprüche

1. Zusammensetzung für die Verwendung beim Behandeln eines sekundären multifokalen metastasierenden Krebses im Gehirn, wobei die Zusammensetzung eine isolierte Nichtkrebs-Stammzelle umfasst, ausgewählt aus der Gruppe bestehend aus einer mesenchymalen Stammzelle (MSC), einer neuronalen Stammzelle und einer induzierten pluripotenten Stammzelle oder einer Population davon, umfassend infektiöses rekombinantes onkolytisches Herpes-simplex-Virus (oHSV), wobei die Zusammensetzung über intracarotide Injektion verabreicht wird.

2. Zusammensetzung für die Verwendung nach Anspruch 1, wobei die isolierte Nichtkrebs-Stammzelle oder die Population davon human ist.

3. Zusammensetzung für die Verwendung nach Anspruch 1, wobei die MSC aus Knochenmark, Nabelschnurblut oder Fettgewebe abgeleitet ist.

4. Zusammensetzung für die Verwendung nach Anspruch 1, wobei das onkolytische HSV verändert ist, so dass es durch Zugeben eines exogenen Faktors induzierbar ist und optional wobei das onkolytische HSV verändert ist, so dass es einen durch Tetracyclin induzierbaren Promotor, der die Exprimierung von ICP27 antreibt, aufweist.

5. Zusammensetzung für die Verwendung nach Anspruch 1, wobei das onkolytische HSV verändert ist, so dass es eine Nukleinsäuresequenz umfasst, die für den Tumornekrosefaktor-verwandten Apoptose induzierenden Liganden (TRAIL) oder für ein biologisch aktives Fragment davon in exprimierbarer Form kodiert, und optional wobei der TRAIL eine sezernierte Form von TRAIL (S-TRAIL) ist.

6. Zusammensetzung für die Verwendung nach Anspruch 1, wobei das oHSV ausgewählt ist aus der Gruppe bestehend aus G207, G47Δ HSV-R3616, 1716, R3616 und R4009.

7. Zusammensetzung für die Verwendung nach Anspruch 5, wobei der TRAIL ein TRAIL-Fusionsprotein ist; und/oder wobei der TRAIL durch den HSV-Immediate-early-4/5-Promotor geregelt wird.

8. Zusammensetzung für die Verwendung nach Anspruch 1, wobei das Virus eine zusätzliche exogene Nukleinsäure in exprimierbarer Form enthält; oder wobei das Virus keine zusätzlichen exogenen Nukleinsäuren enthält.

9. Zusammensetzung für die Verwendung nach Anspruch 1, die in einer synthetischen extrazellulären Matrix (SECM) eingekapselt ist.

10. Pharmazeutische Zusammensetzung für die Verwendung nach Anspruch 1, umfassend die Zusammensetzung gemäß einem der Ansprüche 1 bis 9 und einen pharmazeutisch unbedenklichen Träger.

## Revendications

1. Composition pour une utilisation dans un traitement d'un cancer métastatique multifocal secondaire dans le cerveau, la composition comprenant une cellule souche non cancéreuse isolée choisie dans le groupe constitué par une cellule souche mésenchymateuse (MSC), une cellule souche neuronale, et une cellule souche pluripotente induite, ou une population correspondante, comprenant un virus de l'herpès simplex oncolytique recombinant infectieux (oHSV), la composition étant administrée via une injection intracarotidienne.

2. Composition pour une utilisation selon la revendication 1, la cellule souche non cancéreuse isolée ou la population correspondante étant humaine.

3. Composition pour une utilisation selon la revendication 1, la MSC étant issue de moelle osseuse, de sang de cordon ombilical, ou de tissu adipeux.

4. Composition pour une utilisation selon la revendication 1, le HSV oncolytique étant conçu pour être inductible par ajout d'un facteur exogène, éventuellement le HSV oncolytique étant conçu pour posséder un promoteur inductible de tétracycline dirigeant l'expression de ICP27.

5. Composition pour une utilisation selon la revendication 1, le HSV oncolytique étant conçu pour comprendre une séquence d'acides nucléiques codant pour le ligand induisant l'apoptose apparentée au facteur de nécrose tumorale (TRAIL) ou un fragment biologiquement actif correspondant, sous forme exprimable, éventuellement le TRAIL étant une forme sécrétée de TRAIL (S-TRAIL).

6. Composition pour une utilisation selon la revendication 1, le oHSV étant choisi dans le groupe constitué par G207, G47Δ, HSV-R3616, 1716, R3616, et R4009.

7. Composition pour une utilisation selon la revendication 5, le TRAIL étant une protéine de fusion de TRAIL ; et/ou le TRAIL étant régulé par le promoteur 4/5 précoce immédiat de HSV.

8. Composition pour une utilisation selon la revendication 1, le virus contenant un acide nucléique exogène supplémentaire sous forme exprimable ; ou le virus ne contenant aucun acide nucléique exogène supplémentaire.

9. Composition pour une utilisation selon la revendication 1, qui est encapsulée dans une matrice extracellulaire synthétique (sECM).

10. Composition pharmaceutique pour une utilisation selon la revendication 1 comprenant la composition selon l'une quelconque des revendications 1 à 9, et un support pharmaceutiquement acceptable.
